# EUROPEAN PATENT APPLICATION

(11) **EP 0 699 751 A1**
(43) Date of publication of application: **06.03.1996**
(21) Application number: 93113572.7
(22) Date of filing: 25.08.1993
(51) Int. Cl.: C12N 15/11, A61K 31/70, C07H 21/04, C12N 15/51, C12N 15/62, C12N 15/86

(54) **Antisense complementary to HCV genome**

(30) Priority: 25.08.1992 JP 248796/92; 03.03.1993 JP 42736/93; 09.08.1993 JP 217095/93
(71) Applicant: Mitsubishi Chemical Corporation, Chiyoda-ku Tokyo (JP)
(72) Inventor: Seki, Makoto, Machida-shi, Tokyo-to (JP); Honda, Yoshikazu, Kamakura-shi, Kanagawa-ken (JP); Yamada, Ei, Kawasaki-shi, Kanagawa-ken (JP)
(74) Representative: Hansen, Bernd, Dr. Dipl.-Chem.

(57) **Abstract**

Antisense compounds which are complementary to a genome derived from hepatitis C virus (HCV) were provided. Because the antisense compounds of the present invention act specifically on mRNA of HCV and inhibits translation of HCV gene, they may be useful as an antiviral agent.

SEQUENCE LISTING

(1) GENERAL INFORMATION:

(i) APPLICANT:
(A) NAME: Mitsubishi Kasei Corporation
(B) STREET: 5-2, Maronouchi 2-chome, Chiyoda-ku
(C) CITY: Tokyo
(E) COUNTRY: Japan
(F) POSTAL CODE (ZIP): none

(ii) TITLE OF INVENTION: Antisense compounds complementary to HCV
genome

(iii) NUMBER OF SEQUENCES: 410

(iv) COMPUTER READABLE FORM:
(A) MEDIUM TYPE: Floppy disk
(B) COMPUTER: IBM PC compatible
(C) OPERATING SYSTEM: PC-DOS/MS-DOS

(v) CURRENT APPLICATION DATA:
APPLICATION NUMBER: EP 93113572.7
(vi) PRIOR APPLICATION DATA:
(A) APPLICATION NUMBER: JP 248796/1992
(B) FILING DATE: 25-AUG-1992

(vi) PRIOR APPLICATION DATA:
(A) APPLICATION NUMBER: JP 42736/1993
(B) FILING DATE: 03-MAR-1993

(vi) PRIOR APPLICATION DATA:
(A) APPLICATION NUMBER: JP 217095/1993
(B) FILING DATE: 09-AUG-1993

(2) INFORMATION FOR SEQ ID NO:1:
(i) SEQUENCE CHARACTERISTICS:
(A) LENGTH: 2033 base pairs
(B) TYPE: nucleic acid
(C) STRANDEDNESS: double
(D) TOPOLOGY: linear
(ii) MOLECULE TYPE: DNA
(iv) ANTI-SENSE: No
(vi) ORIGINAL SOURCE:
(A) ORGANISM: Hepatitis C virus
(vii) IMMEDIATE SOURCE:
(B) CLONE: T7N1-19

(xi) SEQUENCE DESCRIPTION: SEQ ID NO:1:

ACTAGTTAAT ACGACTCACT ATAGGGTGCC AGCCCCCTGA TGGGGGCGAC ACTCCACCAT 60
AGATCACTCC CCTGTGAGGA ACTACTGTCT TCACGCAGAA AGCGTCTAGC CATGGCGTTA 120
GTATGAGTGT CGTGCAGCCT CCAGGACCCC CCCTCCCGGG AGAGCCATAG TGGTCTGCGG 180
AACCGGTGAG TACACCGGAA TTGCCAGGAC GACCGGGTCC TTTCTTGGAT CAACCCGCTC 240
AATGCCTGGA GATTTGGGCG TGCCCCCGCG AGACTGCTAG CCGAGTAGTG TTGGGTCGCG 300
AAAGGCCTTG TGGTACTGCC TGATAGGGTG CTTGCGAGTG CCCCGGGAGG TCTCGTAGAC 360
CGTGCATC ATG AGC ACA AAT CCA AAA CCC CAA AGA AAA ATC AAA CGT AAC 410
Met Ser Thr Asn Pro Lys Pro Gln Arg Lys Ile Lys Arg Asn

                 1               5                   10
ACC AAC CGC CGC CCA CAG GAC GTT AAG TTC CCG GGC GGT GGT CAG ATC   458
Thr Asn Arg Arg Pro Gln Asp Val Lys Phe Pro Gly Gly Gly Gln Ile
 15                  20                  25                  30
GTT GGT GGA GTT TAC CTG TTG CCG CGC AGG GGC CCC AGG TTG GGT GTG   506
Val Gly Gly Val Tyr Leu Leu Pro Arg Arg Gly Pro Arg Leu Gly Val
                 35                  40                  45
CGC GCG ACT AGG AAG ACT TCC GAG CGG CCG CAA CCT CGT GGA AGG CGA   554
Arg Ala Thr Arg Lys Thr Ser Glu Arg Pro Gln Pro Arg Gly Arg Arg
             50                  55                  60
CAA CCT ATC CCC AAG GCT CGC CAA CCC GAG GGT AGG GCC TGG GCT CAG   602
Gln Pro Ile Pro Lys Ala Arg Gln Pro Glu Gly Arg Ala Trp Ala Gln
         65                  70                  75
CCC GGG TAC CCT TGG CCC CTC TAT GGC AAT GAG GGC TTG GGG TGG GCA   650
Pro Gly Tyr Pro Trp Pro Leu Tyr Gly Asn Glu Gly Leu Gly Trp Ala
     80                  85                  90
GGA TGG CTC CTG TCA CCC CGC GGC TCC CGG CCT AGT TGG GGC CCC ACG   698
Gly Trp Leu Leu Ser Pro Arg Gly Ser Arg Pro Ser Trp Gly Pro Thr
 95                 100                 105                 110
GAC CCC CGG CGT AGG TCG CGT AAT TTG GGT AAG GTC ATC GAT ACC CTC   746
Asp Pro Arg Arg Arg Ser Arg Asn Leu Gly Lys Val Ile Asp Thr Leu
                115                 120                 125
ACA TGC GGC TTC GCC GAC CTC ATG GGG TAC ATT CCG CTC GTC GGC GCC   794
Thr Cys Gly Phe Ala Asp Leu Met Gly Tyr Ile Pro Leu Val Gly Ala
            130                 135                 140
CCC CTA GGG GGC GCT GCC AGG GCT CTA GCG CAT GGC GTC CGG GTT CTG   842
Pro Leu Gly Gly Ala Ala Arg Ala Leu Ala His Gly Val Arg Val Leu
        145                 150                 155
GAG GAC GGC GTG AAC TAT GCA ACA GGG AAT CTG CCT GGT TGC TCC TTT   890
Glu Asp Gly Val Asn Tyr Ala Thr Gly Asn Leu Pro Gly Cys Ser Phe
    160                 165                 170
TCT ATC TTC CTT TTG GCT TTG CTG TCC TGT TTG ACC ATC CCA GCT TCC   938
Ser Ile Phe Leu Leu Ala Leu Leu Ser Cys Leu Thr Ile Pro Ala Ser
175                 180                 185                 190
GCC TAC CAA GTG CGC AAC GCG TCC GGG GTG TAC CAT GTC ACG AAC GAC   986
Ala Tyr Gln Val Arg Asn Ala Ser Gly Val Tyr His Val Thr Asn Asp
                195                 200                 205
TGC TCC AAC TCA AGT ATT GTG TAT GAG GCG GCG GAC GTG ATT ATG CAC   1034
Cys Ser Asn Ser Ser Ile Val Tyr Glu Ala Ala Asp Val Ile Met His
            210                 215                 220
ACC CCC GGG TGC GTG CCC TGC GTC CGG GAG AAC AAT TCC TCC CGC TGC   1082
Thr Pro Gly Cys Val Pro Cys Val Arg Glu Asn Asn Ser Ser Arg Cys
        225                 230                 235
TGG GTA GCG CTC ACT CCC ACG CTT GCG GCC AGG AAC AGC AGC ATC CCC   1130
Trp Val Ala Leu Thr Pro Thr Leu Ala Ala Arg Asn Ser Ser Ile Pro
    240                 245                 250
ACT ACG ACA ATA CGG CGT CAT GTC GAC TTG CTC GTT GGG GCA GCT GCT   1178
Thr Thr Thr Ile Arg Arg His Val Asp Leu Leu Val Gly Ala Ala Ala
255                 260                 265                 270
CTC TGT TCC GCT ATG TAT GTG GGG GAT TTT TGC GGA TCT GTT TTC CTC   1226
Leu Cys Ser Ala Met Tyr Val Gly Asp Phe Cys Gly Ser Val Phe Leu
                275                 280                 285

GTC TCC CAG CTG TTC ACT TTC TCA CCT CGC CGG TAT GAG ACG GTG CAA   1274
Val Ser Gln Leu Phe Thr Phe Ser Pro Arg Arg Tyr Glu Thr Val Gln
            290                 295                 300
GAC TGC AAT TGC TCA ATC TAT CCC GGC CAT GTA TCA GGC CAT CGC ATG   1322
Asp Cys Asn Cys Ser Ile Tyr Pro Gly His Val Ser Gly His Arg Met
        305                 310                 315
GCT TGG GAT ATG ATA ATG AAT TGG TCA CCT ACA ACA GCC CTA GTG GTA   1370
Ala Trp Asp Met Ile Met Asn Trp Ser Pro Thr Thr Ala Leu Val Val
    320                 325                 330
TCG CAG CTA CTC CGG ATC CCA CAA GCC GTG GTG GAT ATG GTG GCA GGG   1418

Ser Gln Leu Leu Arg Ile Pro Gln Ala Val Val Asp Met Val Ala Gly
335                 340                 345                 350
GCC CAC TGG GGA GTC CTG GCG GGC CTT GCC TAC TAT TCC ATG GTG GGG   1466
Ala His Trp Gly Val Leu Ala Gly Leu Ala Tyr Tyr Ser Met Val Gly
                355                 360                 365
AAC TGG GCT AAG GTC TTG GTT GTG ATG CTG CTC TTC GCC GGT GTT GAC   1514
Asn Trp Ala Lys Val Leu Val Val Met Leu Leu Phe Ala Gly Val Asp
            370                 375                 380
GGG GGG ACC CAC GTG ACA GGG GGG AAG GTA GCC TAC ACC ACC CAG GGC   1562
Gly Gly Thr His Val Thr Gly Gly Lys Val Ala Tyr Thr Thr Gln Gly
        385                 390                 395
TTT ACA TCC TTC TTT TCA CGA GGG CCG TCT CAG AAA ATC CAA CTT GTA   1610
Phe Thr Ser Phe Phe Ser Arg Gly Pro Ser Gln Lys Ile Gln Leu Val
    400                 405                 410
AAC ACT AAC GGC AGC TGG CAC ATC AAT AGG ACT GCC CTC AAT TGC AAT   1658
Asn Thr Asn Gly Ser Trp His Ile Asn Arg Thr Ala Leu Asn Cys Asn
415                 420                 425                 430
GAC TCC CTT AAC ACC GGG TTC CTT GCC GCG CTG TTC TAC ACC CAC AGC   1706
Asp Ser Leu Asn Thr Gly Phe Leu Ala Ala Leu Phe Tyr Thr His Ser
                435                 440                 445
TTC AAC GCG TCC GGA TGT CCG GAG CGT ATG GCC GGT TGC CGC CCC ATT   1754
Phe Asn Ala Ser Gly Cys Pro Glu Arg Met Ala Gly Cys Arg Pro Ile
            450                 455                 460
GAC GAG TTC GCT CAG GGG TGG GGT CCC ATC ACT CAT GTT GTG CCT AAC   1802
Asp Glu Phe Ala Gln Gly Trp Gly Pro Ile Thr His Val Val Pro Asn
        465                 470                 475
ATC TCG GAC CAG AGG CCC TAT TGC TGG CAC TAC GCG CCT CGA CCG TGT   1850
Ile Ser Asp Gln Arg Pro Tyr Cys Trp His Tyr Ala Pro Arg Pro Cys
    480                 485                 490
GGT ATC GTA CCC GCG TCG CAG GTG TGT GGT CCG GTG TAT TGC TTC ACC   1898
Gly Ile Val Pro Ala Ser Gln Val Cys Gly Pro Val Tyr Cys Phe Thr
495                 500                 505                 510
CCA AGC CCT GTT GTG GTG GGG ACG ACC GAT CGT TTC GGC GCC CCC ACG   1946
Pro Ser Pro Val Val Val Gly Thr Thr Asp Arg Phe Gly Ala Pro Thr
                515                 520                 525
TAC AAC TGG GGA AAC AAT GAG ACG GAT GTG CTA CTC CTC AAC AAC ACA   1994
Tyr Asn Trp Gly Asn Asn Glu Thr Asp Val Leu Leu Leu Asn Asn Thr
            530                 535                 540
CGG CCG CCG CAG GGC AAC TGG TTC GGT TGT ACC TGG ATG               2033
Arg Pro Pro Gln Gly Asn Trp Phe Gly Cys Thr Trp Met
        545                 550                 555

(2) INFORMATION FOR SEQ ID NO:2:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:2:

TCCTGGAGGC TGCACG                                                 16

(2) INFORMATION FOR SEQ ID NO:3:
(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: DNA
(iv) ANTI-SENSE: Yes

(xi) SEQUENCE DESCRIPTION: SEQ ID NO:3:

GTCCTGGAGG CTGCACG 17

(2) INFORMATION FOR SEQ ID NO:4:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:4:

GGTCCTGGAG GCTGCACG 18

(2) INFORMATION FOR SEQ ID NO:5:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:5:

GGGTCCTGGA GGCTGCACG 19

(2) INFORMATION FOR SEQ ID NO:6:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:6:

GGGGTCCTGG AGGCTGCACG 20

(2) INFORMATION FOR SEQ ID NO:7:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:7:

TCCTGGAGGC TGCACGA 17

(2) INFORMATION FOR SEQ ID NO:8:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:8:

GTCCTGGAGG CTGCACGA 18

(2) INFORMATION FOR SEQ ID NO:9:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:9:

GGTCCTGGAG GCTGCACGA 19

(2) INFORMATION FOR SEQ ID NO:10:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:10:

GGGTCCTGGA GGCTGCACGA 20

(2) INFORMATION FOR SEQ ID NO:11:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:11:

GGGGTCCTGG AGGCTGCACG A 21

(2) INFORMATION FOR SEQ ID NO:12:
(i) SEQUENCE CHARACTERISTICS:
(A) LENGTH: 18 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:12:

TCCTGGAGGC TGCACGAC 18

(2) INFORMATION FOR SEQ ID NO:13:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:13:
GTCCTGGAGG CTGCACGAC 19

(2) INFORMATION FOR SEQ ID NO:14:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:14:

GGTCCTGGAG GCTGCACGAC 20

(2) INFORMATION FOR SEQ ID NO:15:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:15:

GGGTCCTGGA GGCTGCACGA C 21

(2) INFORMATION FOR SEQ ID NO:16:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:16:

GGGGTCCTGG AGGCTGCACG AC 22

(2) INFORMATION FOR SEQ ID NO:17:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:17:

TCCTGGAGGC TGCACGACA 19

(2) INFORMATION FOR SEQ ID NO:18:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:18:
GTCCTGGAGG CTGCACGACA 20

(2) INFORMATION FOR SEQ ID NO:19:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:19:
GGTCCTGGAG GCTGCACGAC A 21

(2) INFORMATION FOR SEQ ID NO:20:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:20:
GGGTCCTGGA GGCTGCACGA CA 22

(2) INFORMATION FOR SEQ ID NO:21:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA

(iv) ANTI-SENSE: Yes

(xi) SEQUENCE DESCRIPTION: SEQ ID NO:21:
GGGGTCCTGG AGGCTGCACG ACA 23

(2) INFORMATION FOR SEQ ID NO:22:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:22:
TCCTGGAGGC TGCACGACAC 20

(2) INFORMATION FOR SEQ ID NO:23:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:23:
GTCCTGGAGG CTGCACGACA C 21

(2) INFORMATION FOR SEQ ID NO:24:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:24:
GGTCCTGGAG GCTGCACGAC AC 22

(2) INFORMATION FOR SEQ ID NO:25:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:25:
GGGTCCTGGA GGCTGCACGA CAC 23

(2) INFORMATION FOR SEQ ID NO:26:
(i) SEQUENCE CHARACTERISTICS:
(A) LENGTH: 24 base pairs
(B) TYPE: nucleic acid
(C) STRANDEDNESS: single

(D) TOPOLOGY: linear
(ii) MOLECULE TYPE: DNA
(iv) ANTI-SENSE: Yes

(xi) SEQUENCE DESCRIPTION: SEQ ID NO:26:
GGGGTCCTGG AGGCTGCACG ACAC 24

(2) INFORMATION FOR SEQ ID NO:27:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:27:
CTCTCCCGGG AGGGG 15

(2) INFORMATION FOR SEQ ID NO:28:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:28:
GGCTCTCCCG GGAGGGG 17

(2) INFORMATION FOR SEQ ID NO:29:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:29:
ATGGCTCTCC CGGGAGGGG 19

(2) INFORMATION FOR SEQ ID NO:30:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:30:
CTATGGCTCT CCCGGGAGGG G 21

(2) INFORMATION FOR SEQ ID NO:31:
(i) SEQUENCE CHARACTERISTICS:
(A) LENGTH: 23 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:31:
CACTATGGCT CTCCCGGGAG GGG 24

(2) INFORMATION FOR SEQ ID NO:32:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:32:

ACCACTATGG CTCTCCCGGG AGGGG 25

(2) INFORMATION FOR SEQ ID NO:33:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:33:

GCTCTCCCGG GAGGG 15

(2) INFORMATION FOR SEQ ID NO:34:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:34:

TGGCTCTCCC GGGAGGG 17

(2) INFORMATION FOR SEQ ID NO:35:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:35:

CTATGGCTCT CCCGGGAGGG 20

(2) INFORMATION FOR SEQ ID NO:36:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:36:

ACCACTATGG CTCTCCCGGG AGGG 24

(2) INFORMATION FOR SEQ ID NO:37:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:37:

CAGACCACTA TGGCTCTCCC GGGAGGG 27

(2) INFORMATION FOR SEQ ID NO:38:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:38:

CCGCAGACCA CTATGGCTCT CCCGGGAGGG 30

(2) INFORMATION FOR SEQ ID NO:39:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:39:

CCGCAGACCA CTATG 15

(2) INFORMATION FOR SEQ ID NO:40:
(i) SEQUENCE CHARACTERISTICS:
(A) LENGTH: 17 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:40:

CCGCAGACCA CTATGGC 17

(2) INFORMATION FOR SEQ ID NO:41:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:41:

CCGCAGACCA CTATGGCTCT 20

(2) INFORMATION FOR SEQ ID NO:42:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:42:

CCGCAGACCA CTATGGCTCT CCCG 24

(2) INFORMATION FOR SEQ ID NO:43:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:43:

CCGCAGACCA CTATGGCTCT CCCGGGA 27

(2) INFORMATION FOR SEQ ID NO:44:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:44:

CGACCCAACA CTACT 15

(2) INFORMATION FOR SEQ ID NO:45:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:45:

CGCGACCCAA CACTACT 17

(2) INFORMATION FOR SEQ ID NO:46:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:46:

TTTCGCGACC CAACACTACT 20

(2) INFORMATION FOR SEQ ID NO:47:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:47:

GCGACCCAAC ACTAC 15

(2) INFORMATION FOR SEQ ID NO:48:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:48:

TTCGCGACCC AACACTAC 18

(2) INFORMATION FOR SEQ ID NO:49:
(i) SEQUENCE CHARACTERISTICS:
(A) LENGTH: 20 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:49:

CTTTCGCGAC CCAACACTAC 20

(2) INFORMATION FOR SEQ ID NO:50:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:50:

ACCACAAGGC CTTTC 15

(2) INFORMATION FOR SEQ ID NO:51:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:51:

ACCACAAGGC CTTTCGC 17

(2) INFORMATION FOR SEQ ID NO:52:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:52:

ACCACAAGGC CTTTCGCGAC 20

(2) INFORMATION FOR SEQ ID NO:53:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:53:

TACCACAAGG CCTTT 15

(2) INFORMATION FOR SEQ ID NO:54:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:54:

TACCACAAGG CCTTTCG 17

(2) INFORMATION FOR SEQ ID NO:55:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:55:

TACCACAAGG CCTTTCGCGA 20

(2) INFORMATION FOR SEQ ID NO:56:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:56:

AGTACCACAA GGCCT 15

(2) INFORMATION FOR SEQ ID NO:57:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:57:

AGTACCACAA GGCCTTT 17

(2) INFORMATION FOR SEQ ID NO:58:
(i) SEQUENCE CHARACTERISTICS:
(A) LENGTH: 20 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:58:

AGTACCACAA GGCCTTTCGC 20

(2) INFORMATION FOR SEQ ID NO:59:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:59:

TCTACGAGAC CTCCCGG 17

(2) INFORMATION FOR SEQ ID NO:60:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:60:

CGGTCTACGA GACCTCCCGG 20

(2) INFORMATION FOR SEQ ID NO:61:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:61:

GCACGGTCTA CGAGACCTCC CGG 23

(2) INFORMATION FOR SEQ ID NO:62:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:62:

GATGCACGGT CTACGAGACC TCCCGG 26

(2) INFORMATION FOR SEQ ID NO:63:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:63:

ATGATGCACG GTCTACGAGA CCTCCCGG 28

(2) INFORMATION FOR SEQ ID NO:64:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:64:

TCATGATGCA CGGTCTACGA GACCTCCCGG 30

(2) INFORMATION FOR SEQ ID NO:65:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:65:

TCTACGAGAC CTCCC 15

(2) INFORMATION FOR SEQ ID NO:66:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:66:

CGGTCTACGA GACCTCCC 18

(2) INFORMATION FOR SEQ ID NO:67:
(i) SEQUENCE CHARACTERISTICS:
(A) LENGTH: 21 base pairs
(B) TYPE: nucleic acid
(C) STRANDEDNESS: single

(D) TOPOLOGY: linear
(ii) MOLECULE TYPE: DNA
(iv) ANTI-SENSE: Yes

(xi) SEQUENCE DESCRIPTION: SEQ ID NO:67:

GCACGGTCTA CGAGACCTCC C 21

(2) INFORMATION FOR SEQ ID NO:68:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:68:

GATGCACGGT CTACGAGACC TCCC 24

(2) INFORMATION FOR SEQ ID NO:69:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:69:

ATGATGCACG GTCTACGAGA CCTCCC 26

(2) INFORMATION FOR SEQ ID NO:70:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:70:

TCATGATGCA CGGTCTACGA GACCTCCC 28

(2) INFORMATION FOR SEQ ID NO:71:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:71:

CTCATGATGC ACGGTCTACG AGACCTCCC 29

(2) INFORMATION FOR SEQ ID NO:72:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:72:

GCTCATGATG CACGGTCTAC GAGACCTCCC 30

(2) INFORMATION FOR SEQ ID NO:73:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:73:

CGGTCTACGA GACCT 15

(2) INFORMATION FOR SEQ ID NO:74:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:74:

GCACGGTCTA CGAGACCT 18

(2) INFORMATION FOR SEQ ID NO:75:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:75:

GATGCACGGT CTACGAGACC T 21

(2) INFORMATION FOR SEQ ID NO:76:
(i) SEQUENCE CHARACTERISTICS:
(A) LENGTH: 23 base pairs
(B) TYPE: nucleic acid
(C) STRANDEDNESS: single

(D) TOPOLOGY: linear
(ii) MOLECULE TYPE: DNA

(iv) ANTI-SENSE: Yes

(xi) SEQUENCE DESCRIPTION: SEQ ID NO:76:

ATGATGCACG GTCTACGAGA CCT 23

(2) INFORMATION FOR SEQ ID NO:77:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:77:

TCATGATGCA CGGTCTACGA GACCT 25

(2) INFORMATION FOR SEQ ID NO:78:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:78:

CTCATGATGC ACGGTCTACG AGACCT 26

(2) INFORMATION FOR SEQ ID NO:79:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:79:

GCTCATGATG CACGGTCTAC GAGACCT 27

(2) INFORMATION FOR SEQ ID NO:80
(i) SEQUENCE CHARACTERISTICS:
(A) LENGTH: 29 base pairs:
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:80:

GTGCTCATGA TGCACGGTCT ACGAGACCT 29

(2) INFORMATION FOR SEQ ID NO:81:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:81:

GCACGGTCTA CGAGA 15

(2) INFORMATION FOR SEQ ID NO:82:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:82:

GATGCACGGT CTACGAGA 18

(2) INFORMATION FOR SEQ ID NO:83:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:83:

ATGATGCACG GTCTACGAGA 20

(2) INFORMATION FOR SEQ ID NO:84:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:84:

TCATGATGCA CGGTCTACGA GA 22

(2) INFORMATION FOR SEQ ID NO:85:
(i) SEQUENCE CHARACTERISTICS:
(A) LENGTH: 23 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:85:

CTCATGATGC ACGGTCTACG AGA 23

(2) INFORMATION FOR SEQ ID NO:86:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:86:

GCTCATGATG CACGGTCTAC GAGA 24

(2) INFORMATION FOR SEQ ID NO:87:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:87:

GTGCTCATGA TGCACGGTCT ACGAGA 26

(2) INFORMATION FOR SEQ ID NO:88:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:88:

ATGCACGGTC TACGA 15

(2) INFORMATION FOR SEQ ID NO:89:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:89:

ATGATGCACG GTCTACGA 18

(2) INFORMATION FOR SEQ ID NO:90:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:90:

TCATGATGCA CGGTCTACGA 20

(2) INFORMATION FOR SEQ ID NO:91:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:91:

CTCATGATGC ACGGTCTACG A 21

(2) INFORMATION FOR SEQ ID NO:92:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:92:

GCTCATGATG CACGGTCTAC GA 22

(2) INFORMATION FOR SEQ ID NO:93:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:93:

GTGCTCATGA TGCACGGTCT ACGA 24

(2) INFORMATION FOR SEQ ID NO:94:
(i) SEQUENCE CHARACTERISTICS:
(A) LENGTH: 15 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:94:

TGATGCACGG TCTAC 15

(2) INFORMATION FOR SEQ ID NO:95:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:95:

ATGATGCACG GTCTAC 16

(2) INFORMATION FOR SEQ ID NO:96:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:96:

TCATGATGCA CGGTCTAC 18

(2) INFORMATION FOR SEQ ID NO:97:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:97:

CTCATGATGC ACGGTCTAC 19

(2) INFORMATION FOR SEQ ID NO:98:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:98:

GCTCATGATG CACGGTCTAC 20

(2) INFORMATION FOR SEQ ID NO:99:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:99:

GTGCTCATGA TGCACGGTCT AC 22

(2) INFORMATION FOR SEQ ID NO:100:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:100:

CTCATGATGC ACGGTC 16

(2) INFORMATION FOR SEQ ID NO:101:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:101:

GCTCATGATG CACGGTC 17

(2) INFORMATION FOR SEQ ID NO:102:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:102:

GTGCTCATGA TGCACGGTC 19

(2) INFORMATION FOR SEQ ID NO:103:
(i) SEQUENCE CHARACTERISTICS:
(A) LENGTH: 15 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:103:

GCTCATGATG CACGG 15

(2) INFORMATION FOR SEQ ID NO:104:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:104:

GTGCTCATGA TGCACGG 17

(2) INFORMATION FOR SEQ ID NO:105:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:105:

GTGCTCATGA TGCAC 15

(2) INFORMATION FOR SEQ ID NO:106:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:106:

CCGGGAGGGG GGGTCCTGG 19

(2) INFORMATION FOR SEQ ID NO:107:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:107:

TACTCACCGG TTCCGCAGAC CACTAT 26

(2) INFORMATION FOR SEQ ID NO:108:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:108:

TCATACTAAC GCCATGGCTA 20

(2) INFORMATION FOR SEQ ID NO:109:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:109:

GTAGTTCCTC ACAGGGGAGT 20

(2) INFORMATION FOR SEQ ID NO:110:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:110:

ACCACTATGG CTCTCCCGGG 20

(2) INFORMATION FOR SEQ ID NO:111:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:111:

GGTTCCGCAG ACCACTATGG 20

(2) INFORMATION FOR SEQ ID NO:112:
(i) SEQUENCE CHARACTERISTICS:
(A) LENGTH: 20 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:112:

TGGAGGCTGC ACGACACTCA 20

(2) INFORMATION FOR SEQ ID NO:113
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:113:

CAGTACCACA AGGCCTTTCG 20

(2) INFORMATION FOR SEQ ID NO: 114:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:114:

GGGGGGGTCC TGGAGGCTGC ACGACAC 27

(2) INFORMATION FOR SEQ ID NO: 115:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:115:

AGGGGGGGTC CTGGAGGCTG CACGACAC 28

(2) INFORMATION FOR SEQ ID NO: 116:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:116:

GAGGGGGGGT CCTGGAGGCT GCACGACAC 29

(2) INFORMATION FOR SEQ ID NO: 117:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:117:

GGAGGGGGGG TCCTGGAGGC TGCACGACAC 30

(2) INFORMATION FOR SEQ ID NO: 118:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:118:

GGGGGGGTCC TGGAGGCTGC ACGACA 26

(2) INFORMATION FOR SEQ ID NO: 119:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:119:

AGGGGGGGTC CTGGAGGCTG CACGACA 27

(2) INFORMATION FOR SEQ ID NO: 120:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:120:

GAGGGGGGGT CCTGGAGGCT GCACGACA 28

(2) INFORMATION FOR SEQ ID NO: 121:
(i) SEQUENCE CHARACTERISTICS:
(A) LENGTH: 29 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:121:

GGAGGGGGGG TCCTGGAGGC TGCACGACA 29

(2) INFORMATION FOR SEQ ID NO: 122:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:122:

GGGAGGGGGG GTCCTGGAGG CTGCACGACA 30

(2) INFORMATION FOR SEQ ID NO: 123:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:123:

GGGGGGGTCC TGGAGGCTGC ACGAC 25

(2) INFORMATION FOR SEQ ID NO: 124:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:124:

AGGGGGGGTC CTGGAGGCTG CACGAC 26

(2) INFORMATION FOR SEQ ID NO: 125:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:125:

GAGGGGGGGT CCTGGAGGCT GCACGAC 27

(2) INFORMATION FOR SEQ ID NO: 126:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:126:

GGAGGGGGGG TCCTGGAGGC TGCACGAC 28

(2) INFORMATION FOR SEQ ID NO: 127:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:127:

GGGAGGGGGG GTCCTGGAGG CTGCACGAC 29

(2) INFORMATION FOR SEQ ID NO: 128:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:128:

CGGGAGGGGG GGTCCTGGAG GCTGCACGAC 30

(2) INFORMATION FOR SEQ ID NO: 129:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:129:

GGGGGGGTCC TGGAGGCTGC ACGA 24

(2) INFORMATION FOR SEQ ID NO: 130:
(i) SEQUENCE CHARACTERISTICS:
(A) LENGTH: 25 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:130:
   AGGGGGGGTC CTGGAGGCTG CACGA 25

(2) INFORMATION FOR SEQ ID NO: 131:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:131:

GAGGGGGGGT CCTGGAGGCT GCACGA 26

(2) INFORMATION FOR SEQ ID NO: 132:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:132:

GGAGGGGGGG TCCTGGAGGC TGCACGA 27

(2) INFORMATION FOR SEQ ID NO: 133:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:133:

GGGAGGGGGG GTCCTGGAGG CTGCACGA 28

(2) INFORMATION FOR SEQ ID NO: 134:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:134:

CGGGAGGGGG GGTCCTGGAG GCTGCACGA 29

(2) INFORMATION FOR SEQ ID NO: 135:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:135:

CCGGGAGGGG GGGTCCTGGA GGCTGCACGA 30

(2) INFORMATION FOR SEQ ID NO: 136:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:136:

GGGGGGGTCC TGGAGGCTGC ACG 23

(2) INFORMATION FOR SEQ ID NO: 137:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:137:

AGGGGGGGTC CTGGAGGCTG CACG 24

(2) INFORMATION FOR SEQ ID NO: 138:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:138:

GAGGGGGGGT CCTGGAGGCT GCACG 25

(2) INFORMATION FOR SEQ ID NO: 139:
(i) SEQUENCE CHARACTERISTICS:
(A) LENGTH: 26 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:139:

GGAGGGGGGG TCCTGGAGGC TGCACG 26

(2) INFORMATION FOR SEQ ID NO: 140:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:140:

GGGAGGGGGG GTCCTGGAGG CTGCACG 27

(2) INFORMATION FOR SEQ ID NO: 141:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:141:

CGGGAGGGGG GGTCCTGGAG GCTGCACG 28

(2) INFORMATION FOR SEQ ID NO: 142:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:142:

CCGGGAGGGG GGGTCCTGGA GGCTGCACG 29

(2) INFORMATION FOR SEQ ID NO: 143:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:143:

CCCGGGAGGG GGGGTCCTGG AGGCTGCACG 30

(2) INFORMATION FOR SEQ ID NO: 144:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:144:

GGGGGGGTCC TGGAGGCTGC AC 22

(2) INFORMATION FOR SEQ ID NO: 145:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:145:

AGGGGGGGTC CTGGAGGCTG CAC 23

(2) INFORMATION FOR SEQ ID NO: 146:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:146:

GAGGGGGGGT CCTGGAGGCT GCAC 24

(2) INFORMATION FOR SEQ ID NO: 147:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:147:

GGAGGGGGGG TCCTGGAGGC TGCAC 25

(2) INFORMATION FOR SEQ ID NO: 148:
(i) SEQUENCE CHARACTERISTICS:
(A) LENGTH: 26 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:148:

GGGAGGGGGG GTCCTGGAGG CTGCAC 26

(2) INFORMATION FOR SEQ ID NO: 149:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:149:

CGGGAGGGGG GGTCCTGGAG GCTGCAC 27

(2) INFORMATION FOR SEQ ID NO: 150:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:150:

CCGGGAGGGG GGGTCCTGGA GGCTGCAC 28

(2) INFORMATION FOR SEQ ID NO: 151:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:151:

CCCGGGAGGG GGGGTCCTGG AGGCTGCAC 29

(2) INFORMATION FOR SEQ ID NO: 152:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:152:

TCCCGGGAGG GGGGGTCCTG GAGGCTGCAC 30

(2) INFORMATION FOR SEQ ID NO: 153:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:153:

GGGGGGGTCC TGGAGGCTGC A 21

(2) INFORMATION FOR SEQ ID NO: 154:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:154:

AGGGGGGGTC CTGGAGGCTG CA 22

(2) INFORMATION FOR SEQ ID NO: 155:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:155:

GAGGGGGGGT CCTGGAGGCT GCA 23

(2) INFORMATION FOR SEQ ID NO: 156:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:156:

GGAGGGGGGG TCCTGGAGGC TGCA 24

(2) INFORMATION FOR SEQ ID NO: 157:
(i) SEQUENCE CHARACTERISTICS:
(A) LENGTH: 25 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:157:

GGGAGGGGGG GTCCTGGAGG CTGCA 25

(2) INFORMATION FOR SEQ ID NO: 158:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:158:

CGGGAGGGGG GGTCCTGGAG GCTGCA 26

(2) INFORMATION FOR SEQ ID NO: 159:
(i) SEQUENCE CHARACTERISTICS:
(A) LENGTH: 27 base pairs
(B) TYPE: nucleic acid
(C) STRANDEDNESSSS: single
(D) TOPOLOGY: linear
(ii) MOLECULE TYPE: DNA
(iv) ANTI-SENSE: Yes

(xi) SEQUENCE DESCRIPTION: SEQ ID NO:159:

CCGGGAGGGG GGGTCCTGGA GGCTGCA 27

(2) INFORMATION FOR SEQ ID NO: 160:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:160:

CCCGGGAGGG GGGGTCCTGG AGGCTGCA 28

(2) INFORMATION FOR SEQ ID NO: 161:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:161:

TCCCGGGAGG GGGGGTCCTG GAGGCTGCA 29

(2) INFORMATION FOR SEQ ID NO: 162:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:162:

CTCCCGGGAG GGGGGGTCCT GGAGGCTGCA 30

(2) INFORMATION FOR SEQ ID NO: 163:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:163:

GGGGGGGTCC TGGAGGCTGC 20

(2) INFORMATION FOR SEQ ID NO: 164:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:164:

AGGGGGGGTC CTGGAGGCTG C 21

(2) INFORMATION FOR SEQ ID NO: 165:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:165:

GAGGGGGGGT CCTGGAGGCT GC 22

(2) INFORMATION FOR SEQ ID NO: 166:
(i) SEQUENCE CHARACTERISTICS:
(A) LENGTH: 23 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:166:

GGAGGGGGGG TCCTGGAGGC TGC 23

(2) INFORMATION FOR SEQ ID NO: 167:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:167:

GGGAGGGGGG GTCCTGGAGG CTGC 24

(2) INFORMATION FOR SEQ ID NO: 168:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:168:

CGGGAGGGGG GGTCCTGGAG GCTGC 25

(2) INFORMATION FOR SEQ ID NO: 169:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:169:

CCGGGAGGGG GGGTCCTGGA GGCTGC 26

(2) INFORMATION FOR SEQ ID NO: 170:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:170:

CCCGGGAGGG GGGGTCCTGG AGGCTGC 27

(2) INFORMATION FOR SEQ ID NO: 171:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:171:

TCCCGGGAGG GGGGGTCCTG GAGGCTGC 28

(2) INFORMATION FOR SEQ ID NO: 172:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:172:

CTCCCGGGAG GGGGGGTCCT GGAGGCTGC 29

(2) INFORMATION FOR SEQ ID NO: 173:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:173:

TCTCCCGGGA GGGGGGGTCC TGGAGGCTGC 30

(2) INFORMATION FOR SEQ ID NO: 174:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:174:

GGGGGGGTCC TGGAGGCTG 19

(2) INFORMATION FOR SEQ ID NO: 175:
(i) SEQUENCE CHARACTERISTICS:
(A) LENGTH: 20 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:175:

AGGGGGGTC CTGGAGGCTG 20

(2) INFORMATION FOR SEQ ID NO: 176:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:176:

GAGGGGGGT CCTGGAGGCT G 21

(2) INFORMATION FOR SEQ ID NO: 177:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:177:

GGAGGGGGG TCCTGGAGGC TG 22

(2) INFORMATION FOR SEQ ID NO: 178:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:178:

GGGAGGGGG GTCCTGGAGG CTG 23

(2) INFORMATION FOR SEQ ID NO: 179:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:179:

CGGGAGGGGG GGTCCTGGAG GCTG 24

(2) INFORMATION FOR SEQ ID NO: 180:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:180:

CCGGGAGGGG GGGTCCTGGA GGCTG 25

(2) INFORMATION FOR SEQ ID NO: 181:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:181:

CCCGGGAGGG GGGGTCCTGG AGGCTG 26

(2) INFORMATION FOR SEQ ID NO: 182:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:182:

TCCCGGGAGG GGGGGTCCTG GAGGCTG 27

(2) INFORMATION FOR SEQ ID NO: 183:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:183:

CTCCCGGGAG GGGGGGTCCT GGAGGCTG 28

(2) INFORMATION FOR SEQ ID NO: 184:
(i) SEQUENCE CHARACTERISTICS:
(A) LENGTH: 29 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:184:

TCTCCCGGGA GGGGGGTCC TGGAGGCTG 29

(2) INFORMATION FOR SEQ ID NO: 185:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:185:

CTCTCCCGGG AGGGGGGGTC CTGGAGGCTG 30

(2) INFORMATION FOR SEQ ID NO: 186:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:186:

GGGGGGGTCC TGGAGGCT 18

(2) INFORMATION FOR SEQ ID NO: 187:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:187:

AGGGGGGGTC CTGGAGGCT 19

(2) INFORMATION FOR SEQ ID NO: 188:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:188:

GAGGGGGGGT CCTGGAGGCT 20

(2) INFORMATION FOR SEQ ID NO: 189:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:189:

GGAGGGGGGG TCCTGGAGGC T 21

(2) INFORMATION FOR SEQ ID NO: 190:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:190:

GGGAGGGGGG GTCCTGGAGG CT 22

(2) INFORMATION FOR SEQ ID NO: 191:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:191:

CGGGAGGGGG GGTCCTGGAG GCT 23

(2) INFORMATION FOR SEQ ID NO: 192:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:192:

CCGGGAGGGG GGGTCCTGGA GGCT 24

(2) INFORMATION FOR SEQ ID NO: 193:
(i) SEQUENCE CHARACTERISTICS:
(A) LENGTH: 25 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:193:

CCCGGGAGGG GGGGTCCTGG AGGCT 25

(2) INFORMATION FOR SEQ ID NO: 194:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:194:

TCCCGGGAGG GGGGGTCCTG GAGGCT 26

(2) INFORMATION FOR SEQ ID NO: 195:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:195:

CTCCCGGGAG GGGGGGTCCT GGAGGCT 27

(2) INFORMATION FOR SEQ ID NO: 196:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:196:

TCTCCCGGGA GGGGGGGTCC TGGAGGCT 28

(2) INFORMATION FOR SEQ ID NO: 197:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:197:

CTCTCCCGGG AGGGGGGGTC CTGGAGGCT 29

(2) INFORMATION FOR SEQ ID NO: 198:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:198:

GCTCTCCCGG GAGGGGGGGT CCTGGAGGCT 30

(2) INFORMATION FOR SEQ ID NO: 199:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:199:

GGGGGGGTCC TGGAGGC 17

(2) INFORMATION FOR SEQ ID NO: 200:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:200:

AGGGGGGGTC CTGGAGGC 18

(2) INFORMATION FOR SEQ ID NO: 201:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:201:

GAGGGGGGGT CCTGGAGGC 19

(2) INFORMATION FOR SEQ ID NO: 202:
(i) SEQUENCE CHARACTERISTICS:
(A) LENGTH: 20 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:202:

GGAGGGGGG TCCTGGAGGC 20

(2) INFORMATION FOR SEQ ID NO: 203:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:203:

GGGAGGGGGG GTCCTGGAGG C 21

(2) INFORMATION FOR SEQ ID NO: 204:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:204:

CGGGAGGGGG GGTCCTGGAG GC 22

(2) INFORMATION FOR SEQ ID NO: 205:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:205:

CCGGGAGGGG GGGTCCTGGA GGC 23

(2) INFORMATION FOR SEQ ID NO: 206:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:206:

CCCGGGAGGG GGGGTCCTGG AGGC 24

(2) INFORMATION FOR SEQ ID NO: 207:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:207:

TCCCGGGAGG GGGGGTCCTG GAGGC 25

(2) INFORMATION FOR SEQ ID NO: 208:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:208:

CTCCCGGGAG GGGGGGTCCT GGAGGC 26

(2) INFORMATION FOR SEQ ID NO: 209:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:209:

TCTCCCGGGA GGGGGGGTCC TGGAGGC 27

(2) INFORMATION FOR SEQ ID NO: 210:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:210:

CTCTCCCGGG AGGGGGGGTC CTGGAGGC 28

(2) INFORMATION FOR SEQ ID NO: 211:
(i) SEQUENCE CHARACTERISTICS:
(A) LENGTH: 29 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:211:

GCTCTCCCGG GAGGGGGGT CCTGGAGGC 29

(2) INFORMATION FOR SEQ ID NO: 212:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:212:

GGCTCTCCCG GGAGGGGGGG TCCTGGAGGC 30

(2) INFORMATION FOR SEQ ID NO: 213:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:213:

GGGGGGGTCC TGGAGG 16

(2) INFORMATION FOR SEQ ID NO: 214:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:214:

AGGGGGGGTC CTGGAGG 17

(2) INFORMATION FOR SEQ ID NO: 215:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
MOLECULE T: Other nucleic acid(iv) ANTI-SENSE: Yes

(xi) SEQUENCE DESCRIPTION: SEQ ID NO:215:
   GAGGGGGGGT CCTGGAGG 18

(2) INFORMATION FOR SEQ ID NO: 216:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:216:

GGAGGGGGGG TCCTGGAGG 19

(2) INFORMATION FOR SEQ ID NO: 217:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:217:

GGGAGGGGGG GTCCTGGAGG 20

(2) INFORMATION FOR SEQ ID NO: 218:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:218:

CGGGAGGGGG GGTCCTGGAG G 21

(2) INFORMATION FOR SEQ ID NO: 219:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:219:

CCGGGAGGGG GGGTCCTGGA GG 22

(2) INFORMATION FOR SEQ ID NO: 220:
(i) SEQUENCE CHARACTERISTICS:
(A) LENGTH: 23 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:220:

CCCGGGAGGG GGGGTCCTGG AGG 23

(2) INFORMATION FOR SEQ ID NO: 221:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:221:

TCCCGGGAGG GGGGGTCCTG GAGG 24

(2) INFORMATION FOR SEQ ID NO: 222:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:222:

CTCCCGGGAG GGGGGGTCCT GGAGG 25

(2) INFORMATION FOR SEQ ID NO: 223:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:223:

TCTCCCGGGA GGGGGGGTCC TGGAGG 26

(2) INFORMATION FOR SEQ ID NO: 224:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:224:

CTCTCCCGGG AGGGGGGGTC CTGGAGG 27

(2) INFORMATION FOR SEQ ID NO: 225:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:225:

GCTCTCCCGG GAGGGGGGGT CCTGGAGG 28

(2) INFORMATION FOR SEQ ID NO: 226:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:226:

GGCTCTCCCG GGAGGGGGGG TCCTGGAGG 29

(2) INFORMATION FOR SEQ ID NO: 227:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:227:

TGGCTCTCCC GGGAGGGGGG GTCCTGGAGG 30

(2) INFORMATION FOR SEQ ID NO: 228:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:228:

GGGGGGGTCC TGGAG 15

(2) INFORMATION FOR SEQ ID NO: 229:
(i) SEQUENCE CHARACTERISTICS:
(A) LENGTH: 16 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:229:

AGGGGGGTC CTGGAG 16

(2) INFORMATION FOR SEQ ID NO: 230:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:230:

GAGGGGGGT CCTGGAG 17

(2) INFORMATION FOR SEQ ID NO: 231:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:231:

GGAGGGGGG TCCTGGAG 18

(2) INFORMATION FOR SEQ ID NO: 232:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:232:

GGGAGGGGG GTCCTGGAG 19

(2) INFORMATION FOR SEQ ID NO: 233:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:233:

CGGGAGGGGG GGTCCTGGAG 20

(2) INFORMATION FOR SEQ ID NO: 234:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:234:

CCGGGAGGGG GGGTCCTGGA G 21

(2) INFORMATION FOR SEQ ID NO: 235:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:235:

CCCGGGAGGG GGGGTCCTGG AG 22

(2) INFORMATION FOR SEQ ID NO: 236:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:236:

TCCCGGGAGG GGGGGTCCTG GAG 23

(2) INFORMATION FOR SEQ ID NO: 237:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:237:

CTCCCGGGAG GGGGGGTCCT GGAG 24

(2) INFORMATION FOR SEQ ID NO: 238:
(i) SEQUENCE CHARACTERISTICS:
(A) LENGTH: 25 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:238:

TCTCCCGGGA GGGGGGTCC TGGAG 25

(2) INFORMATION FOR SEQ ID NO: 239:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:239:

CTCTCCCGGG AGGGGGGTC CTGGAG 26

(2) INFORMATION FOR SEQ ID NO: 240:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:240:

GCTCTCCCGG GAGGGGGGT CCTGGAG 27

(2) INFORMATION FOR SEQ ID NO: 241:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:241:

GGCTCTCCCG GGAGGGGGG TCCTGGAG 28

(2) INFORMATION FOR SEQ ID NO: 242:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:242:

TGGCTCTCCC GGGAGGGGGG GTCCTGGAG 29

(2) INFORMATION FOR SEQ ID NO: 243:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:243:

ATGGCTCTCC CGGGAGGGGG GGTCCTGGAG 30

(2) INFORMATION FOR SEQ ID NO: 244:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:244:

AGGGGGGGTC CTGGA 15

(2) INFORMATION FOR SEQ ID NO: 245:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:245:

GAGGGGGGGT CCTGGA 16

(2) INFORMATION FOR SEQ ID NO: 246:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:246:

GGAGGGGGGG TCCTGGA 17

(2) INFORMATION FOR SEQ ID NO: 247:
(i) SEQUENCE CHARACTERISTICS:
(A) LENGTH: 18 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:247:

GGGAGGGGGG GTCCTGGA 18

(2) INFORMATION FOR SEQ ID NO: 248:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:248:

CGGGAGGGGG GGTCCTGGA 19

(2) INFORMATION FOR SEQ ID NO: 249:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:249:

CCGGGAGGGG GGGTCCTGGA 20

(2) INFORMATION FOR SEQ ID NO: 250
(i) SEQUENCE CHARACTERISTICS:
(A) LENGTH: 21 base pairs:
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:250:

CCCGGGAGGG GGGGTCCTGG A 21

(2) INFORMATION FOR SEQ ID NO: 251:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:251:

TCCCGGGAGG GGGGGTCCTG GA 22

(2) INFORMATION FOR SEQ ID NO: 252:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:252:

CTCCCGGGAG GGGGGTCCT GGA 23

(2) INFORMATION FOR SEQ ID NO: 253:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:253:

TCTCCCGGGA GGGGGGGTCC TGGA 24

(2) INFORMATION FOR SEQ ID NO: 254:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:254:

CTCTCCCGGG AGGGGGGGTC CTGGA 25

(2) INFORMATION FOR SEQ ID NO: 255:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:255:

GCTCTCCCGG GAGGGGGGGT CCTGGA 26

(2) INFORMATION FOR SEQ ID NO: 256:
(i) SEQUENCE CHARACTERISTICS:
(A) LENGTH: 27 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:256:

GGCTCTCCCG GGAGGGGGGG TCCTGGA 27

(2) INFORMATION FOR SEQ ID NO: 257:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:257:

TGGCTCTCCC GGGAGGGGGG GTCCTGGA 28

(2) INFORMATION FOR SEQ ID NO: 258:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:258:

ATGGCTCTCC CGGGAGGGGG GGTCCTGGA 29

(2) INFORMATION FOR SEQ ID NO: 259:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:259:

TATGGCTCTC CCGGGAGGGG GGGTCCTGGA 30

(2) INFORMATION FOR SEQ ID NO: 260:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:260:

GAGGGGGGGT CCTGG 15

(2) INFORMATION FOR SEQ ID NO: 261:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:261:

GGAGGGGGGG TCCTGG 16

(2) INFORMATION FOR SEQ ID NO: 262:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:262:

GGGAGGGGGG GTCCTGG 17

(2) INFORMATION FOR SEQ ID NO: 263:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:263:

CGGGAGGGGG GGTCCTGG 18

(2) INFORMATION FOR SEQ ID NO: 264:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:264:

CCCGGGAGGG GGGGTCCTGG 20

(2) INFORMATION FOR SEQ ID NO: 265:
(i) SEQUENCE CHARACTERISTICS:
(A) LENGTH: 21 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:265:

TCCCGGGAGG GGGGGTCCTG G 21

(2) INFORMATION FOR SEQ ID NO: 266:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:266:

CTCCCGGGAG GGGGGGTCCT GG 22

(2) INFORMATION FOR SEQ ID NO: 267:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:267:

TCTCCCGGGA GGGGGGGTCC TGG 23

(2) INFORMATION FOR SEQ ID NO: 268:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:268:

CTCTCCCGGG AGGGGGGGTC CTGG 24

(2) INFORMATION FOR SEQ ID NO: 269:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:269:
   GCTCTCCCGG GAGGGGGGGT CCTGG 25

(2) INFORMATION FOR SEQ ID NO: 270:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:270:

GGCTCTCCCG GGAGGGGGGG TCCTGG 26

(2) INFORMATION FOR SEQ ID NO: 271:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:271:

TGGCTCTCCC GGGAGGGGGG GTCCTGG 27

(2) INFORMATION FOR SEQ ID NO: 272:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:272:

ATGGCTCTCC CGGGAGGGGG GGTCCTGG 28

(2) INFORMATION FOR SEQ ID NO: 273:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:273:

TATGGCTCTC CCGGGAGGGG GGGTCCTGG 29

(2) INFORMATION FOR SEQ ID NO: 274:
(i) SEQUENCE CHARACTERISTICS:
(A) LENGTH: 30 base pairs

(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:274:

CTATGGCTCT CCCGGGAGGG GGGGTCCTGG                                        30


(2) INFORMATION FOR SEQ ID NO:  275:
(i) SEQUENCE CHARACTERISTICS:
(A) LENGTH:  15 base pairs
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:275:

GGAGGGGGGG TCCTG                                                        15


(2) INFORMATION FOR SEQ ID NO:  276:
(i) SEQUENCE CHARACTERISTICS:
(A) LENGTH:  16 base pairs
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:276:

GGGAGGGGGG GTCCTG                                                       16


(2) INFORMATION FOR SEQ ID NO:  277:
(i) SEQUENCE CHARACTERISTICS:
(A) LENGTH:  17 base pairs
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:277:

CGGGAGGGGG GGTCCTG                                                      17


(2) INFORMATION FOR SEQ ID NO:  278:
(i) SEQUENCE CHARACTERISTICS:
(A) LENGTH:  18 base pairs
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:278:

CCGGGAGGGG GGGTCCTG 18

(2) INFORMATION FOR SEQ ID NO: 279:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:279:

CCCGGGAGGG GGGGTCCTG 19

(2) INFORMATION FOR SEQ ID NO: 280:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:280:

TCCCGGGAGG GGGGGTCCTG 20

(2) INFORMATION FOR SEQ ID NO: 281:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:281:

CTCCCGGGAG GGGGGGTCCT G 21

(2) INFORMATION FOR SEQ ID NO: 282:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:282:

TCTCCCGGGA GGGGGGGTCC TG 22

(2) INFORMATION FOR SEQ ID NO: 283:
(i) SEQUENCE CHARACTERISTICS:
(A) LENGTH: 23 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:283:

CTCTCCCGGG AGGGGGGTC CTG 23

(2) INFORMATION FOR SEQ ID NO: 284:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:284:

GCTCTCCCGG GAGGGGGGT CCTG 24

(2) INFORMATION FOR SEQ ID NO: 285:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:285:
   GGCTCTCCCG GGAGGGGGGG TCCTG 25

(2) INFORMATION FOR SEQ ID NO: 286:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:286:

TGGCTCTCCC GGGAGGGGGG GTCCTG 26

(2) INFORMATION FOR SEQ ID NO: 287:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:287:

ATGGCTCTCC CGGGAGGGGG GGTCCTG 27

(2) INFORMATION FOR SEQ ID NO: 288:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:288:

TATGGCTCTC CCGGGAGGGG GGGTCCTG 28

(2) INFORMATION FOR SEQ ID NO: 289:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:289:

CTATGGCTCT CCCGGGAGGG GGGGTCCTG 29

(2) INFORMATION FOR SEQ ID NO: 290:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:290:

ACTATGGCTC TCCCGGGAGG GGGGGTCCTG 30

(2) INFORMATION FOR SEQ ID NO: 291:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:291:

GGGAGGGGGG GTCCT 15

(2) INFORMATION FOR SEQ ID NO: 292:
(i) SEQUENCE CHARACTERISTICS:
(A) LENGTH: 16 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:292:

CGGGAGGGGG GGTCCT 16

(2) INFORMATION FOR SEQ ID NO: 293:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:293:

CCGGGAGGGG GGGTCCT 17

(2) INFORMATION FOR SEQ ID NO: 294:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:294:

CCCGGGAGGG GGGGTCCT 18

(2) INFORMATION FOR SEQ ID NO: 295:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:295:

TCCCGGGAGG GGGGGTCCT 19

(2) INFORMATION FOR SEQ ID NO: 296:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:296:

CTCCCGGGAG GGGGGGTCCT 20

(2) INFORMATION FOR SEQ ID NO: 297:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:297:

TCTCCCGGGA GGGGGGGTCC T 21

(2) INFORMATION FOR SEQ ID NO: 298:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:298:

CTCTCCCGGG AGGGGGGGTC CT 22

(2) INFORMATION FOR SEQ ID NO: 299:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:299:

GCTCTCCCGG GAGGGGGGGT CCT 23

(2) INFORMATION FOR SEQ ID NO: 300:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:300:

GGCTCTCCCG GGAGGGGGGG TCCT 24

(2) INFORMATION FOR SEQ ID NO: 301:
(i) SEQUENCE CHARACTERISTICS:
(A) LENGTH: 25 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:301:

TGGCTCTCCC GGGAGGGGGG GTCCT 25

(2) INFORMATION FOR SEQ ID NO: 302:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:302:

ATGGCTCTCC CGGGAGGGGG GGTCCT 26

(2) INFORMATION FOR SEQ ID NO: 303:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:303:

TATGGCTCTC CCGGGAGGGG GGGTCCT 27

(2) INFORMATION FOR SEQ ID NO: 304:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:304:

CTATGGCTCT CCCGGGAGGG GGGGTCCT 28

(2) INFORMATION FOR SEQ ID NO: 305:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:305:

ACTATGGCTC TCCCGGGAGG GGGGGTCCT 29

(2) INFORMATION FOR SEQ ID NO: 306:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:306:

CACTATGGCT CTCCCGGGAG GGGGGGTCCT 30

(2) INFORMATION FOR SEQ ID NO: 307:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:307:

CGGGAGGGGG GGTCC 15

(2) INFORMATION FOR SEQ ID NO: 308:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:308:

CCGGGAGGGG GGGTCC 16

(2) INFORMATION FOR SEQ ID NO: 309:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:309:

CCCGGGAGGG GGGGTCC 17

(2) INFORMATION FOR SEQ ID NO: 310:
(i) SEQUENCE CHARACTERISTICS:
(A) LENGTH: 18 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:310:

TCCCGGGAGG GGGGGTCC 18

(2) INFORMATION FOR SEQ ID NO: 311:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:311:

CTCCCGGGAG GGGGGGTCC 19

(2) INFORMATION FOR SEQ ID NO: 312:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:312:

TCTCCCGGGA GGGGGGGTCC 20

(2) INFORMATION FOR SEQ ID NO: 313:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:313:

CTCTCCCGGG AGGGGGGGTC C 21

(2) INFORMATION FOR SEQ ID NO: 314:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:314:

GCTCTCCCGG GAGGGGGGGT CC 22

(2) INFORMATION FOR SEQ ID NO: 315:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:315:

GGCTCTCCCG GGAGGGGGGG TCC 23

(2) INFORMATION FOR SEQ ID NO: 316:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:316:

TGGCTCTCCC GGGAGGGGGG GTCC 24

(2) INFORMATION FOR SEQ ID NO: 317:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:317:

ATGGCTCTCC CGGGAGGGGG GGTCC 25

(2) INFORMATION FOR SEQ ID NO: 318:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:318:

TATGGCTCTC CCGGGAGGGG GGGTCC 26

(2) INFORMATION FOR SEQ ID NO: 319:
(i) SEQUENCE CHARACTERISTICS:
(A) LENGTH: 27 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:319:

CTATGGCTCT CCCGGGAGGG GGGGTCC 27

(2) INFORMATION FOR SEQ ID NO: 320:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:320:

ACTATGGCTC TCCCGGGAGG GGGGGTCC 28

(2) INFORMATION FOR SEQ ID NO: 321:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:321:

CACTATGGCT CTCCCGGGAG GGGGGGTCC 29

(2) INFORMATION FOR SEQ ID NO: 322:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:322:

CCACTATGGC TCTCCCGGGA GGGGGGGTCC 30

(2) INFORMATION FOR SEQ ID NO: 323:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:323:

CCGGGAGGGG GGGTC 15

(2) INFORMATION FOR SEQ ID NO: 324:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:324:

CCCGGGAGGG GGGGTC 16

(2) INFORMATION FOR SEQ ID NO: 325:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:325:

TCCCGGGAGG GGGGGTC 17

(2) INFORMATION FOR SEQ ID NO: 326:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:326:

CTCCCGGGAG GGGGGGTC 18

(2) INFORMATION FOR SEQ ID NO: 327:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:327:

TCTCCCGGGA GGGGGGGTC 19

(2) INFORMATION FOR SEQ ID NO: 328:
(i) SEQUENCE CHARACTERISTICS:
(A) LENGTH: 20 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:328:

CTCTCCCGGG AGGGGGGGTC 20

(2) INFORMATION FOR SEQ ID NO: 329:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:329:

GCTCTCCCGG GAGGGGGGGT C 21

(2) INFORMATION FOR SEQ ID NO: 330:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:330:

GGCTCTCCCG GGAGGGGGGG TC 22

(2) INFORMATION FOR SEQ ID NO: 331:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:331:

TGGCTCTCCC GGGAGGGGGG GTC 23

(2) INFORMATION FOR SEQ ID NO: 332:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:332:

ATGGCTCTCC CGGGAGGGGG GGTC 24

(2) INFORMATION FOR SEQ ID NO: 333:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:333:

TATGGCTCTC CCGGGAGGGG GGGTC 25

(2) INFORMATION FOR SEQ ID NO: 334:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:334:

CTATGGCTCT CCCGGGAGGG GGGGTC 26

(2) INFORMATION FOR SEQ ID NO: 335:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:335:

ACTATGGCTC TCCCGGGAGG GGGGGTC 27

(2) INFORMATION FOR SEQ ID NO: 336:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:336:

CACTATGGCT CTCCCGGGAG GGGGGGTC 28

(2) INFORMATION FOR SEQ ID NO: 337:
(i) SEQUENCE CHARACTERISTICS:
(A) LENGTH: 29 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:337:

CCACTATGGC TCTCCCGGGA GGGGGGGTC 29

(2) INFORMATION FOR SEQ ID NO: 338:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:338:

ACCACTATGG CTCTCCCGGG AGGGGGGGTC 30

(2) INFORMATION FOR SEQ ID NO: 339:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:339:

CCCGGGAGGG GGGGT 15

(2) INFORMATION FOR SEQ ID NO: 340:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:340:

TCCCGGGAGG GGGGGT 16

(2) INFORMATION FOR SEQ ID NO: 341:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:341:

CTCCCGGGAG GGGGGGT 17

(2) INFORMATION FOR SEQ ID NO: 342:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:342:

TCTCCCGGGA GGGGGGGT 18

(2) INFORMATION FOR SEQ ID NO: 343:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:343:

CTCTCCCGGG AGGGGGGGT 19

(2) INFORMATION FOR SEQ ID NO: 344:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:344:

GCTCTCCCGG GAGGGGGGGT 20

(2) INFORMATION FOR SEQ ID NO: 345:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:345:

GGCTCTCCCG GGAGGGGGGG T 21

(2) INFORMATION FOR SEQ ID NO: 346:
(i) SEQUENCE CHARACTERISTICS:
(A) LENGTH: 22 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:346:

TGGCTCTCCC GGGAGGGGGG GT 22

(2) INFORMATION FOR SEQ ID NO: 347:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:347:

ATGGCTCTCC CGGGAGGGGG GGT 23

(2) INFORMATION FOR SEQ ID NO: 348:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:348:

TATGGCTCTC CCGGGAGGGG GGGT 24

(2) INFORMATION FOR SEQ ID NO: 349:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:349:

CTATGGCTCT CCCGGGAGGG GGGGT 25

(2) INFORMATION FOR SEQ ID NO: 350:
(i) SEQUENCE CHARACTERISTICS:
(A) LENGTH: 26 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:350:

ACTATGGCTC TCCCGGGAGG GGGGGT 26

(2) INFORMATION FOR SEQ ID NO: 351:
(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:351:

CACTATGGCT CTCCCGGGAG GGGGGGT 27

(2) INFORMATION FOR SEQ ID NO: 352:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:352:

CCACTATGGC TCTCCCGGGA GGGGGGGT 28

(2) INFORMATION FOR SEQ ID NO: 353:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:353:

ACCACTATGG CTCTCCCGGG AGGGGGGGT 29

(2) INFORMATION FOR SEQ ID NO: 354:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:354:

GACCACTATG GCTCTCCCGG GAGGGGGGGT 30

(2) INFORMATION FOR SEQ ID NO: 355:
(i) SEQUENCE CHARACTERISTICS:
(A) LENGTH: 15 base pairs

(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:355:

TCCCGGGAGG GGGGG 15

(2) INFORMATION FOR SEQ ID NO: 356:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:356:

CTCCCGGGAG GGGGGG 16

(2) INFORMATION FOR SEQ ID NO: 357:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:357:

TCTCCCGGGA GGGGGGG 17

(2) INFORMATION FOR SEQ ID NO: 358:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:358:

CTCTCCCGGG AGGGGGGG 18

(2) INFORMATION FOR SEQ ID NO: 359:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:359:

GCTCTCCCGG GAGGGGGGG 19

(2) INFORMATION FOR SEQ ID NO: 360:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:360:

GGCTCTCCCG GGAGGGGGGG 20

(2) INFORMATION FOR SEQ ID NO: 361:
(i) SEQUENCE CHARACTERISTICS:
(A) LENGTH: 21 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:361:

TGGCTCTCCC GGGAGGGGGG G 21

(2) INFORMATION FOR SEQ ID NO: 362:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:362:

ATGGCTCTCC CGGGAGGGGG GG 22

(2) INFORMATION FOR SEQ ID NO: 363:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:363:

TATGGCTCTC CCGGGAGGGG GGG 23

(2) INFORMATION FOR SEQ ID NO: 364:
(i) SEQUENCE CHARACTERISTICS:
(A) LENGTH: 24 base pairs

(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:364:

CTATGGCTCT CCCGGGAGGG GGGG                                        24


(2) INFORMATION FOR SEQ ID NO:  365:
(i) SEQUENCE CHARACTERISTICS:
(A) LENGTH:  25 base pairs
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:365:

ACTATGGCTC TCCCGGGAGG GGGGG                                       25


(2) INFORMATION FOR SEQ ID NO:  366:
(i) SEQUENCE CHARACTERISTICS:
(A) LENGTH:  26 base pairs
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:366:

CACTATGGCT CTCCCGGGAG GGGGGG                                      26


(2) INFORMATION FOR SEQ ID NO:  367:
(i) SEQUENCE CHARACTERISTICS:
(A) LENGTH:  27 base pairs
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:367:

CCACTATGGC TCTCCCGGGA GGGGGGG                                     27


(2) INFORMATION FOR SEQ ID NO:  368
(i) SEQUENCE CHARACTERISTICS:
(A) LENGTH:  28 base pairs:
(B) TYPE:  nucleic acid
(C) STRANDEDNESS:  single
(D) TOPOLOGY:  linear
(ii) MOLECULE TYPE:  DNA
(iv) ANTI-SENSE:  Yes

(xi) SEQUENCE DESCRIPTION: SEQ ID NO:368:

ACCACTATGG CTCTCCCGGG AGGGGGGG 28

(2) INFORMATION FOR SEQ ID NO: 369:
(i) SEQUENCE CHARACTERISTICS:
(A) LENGTH: 29 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:369:

GACCACTATG GCTCTCCCGG GAGGGGGGG 29

(2) INFORMATION FOR SEQ ID NO: 370:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:370:

GGGGGTCCTG GAGGCTGCAC 20

(2) INFORMATION FOR SEQ ID NO: 371:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:371:

CCCGGACGCC ATGCG 15

(2) INFORMATION FOR SEQ ID NO: 372:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:372:

CCCGGACGCC ATGCGCT 17

(2) INFORMATION FOR SEQ ID NO: 373:
(i) SEQUENCE CHARACTERISTICS:
(A) LENGTH: 20 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:373:

CCCGGACGCC ATGCGCTAGA 20

(2) INFORMATION FOR SEQ ID NO: 374:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:374:

CCCGGACGCC ATGCGCTAGA GCCCT 25

(2) INFORMATION FOR SEQ ID NO: 375:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:375:

CCCGGACGCC ATGCGCTAGA GCCCTGGCAG 30

(2) INFORMATION FOR SEQ ID NO: 376:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:376:

GAACCCGGAC GCCATG 16

(2) INFORMATION FOR SEQ ID NO: 377:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:377:

GAACCCGGAC GCCATGCGCT 20

(2) INFORMATION FOR SEQ ID NO: 378:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:378:

GAACCCGGAC GCCATGCGCT AGAGC 25

(2) INFORMATION FOR SEQ ID NO: 379:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:379:

GAACCCGGAC GCCATGCGCT AGAGCCCTGG 30

(2) INFORMATION FOR SEQ ID NO: 380:
(i) SEQUENCE CHARACTERISTICS:
(A) LENGTH: 15 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:380:

CAGAACCCGG ACGCC 15

(2) INFORMATION FOR SEQ ID NO: 381:
(i) SEQUENCE CHARACTERISTICS:
(A) LENGTH: 18 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:381:

CAGAACCCGG ACGCCATG 18

(2) INFORMATION FOR SEQ ID NO: 382:
(i) SEQUENCE CHARACTERISTICS:
(A) LENGTH: 20 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:382:

CAGAACCCGG ACGCCATGCG 20

(2) INFORMATION FOR SEQ ID NO: 383:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:383:

CAGAACCCGG ACGCCATGCG CTAGA 25

(2) INFORMATION FOR SEQ ID NO: 384:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:384:

CAGAACCCGG ACGCCATGCG CTAGAGCCCT 30

(2) INFORMATION FOR SEQ ID NO: 385:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:385:

CGTCCTCCAG AACCCGGACG 20

(2) INFORMATION FOR SEQ ID NO: 386:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:386:

CGTCCTCCAG AACCCGGACG CCATG 25

(2) INFORMATION FOR SEQ ID NO: 387:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:387:

CGTCCTCCAG AACCCGGACG CCATGCGCTA 30

(2) INFORMATION FOR SEQ ID NO: 388:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:388:

CACGCCGTCC TCCAGAACCC GGACG 25

(2) INFORMATION FOR SEQ ID NO: 389:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:389:

CACGCCGTCC TCCAGAACCC GGACGCCATG 30

(2) INFORMATION FOR SEQ ID NO: 390:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:390:

TAGTTCACGC CGTCCTCCAG AACCCGGACG 30

(2) INFORMATION FOR SEQ ID NO: 391:
(i) SEQUENCE CHARACTERISTICS:
(A) LENGTH: 20 base pairs
(B) TYPE: nucleic acid

(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:391:

GCGGGGGCAC GCCCAAATCT 20

(2) INFORMATION FOR SEQ ID NO: 392:
(i) SEQUENCE CHARACTERISTICS:
(A) LENGTH: 19 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:392:

AAGGGTGGGG GGGAAACGG 19

(2) INFORMATION FOR SEQ ID NO: 393:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:393:

TGTGTTCTCC ATGTTCGGTG 20

(2) INFORMATION FOR SEQ ID NO: 394:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:394:

GTCAATGTCC ATGCCCCAAA 20

(2) INFORMATION FOR SEQ ID NO: 395:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:395:

GCGAGACTGC TAGCCGAGTG 20

(2) INFORMATION FOR SEQ ID NO: 396:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:396:

CCTCCAGAGC ATCTGGCACG 20

(2) INFORMATION FOR SEQ ID NO: 397:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:397:

GCGAGACTGC TAGCCG 16

(2) INFORMATION FOR SEQ ID NO: 398:
(i) SEQUENCE CHARACTERISTICS:
(A) LENGTH: 20 base pairs
(B) TYPE: nucleic acid
(C) STRANDEDNESS: single
(D) TOPOLOGY: linear
(ii) MOLECULE TYPE: DNA
(iv) ANTI-SENSE: Yes

(xi) SEQUENCE DESCRIPTION: SEQ ID NO:398:

CATCACAACC CAGCGCTTTC 20

(2) INFORMATION FOR SEQ ID NO: 399:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:399:

GTAAAACGAC GGCCAGT 17

(2) INFORMATION FOR SEQ ID NO: 400:
(i) SEQUENCE CHARACTERISTICS:
(A) LENGTH: 17 base pairs
(B) TYPE: nucleic acid
(C) STRANDEDNESS: single

(D) TOPOLOGY: linear
(ii) MOLECULE TYPE: DNA

(xi) SEQUENCE DESCRIPTION: SEQ ID NO:400:

CAGGAAACAG CTATGAC 17

(2) INFORMATION FOR SEQ ID NO: 401:
(i) SEQUENCE CHARACTERISTICS:
(A) LENGTH: 16 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:401:

CAGATCTGCA AGCTTG 16

(2) INFORMATION FOR SEQ ID NO: 402:
(i) SEQUENCE CHARACTERISTICS:
(A) LENGTH: 17 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:402:

CAGGAAACAG CTATGAC 17

(2) INFORMATION FOR SEQ ID NO: 403:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:403:

GAATAGTTTT TCAATTTTTA CG 22

(2) INFORMATION FOR SEQ ID NO: 404:
(i) SEQUENCE CHARACTERISTICS:
(A) LENGTH: 22 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:404:

CGTAAAAATT GAAAAACTAT TC 22

(2) INFORMATION FOR SEQ ID NO: 405:
(i) SEQUENCE CHARACTERISTICS:
(A) LENGTH: 17 base pairs

(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:405:

GTAAAACGAC GGCCAGT 17

(2) INFORMATION FOR SEQ ID NO: 406:
(i) SEQUENCE CHARACTERISTICS:
(A) LENGTH: 40 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:406:

GATCCAAAAA TTGAAAAACT AGTCTAATTT ATTGCACGGA 40

(2) INFORMATION FOR SEQ ID NO: 407:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:407:

GAAGCTTGCC AGCCCCCTGA TGGG 24

(2) INFORMATION FOR SEQ ID NO: 408:
(i) SEQUENCE CHARACTERISTICS:
(A) LENGTH: 28 base pairs
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO:408:

CCGGATCCCG GAAGCTGGGA TGGTCAAC 28

(2) INFORMATION FOR SEQ ID NO: 409:
(i) SEQUENCE CHARACTERISTICS:
(A) LENGTH: 2360 base pairs
(B) TYPE: nucleic acid
(C) STRANDEDNESS: double
(D) TOPOLOGY: linear
(ii) MOLECULE TYPE: DNA
(vi) ORIGINAL SOURCE
(A) ORGANISM: Vaccinia virus
(vii) IMMEDIATE SOURCE
(B) CLONE: pHASE

(xi) SEQUENCE DESCRIPTION: SEQ ID NO:409:

TCGACGATTG TTCATGATGG CAAGATTTAT ATATCTGGAG GTTACAACAA TAGTAGTGTA 60
GTTAATGTAA TATCGAATCT AGTCCTTAGC TATAATCCGA TATATGATGA ATGGACCAAA 120
TTATCATCAT TAAACATTCC TAGAATTAAT CCCGCTCTAT GGTCAGCGCA TAATAAATTA 180
TATGTAGGAG GAGGAATATC TGATGATGTT CGAACTAATA CATCTGAAAC ATACGATAAA 240
GAAAAAGATT GTTGGACATT GGATAATGGT CACGTGTTAC CACGCAATTA TATAATGTAT 300
AAATGCGAAC CGATTAAACA TAAATATCCA TTGGAAAAAA CACAGTACAC GAATGATTTT 360
CTAAAGTATT TGGAAAGTTT TATAGGTAGT TGATAGAACA AAATACATAA TTTTGTAAAA 420
ATAAATCACT TTTTATACTA ATATGACACG ATTACCAATA CTTTTGTTAC TAATATCATT 480
AGTATACGCT ACACCTTTTC CTCAGACATC TAAAAAAATA GGTGATGATG CAACTCTATC 540
ATGTAATCGA AATAATACAA ATGACTACGT TGTTATGAGT GCTTGGTATA AGGAGCCCAA 600
TTCCATTATT CTTTTAGCTG CTAAAAGCGA CGTCTTGTAT TTTGATAATT ATACCAAGGA 660
TAAAATATCT TACGACTCTC CATACGATGA TCTAGTTACA ACTATCACAA TTAAATCATT 720
GACTGCTAGA GATGCCGGTA CTTATGTATG TGCATTCTTT ATGACATCAA CTACAAATGA 780
CACTGATAAA GTAGATTATG AAGAATACTC CACAGAGTTG ATTGTAAATA CAGATAGTGA 840
ATCGACTATA GACATAATAC TATCTGGATC TACACATTCA CCAGAAACTA GCTAGTTCTG 900
AGAAACCAGA GGATATAGAT AATTTTAATT GCTCGTCGGT ATTCGAAATC GGGTCGACAT 960
CTATATACTA TATAGTAATA CCAATACTCA AGACTACGAA ACTGATACAA TCTCTTATCA 1020
TGTGGGTAAT GTTCTCGATG TCGATAGCCA TATGCCCGGT AGTTGCGATA TACATAAACT 1080
GATCACTAAT TCCAAACCCA CCCACTTTTT ATAGTAAGTT TTTCACCCAT AAATAATAAA 1140
TACAATAATT AATTTCTCGT AAAAATTGAA AAACTATTCT AATTTATTGC ACGGTAAGGA 1200
AGTAGAATCA TAAAGAACAG TGACTCTAGA GGATCCAAAA ATTGAAAAAC TAGTCTAATT 1260
TATTGCACGG AGATCCAAAA ATTGAAAAAC TAGTCTAATT TATTGCACGG AGATCCAAAA 1320
ATTGAAAAAC TAGTCTAATT TATTGCACGG AGATCCAAAA ATTGAAAAAC TAGTCTAATT 1380
TATTGCACGG AGATCCAAAA ATTGAAAAAC TAGTCTAATT TATTGCACGG AGATCCAAAA 1440
ATTGAAAAAC TAGTCTAATT TATTGCACGG AGATCTGCAA GCTTGGGGTA CCGAGCTCGA 1500
ATTCGACTCC GGAACCAATT ACTGATAATG TAGAAGATCA TACAGACACC GTCACATACA 1560
CTAGCTAGTG ATAGCATTAA TACAGTAAGT GCATCATCTG GAGAATCCAC AACAGACGAG 1620
ACTCCGGAAC CAATTACTGA TAAAGAAGAA GATCATACAG TCACAGACAC TGTCTCATAC 1680
ACTACAGTAA GTACATCATC TGGAATTGTC ACTACTAAAT CAACCACCGA TGATGCGGAT 1740
CTTTATGATA CGTACAATGA TAATGATACA GTACCACCAA CTACTGTAGG CGGTAGTACA 1800
ACCTCTATTA GCAATTATAA AACCAAGGAC TTTGTAGAAA TATTTGGTAT TACCGCATTA 1860
ATTATATTGT CGGCCGTGGC AATATTCTGT ATTACATATT ATATATATAA TAAACGTTCA 1920
CGTAAATACA AAACAGAGAA CAAAGTCTAG ATTTTTGACT TACATAAATG TCTGGGATAG 1980
TAAAATCTAT CATATTGAGC GGACCATCTG GTTCAGGAAA GACAGCCATA GCCAAAAGAC 2040
TATGGGAATA TATTTGGATT TGTGGTGTCC CATACCACTA GATTTCCTCG TCCTATGGAA 2100
CGAGAAGGTG TCGATTACCA TTACGTTAAC AGAGAGGCCA TCTGGAAGGG AATAGCCGCC 2160
GGAAACTTTC TAGAACATAC TGAGTTTTTA GGAAATATTT ACGGAACTTC TAAAACTGCT 2220
GTGAATACAG CGGCTATTAA TAATCGTATT TGTGTGATGG ATCTAAACAT CGATGGCGTT 2280
AGAAGTCTTA AAAATACGTA CCTAATGCCT TACTCGGTGT ATATAAGACC TACCTCTCTT 2340
AAAATGGTTG AGACCAAGCT 2360

(2) INFORMATION FOR SEQ ID NO: 410:
(i) SEQUENCE CHARACTERISTICS:
(A) LENGTH: 4987 base pairs
(B) TYPE: nucleic acid
(C) STRANDEDNESS: double
(D) TOPOLOGY: linear
(ii) MOLECULE TYPE: DNA
(vi) ORIGINAL SOURCE
(A) ORGANISM: Vaccinia virus, Hepatitis C virus, Firefly luciferase gene
(vii) IMMEDIATE SOURCE
(B) CLONE: pHA5CL

(xi) SEQUENCE DESCRIPTION: SEQ ID NO:410:

TCGACGATTG TTCATGATGG CAAGATTTAT ATATCTGGAG GTTACAACAA TAGTAGTGTA 60
GTTAATGTAA TATCGAATCT AGTCCTTAGC TATAATCCGA TATATGATGA ATGGACCAAA 120
TTATCATCAT TAAACATTCC TAGAATTAAT CCCGCTCTAT GGTCAGCGCA TAATAAATTA 180
TATGTAGGAG GAGGAATATC TGATGATGTT CGAACTAATA CATCTGAAAC ATACGATAAA 240
GAAAAAGATT GTTGGACATT GGATAATGGT CACGTGTTAC CACGCAATTA TATAATGTAT 300
AAATGCGAAC CGATTAAACA TAAATATCCA TTGGAAAAAA CACAGTACAC GAATGATTTT 360
CTAAAGTATT TGGAAAGTTT TATAGGTAGT TGATAGAACA AAATACATAA TTTTGTAAAA 420

ATAAATCACT TTTTATACTA ATATGACACG ATTACCAATA CTTTTGTTAC TAATATCATT 480
AGTATACGCT ACACCTTTTC CTCAGACATC TAAAAAAATA GGTGATGATG CAACTCTATC 540
ATGTAATCGA AATAATACAA ATGACTACGT TGTTATGAGT GCTTGGTATA AGGAGCCCAA 600
TTCCATTATT CTTTTAGCTG CTAAAAGCGA CGTCTTGTAT TTTGATAATT ATACCAAGGA 660
TAAAATATCT TACGACTCTC CATACGATGA TCTAGTTACA ACTATCACAA TTAAATCATT 720
GACTGCTAGA GATGCCGGTA CTTATGTATG TGCATTCTTT ATGACATCAA CTACAAATGA 780
CACTGATAAA GTAGATTATG AAGAATACTC CACAGAGTTG ATTGTAAATA CAGATAGTGA 840
ATCGACTATA GACATAATAC TATCTGGATC TACACATTCA CCAGAAACTA GCTAGTTCTG 900
AGAAACCAGA GGATATAGAT AATTTTAATT GCTCGTCGGT ATTCGAAATC GGGTCGACAT 960
CTATATACTA TATAGTAATA CCAATACTCA AGACTACGAA ACTGATACAA TCTCTTATCA 1020
TGTGGGTAAT GTTCTCGATG TCGATAGCCA TATGCCCGGT AGTTGCGATA TACATAAACT 1080
GATCACTAAT TCCAAACCCA CCCACTTTTT ATAGTAAGTT TTTCACCCAT AAATAATAAA 1140
TACAATAATT AATTTCTCGT AAAAATTGAA AAACTATTCT AATTTATTGC ACGGTAAGGA 1200
AGTAGAATCA TAAAGAACAG TGACTCTAGA GGATCCAAAA ATTGAAAAAC TAGTCTAATT 1260
TATTGCACGG AGATCCAAAA ATTGAAAAAC TAGTCTAATT TATTGCACGG AGATCCAAAA 1320
ATTGAAAAAC TAGTCTAATT TATTGCACGG AGATCCAAAA ATTGAAAAAC TAGTCTAATT 1380
TATTGCACGG AGATCCAAAA ATTGAAAAAC TAGTCTAATT TATTGCACGG AGATCCAAAA 1440
ATTGAAAAAC TAGTCTAATT TATTGCACGG AGATCTGCAA GCTTGCCAGC CCCCTGATGG 1500
GGGCGACACT CCACCATAGA TCACTCCCCT GTGAGGAACT ACTGTCTTCA CGCAGAAAGC 1560
GTCTAGCCAT GGCGTTAGTA TGAGTGTCGT GCAGCCTCCA GGACCCCCCC TCCCGGGAGA 1620
GCCATAGTGG TCTGCGGAAC CGGTGAGTAC ACCGGAATTG CCAGGACGAC CGGGTCCTTT 1680
CTTGGATCAA CCCGCTCAAT GCCTGGAGAT TTGGGCGTGC CCCCGCGAGA CTGCTAGCCG 1740
AGTAGTGTTG GGTCGCGAAA GGCCTTGTGG TACTGCCTGA TAGGGTGCTT GCGAGTGCCC 1800
CGGGAGGTCT CGTAGACCGT GCATC ATG AGC ACA AAT CCA AAA CCC CAA AGA 1852
Met Ser Thr Asn Pro Lys Pro Gln Arg
1               5
AAA ATC AAA CGT AAC ACC AAC CGC CGC CCA CAG GAC GTT AAG TTC CCG 1900
Lys Ile Lys Arg Asn Thr Asn Arg Arg Pro Gln Asp Val Lys Phe Pro
10                  15                  20                  25
GGC GGT GGT CAG ATC GTT GGT GGA GTT TAC CTG TTG CCG CGC AGG GGC 1948
Gly Gly Gly Gln Ile Val Gly Gly Val Tyr Leu Leu Pro Arg Arg Gly
                30                  35                  40
CCC AGG TTG GGT GTG CGC GCG ACT AGG AAG ACT TCC GAG CGG CCG CAA 1996
Pro Arg Leu Gly Val Arg Ala Thr Arg Lys Thr Ser Glu Arg Pro Gln
            45                  50                  55
CCT CGT GGA AGG CGA CAA CCT ATC CCC AAG GCT CGC CAA CCC GAG GGT 2044
Pro Arg Gly Arg Arg Gln Pro Ile Pro Lys Ala Arg Gln Pro Glu Gly
        60                  65                  70
AGG GCC TGG GCT CAG CCC GGG TAC CCT TGG CCC CTC TAT GGC AAT GAG 2092
Arg Ala Trp Ala Gln Pro Gly Tyr Pro Trp Pro Leu Tyr Gly Asn Glu
    75                  80                  85
GGC TTG GGG TGG GCA GGA TGG CTC CTG TCA CCC CGC GGC TCC CGG CCT 2140
Gly Leu Gly Trp Ala Gly Trp Leu Leu Ser Pro Arg Gly Ser Arg Pro
90                  95                  100                 105
AGT TGG GGC CCC ACG GAC CCC CGG CGT AGG TCG CGT AAT TTG GGT AAG 2188
Ser Trp Gly Pro Thr Asp Pro Arg Arg Arg Ser Arg Asn Leu Gly Lys
                110                 115                 120
GTC ATC GAT ACC CTC ACA TGC GGC TTC GCC GAC CTC ATG GGG TAC ATT 2236
Val Ile Asp Thr Leu Thr Cys Gly Phe Ala Asp Leu Met Gly Tyr Ile
            125                 130                 135
CCG CTC GTC GGC GCC CCC CTA GGG GGC GCT GCC AGG GCT CTA GCG CAT 2284
Pro Leu Val Gly Ala Pro Leu Gly Gly Ala Ala Arg Ala Leu Ala His
        140                 145                 150
GGC GTC CGG GTT CTG GAG GAC GGC GTG AAC TAT GCA ACA GGG AAT CTG 2332
Gly Val Arg Val Leu Glu Asp Gly Val Asn Tyr Ala Thr Gly Asn Leu
    155                 160                 165
CCT GGT TGC TCC TTT TCT ATC TTC CTT TTG GCT TTG CTG TCC TGT TTG 2380
Pro Gly Cys Ser Phe Ser Ile Phe Leu Leu Ala Leu Leu Ser Cys Leu
170                 175                 180                 185
ACC ATC CCA GCT TCC GGG ATC CAA ATG GAA GAC GCC AAA AAC ATA AAG 2428
Thr Ile Pro Ala Ser Gly Ile Gln Met Glu Asp Ala Lys Asn Ile Lys
                190                 195                 200
AAA GGC CCG GCG CCA TTC TAT CCT CTA GAG GAT GGA ACC GCT GGA GAG 2476

Lys Gly Pro Ala Pro Phe Tyr Pro Leu Glu Asp Gly Thr Ala Gly Glu
            205                 210                 215
CAA CTG CAT AAG GCT ATG AAG AGA TAC GCC CTG GTT CCT GGA ACA ATT 2524
Gln Leu His Lys Ala Met Lys Arg Tyr Ala Leu Val Pro Gly Thr Ile
        220                 225                 230
GCT TTT ACA GAT GCA CAT ATC GAG GTG AAC ATC ACG TAC GCG GAA TAC 2572
Ala Phe Thr Asp Ala His Ile Glu Val Asn Ile Thr Tyr Ala Glu Tyr
    235                 240                 245
TTC GAA ATG TCC GTT CGG TTG GCA GAA GCT ATG AAA CGA TAT GGG CTG 2620
Phe Glu Met Ser Val Arg Leu Ala Glu Ala Met Lys Arg Tyr Gly Leu
250                 255                 260                 265
AAT ACA AAT CAC AGA ATC GTC GTA TGC AGT GAA AAC TCT CTT CAA TTC 2668
Asn Thr Asn His Arg Ile Val Val Cys Ser Glu Asn Ser Leu Gln Phe
                270                 275                 280
TTT ATG CCG GTG TTG GGC GCG TTA TTT ATC GGA GTT GCA GTT GCG CCC 2716
Phe Met Pro Val Leu Gly Ala Leu Phe Ile Gly Val Ala Val Ala Pro
            285                 290                 295
GCG AAC GAC ATT TAT AAT GAA CGT GAA TTG CTC AAC AGT ATG AAC ATT 2764
Ala Asn Asp Ile Tyr Asn Glu Arg Glu Leu Leu Asn Ser Met Asn Ile
        300                 305                 310
TCG CAG CCT ACC GTA GTG TTT GTT TCC AAA AAG GGG TTG CAA AAA ATT 2812
Ser Gln Pro Thr Val Val Phe Val Ser Lys Lys Gly Leu Gln Lys Ile
    315                 320                 325
TTG AAC GTG CAA AAA AAA TTA CCA ATA ATC CAG AAA ATT ATT ATC ATG 2860
Leu Asn Val Gln Lys Lys Leu Pro Ile Ile Gln Lys Ile Ile Ile Met
330                 335                 340                 345
GAT TCT AAA ACG GAT TAC CAG GGA TTT CAG TCG ATG TAC ACG TTC GTC 2908
Asp Ser Lys Thr Asp Tyr Gln Gly Phe Gln Ser Met Tyr Thr Phe Val
                350                 355                 360
ACA TCT CAT CTA CCT CCC GGT TTT AAT GAA TAC GAT TTT GTA CCA GAG 2956
Thr Ser His Leu Pro Pro Gly Phe Asn Glu Tyr Asp Phe Val Pro Glu
            365                 370                 375
TCC TTT GAT CGT GAC AAA ACA ATT GCA CTG ATA ATG AAT TCC TCT GGA 3004
Ser Phe Asp Arg Asp Lys Thr Ile Ala Leu Ile Met Asn Ser Ser Gly
        380                 385                 390
TCT ACT GGG TTA CCT AAG GGT GTG GCC CTT CCG CAT AGA ACT GCC TGC 3052
Ser Thr Gly Leu Pro Lys Gly Val Ala Leu Pro His Arg Thr Ala Cys
    395                 400                 405
GTC AGA TTC TCG CAT GCC AGA GAT CCT ATT TTT GGC AAT CAA ATC ATT 3100
Val Arg Phe Ser His Ala Arg Asp Pro Ile Phe Gly Asn Gln Ile Ile
410                 415                 420                 425
CCG GAT ACT GCG ATT TTA AGT GTT GTT CCA TTC CAT CAC GGT TTT GGA 3148
Pro Asp Thr Ala Ile Leu Ser Val Val Pro Phe His His Gly Phe Gly
                430                 435                 440
ATG TTT ACT ACA CTC GGA TAT TTG ATA TGT GGA TTT CGA GTC GTC TTA 3196
Met Phe Thr Thr Leu Gly Tyr Leu Ile Cys Gly Phe Arg Val Val Leu
            445                 450                 455
ATG TAT AGA TTT GAA GAA GAG CTG TTT TTA CGA TCC CTT CAG GAT TAC 3244
Met Tyr Arg Phe Glu Glu Glu Leu Phe Leu Arg Ser Leu Gln Asp Tyr
        460                 465                 470
AAA ATT CAA AGT GCG TTG CTA GTA CCA ACC CTA TTT TCA TTC TTC GCC 3292
Lys Ile Gln Ser Ala Leu Leu Val Pro Thr Leu Phe Ser Phe Phe Ala
    475                 480                 485
AAA AGC ACT CTG ATT GAC AAA TAC GAT TTA TCT AAT TTA CAC GAA ATT 3340
Lys Ser Thr Leu Ile Asp Lys Tyr Asp Leu Ser Asn Leu His Glu Ile
490                 495                 500                 505
GCT TCT GGG GGC GCA CCT CTT TCG AAA GAA GTC GGG GAA GCG GTT GCA 3388
Ala Ser Gly Gly Ala Pro Leu Ser Lys Glu Val Gly Glu Ala Val Ala
                510                 515                 520
AAA CGC TTC CAT CTT CCA GGG ATA CGA CAA GGA TAT GGG CTC ACT GAG 3436
Lys Arg Phe His Leu Pro Gly Ile Arg Gln Gly Tyr Gly Leu Thr Glu
            525                 530                 535
ACT ACA TCA GCT ATT CTG ATT ACA CCC GAG GGG GAT GAT AAA CCG GGC 3484

Thr Thr Ser Ala Ile Leu Ile Thr Pro Glu Gly Asp Asp Lys Pro Gly
        540                 545                 550
GCG GTC GGT AAA GTT GTT CCA TTT TTT GAA GCG AAG GTT GTG GAT CTG     3532
Ala Val Gly Lys Val Val Pro Phe Phe Glu Ala Lys Val Val Asp Leu
    555                 560                 565
GAT ACC GGG AAA ACG CTG GGC GTT AAT CAG AGA GGC GAA TTA TGT GTC     3580
Asp Thr Gly Lys Thr Leu Gly Val Asn Gln Arg Gly Glu Leu Cys Val
570                 575                 580                 585
AGA GGA CCT ATG ATT ATG TCC GGT TAT GTA AAC AAT CCG GAA GCG ACC     3628
Arg Gly Pro Met Ile Met Ser Gly Tyr Val Asn Asn Pro Glu Ala Thr
                590                 595                 600
AAC GCC TTG ATT GAC AAG GAT GGA TGG CTA CAT TCT GGA GAC ATA GCT     3676
Asn Ala Leu Ile Asp Lys Asp Gly Trp Leu His Ser Gly Asp Ile Ala
            605                 610                 615
TAC TGG GAC GAA GAC GAA CAC TTC TTC ATA GTT GAC CTC TTG AAG TCT     3724
Tyr Trp Asp Glu Asp Glu His Phe Phe Ile Val Asp Leu Leu Lys Ser
        620                 625                 630
TTA ATT AAA TAC AAA GGA TAT CAG GTG GCC CCC GCT GAA TTG GAA TCG     3772
Leu Ile Lys Tyr Lys Gly Tyr Gln Val Ala Pro Ala Glu Leu Glu Ser
    635                 640                 645
ATA TTG TTA CAA CAC CCC AAC ATC TTC GAC GCG GGC GTG GCA GGT CTT     3820
Ile Leu Leu Gln His Pro Asn Ile Phe Asp Ala Gly Val Ala Gly Leu
650                 655                 660                 665
CCC GAC GAT GAC GCC GGT GAA CTT CCC GCC GCC GTT GTT GTT TTG GAG     3868
Pro Asp Asp Asp Ala Gly Glu Leu Pro Ala Ala Val Val Val Leu Glu
                670                 675                 680
CAC GGA AAG ACG ATG ACG GAA AAA GAG ATC GTG GAT TAC GTG GCC AGT     3916
His Gly Lys Thr Met Thr Glu Lys Glu Ile Val Asp Tyr Val Ala Ser
            685                 690                 695
CAA GTA ACA ACC GCG AAA AAG TTG CGC GGA GGA GTT GTG TTT GTG GAC     3964
Gln Val Thr Thr Ala Lys Lys Leu Arg Gly Gly Val Val Phe Val Asp
        700                 705                 710
GAA GTA CCG AAA GGT CTT ACC GGA AAA CTC GAC GCA AGA AAA ATC AGA     4012
Glu Val Pro Lys Gly Leu Thr Gly Lys Leu Asp Ala Arg Lys Ile Arg
    715                 720                 725
GAG ATC CTC ATA AAG GCC AAG AAG GGC GGA AAG TCC AAA TTG TAA AAT     4060
Glu Ile Leu Ile Lys Ala Lys Lys Gly Gly Lys Ser Lys Leu
730                 735                 740
GTAACTGTAT TCAGCGATGA CGAAATTCTT AGCTATTGTA ATCCTCCGAG GCCTCGAGGT     4120
CGACGAATTC CGACTCCGGA ACCAATTACT GATAATGTAG AAGATCATAC AGACACCGTC     4180
ACATACACTA GCTAGTGATA GCATTAATAC AGTAAGTGCA TCATCTGGAG AATCCACAAC     4240
AGACGAGACT CCGGAACCAA TTACTGATAA AGAAGAAGAT CATACAGTCA CAGACACTGT     4300
CTCATACACT ACAGTAAGTA CATCATCTGG AATTGTCACT ACTAAATCAA CCACCGATGA     4360
TGCGGATCTT TATGATACGT ACAATGATAA TGATACAGTA CCACCAACTA CTGTAGGCGG     4420
TAGTACAACC TCTATTAGCA ATTATAAAAC CAAGGACTTT GTAGAAATAT TTGGTATTAC     4480
CGCATTAATT ATATTGTCGG CCGTGGCAAT ATTCTGTATT ACATATTATA TATATAATAA     4540
ACGTTCACGT AAATACAAAA CAGAGAACAA AGTCTAGATT TTTGACTTAC ATAAATGTCT     4600
GGGATAGTAA AATCTATCAT ATTGAGCGGA CCATCTGGTT CAGGAAAGAC AGCCATAGCC     4660
AAAAGACTAT GGGAATATAT TTGGATTTGT GGTGTCCCAT ACCACTAGAT TTCCTCGTCC     4720
TATGGAACGA GAAGGTGTCG ATTACCATTA CGTTAACAGA GAGGCCATCT GGAAGGGAAT     4780
AGCCGCCGGA AACTTTCTAG AACATACTGA GTTTTTAGGA AATATTTACG GAACTTCTAA     4840
AACTGCTGTG AATACAGCGG CTATTAATAA TCGTATTTGT GTGATGGATC TAAACATCGA     4900
TGGCGTTAGA AGTCTTAAAA ATACGTACCT AATGCCTTAC TCGGTGTATA TAAGACCTAC     4960
CTCTCTTAAA ATGGTTGAGA CCAAGCT                                         4987

## Description

The present invention relates to antisense compounds complementary to partial sequences of the genome of hepatitis C virus (referred to as "HCV" hereinafter), and particularly to antisense compounds having antiviral effects such as inhibitory actions on replication of HCV and/or expression of HCV gene products, and the like.

To date, A, B and D types of human hepatitis viruses were discovered and serological diagnoses for these viruses were established. However, it has been a problem that cryptogenic hepatitis still exists (Digestive Diseases and Sciences, 31 122S-132S, 1986; Seminars in Liver Diseases, 6, 56-66, 1986).

On the other hand, in the middle of 1970s, a specific diagnostic technology for detecting hepatitis A virus (HAV) and hepatitis B virus (HBV) was developed and put to practical use. As the result, it has gradually become apparent that most of hepatitis due to blood transfusion is caused by pathogenes other than such viruses as HAV, HBV, and the like which grow in liver cells, and such hepatitis was designated as non-A, non-B hepatitis. In the United States, hepatitis occurs with the frequency of 1 to 10% after blood transfusion, and 90% or more of the cases are non-A, non-B hepatitis (Jikken Igaku, 8, 3, 15-18, 1990). In Japan, hepatitis occurs with the frequency of 10 to 20% after blood transfusion (about 200,000 cases a year), and 95% of the cases were non-A, non-B hepatitis. In addition, 40 to 50% of about 300,000 cases of sporadic hepatitis, which occur every year, are also non-A, non-B hepatitis. Most of these cases, including non-A, non-B hepatitis prevalent only in one district, do not have clear routes of infection such as blood transfusion, but it is considered that they may have other infectious routes (Jikken Igaku, 8, 3, 13-14, 1990).

With respect to the non-A, non-B hepatitis virus which is a main cause of this hepatitis after blood transfusion, Chiron Corporation succeeded, in 1988, in obtaining its gene fragment by a method completely different from conventional methods for exploring viruses, and this virus was designated as hepatitis C virus (HCV). Subsequently, the sequence of whole genome of the structural and non-structural proteins of HCV were published by not only Chiron Corporation but also Shimotoya et al. in the National Cancer Center (Proc. Natl. Acad. Sci. USA, 87, 9524-9528, 1990) and Takamizawa et al. in Osaka University, Microorganism Research Institute (Journal of Virology, 65, 3, 1105-1113, 1991).

Chiron Corporation succeeded in the expression of fused protein in yeast, said fused protein having at the C-terminal the polypeptide (363 residues) occurring in the region from NS3 to NS4, which is part of the non structural protein of HCV, and having at the N-terminal human superoxide dismutase (European Patent Publication No. A1 318216) and developing an ELISA (enzyme-linked immunosorbent assay) using the expressed recombinant antigen with collaboration with Ortho Corporation.

The Ministry of Health and Welfare in Japan approved in the first place in the world the use of a kit comprising an antigen useful for the detection of Anti-HCV antibody in order to screen the blood for transfusion and to assist diagnosis of hepatitis C. On the next day of the approval date (December 26, 1989), the Japanese Red Cross Society nation-widely started the screening of Anti-HCV antibody for blood from blood donors.

Although there are about 1,700,000 patients per year in Japan who receive blood transfusion, it is estimated that 12.3% of which caught hepatitis at the time before the introduction of this test reagent, while only about 3% caught hepatitis after the introduction. Thus, the number of hepatitis C patients (173,000) due to blood transfusion reduced to about one-fourth (The Asahi in Japan, the morning edition on May 2, 1991).

However, C100-3 clone which is a recombinant antigen and developed by Chiron Coroporation lacks near 20% homology, in terms of the nucleotide sequence and amino acid sequence, when compared with an antigen cloned in Japan. Accordingly, there is some possibility that Anti-HCV antibody can not be detected by the use of the Chiron kit. Further, it is described in various reports that there are other regions, (for example, part or most of the region of NS1, NS2, NS3, or NS5 according to the Chiron Corporation's nomenclature), which have only 70% or less homology. Accordingly, it is likely that there are test specimens which cannot be detected by the above-mentioned kit. In addition, there are considerable mutants in terms of genome sequence among HCV (European Patent Publication No. A1 518313). It is believed that such mutation is attributed to the fact that the virus genome consists of a single-strand RNA.

Once hepatitis C develops, it brings about acute hepatitis, chronic hepatitis, hepatocirrhosis, and cancer in high probability and kills the patients. Thus far, a reagent which can inhibit the expression and replication of HCV has not been discovered, and it is desired to develop a reagent which can cure the diseases associated with HCV.

On the other hand, interests in RNA (antisense RNA) and DNA (antisense DNA) having the sequence complementary to mRNA have currently increased. When existing in cells, an antisense RNA or DNA couples with a complementary mRNA to inhibit the translation of the mRNA. As the result, the synthesis of the protein coded by the gene is inhibited. Accordingly, the application of this technology has been thought valuable for developing a drug which exerts its effect through direct action to genes. However, the application of the antisense technology to diseases caused by HCV has not yet been fully investigated.

As described above, it appears that the HCV genome is apt to mutate very easily. The mutation results in the generation of many HCV subtypes wherein various portions, including important sites determining the character of the virus-constituting protein as well as surface antigen are different from each other. Hepatitis C is believed to occur when a human is infected with one of these viruses, and the symptoms can somewhat differ depending on the type of viruses.

The present inventors have isolated from one patient seized with hepatitis C plural viruses which differ from each other in amino acid sequence and nucleotide sequence (European Patent Publication No. A1 518313). Accordingly, the inventors have found it very important to design antisense compounds against these HCV genomes by the use of the conserved regions of HCV genomes.

In order to develop an agent which alleviates the symptom of hepatitis C patients, an extensive study was conducted. In the study, a HCV genome was taken independently and its cDNA was used to obtain a new antiviral agent against HCV in the procedure detailed below. As the result, the antisense compounds were obtained, which can inhibit the growth and replication of HCV and the expression of HCV gene products.

Thus, the present invention provides an antisense compound having a sequence complementary to a base sequence which consists of 10-34 bases and is extracted from:
(i) 93 bases from thymine at position 107 to adenine at position 199,
(ii) 152 bases from adenine at position 250 to cytosine at position 401, or
(iii) 52 bases from cytosine at position 808 to adenine at position 859,
of the base sequence shown in SEQ ID NO: 1, which can inhibit the growth and replication of HCV and the expression of HCV gene products.

Selection of the antisense compounds having the sequence complementary to the partial sequence of HCV genome and the method for determining the inhibition of the expression of HCV gene products by the use of said antisense compounds are detailed below.

The HCV gene is believed to be composed of a single RNA strand. The protein encoded by the strand is first expressed as a single polypeptide. The virus structural protein, RNA polymerase, protease, helicase, and the like are believed to be produced via processing of the single polypeptide. Accordingly, it seems that when the production of the first single polypeptide is inhibited, the expression of viral protease and HCV replication with the aid of viral RNA polymerase do not occur. Thus, if the protein from HCV gene is not produced, HCV does not grow.

As the region which is used for designing the antisense compounds of the present invention, the inventors selected the region which can inhibit the translation of the first single polypeptide which is a precursor of viral proteins. The protein positioned at the N-terminal of this precursor polypeptide is the core protein of HCV, which is followed by E1 (envelope), E2 (NS1 or envelope 2), NS2, and the like.

The inhibitory activity of the antisense compounds of the invention may be determined by the following method.

An mRNA covering the range beginning from 5' end of HCV genome and ending at the middle of E2 is synthesized using T7 RNA polymerase (Strategene). The synthesized mRNA is translated in an in vitro translation system using rabbit reticulocyte lysate (Promega) and canine microsomal membrane (Promega) in the presence of the antisense compounds, and then the amount of the expressed core protein is determined by immunoprecipitation assay with the Anti-HCV core antibody. Furthermore, recombinant vaccinia virus containing HCV gene from 5' end to at least core protein region can be used. After infecting human cell lines with the recombinant vaccinia virus, the cells are cultivated in the presence of the antisense compounds, and then the amount of the expressed core protein is determined by immunoprecipitation assay with the anti-HCV core antibody.

In the meanwhile, the HCV genome has a special translation system, which can also be found in poliovirus, etc. (Pelletier, J. et al., Nature, 334, 320-325, 1988), and IRES (Internal Ribosome Entry Site) region which exists within about 340 bases positioned at 5' side of HCV genome followed by the core protein of HCV (Tsukiyama - Kohara et al., J. of Virol., 66, 1476-1483, 1992), is responsible for the translation activity of this system. It is believed that tertiary structure is important to IRES function, and core protein is only translated correctly when the tertiary structure of IRES is correct. In the above-noted in vitro translation system, ORF (Open Reading Frame) existing in the 5' untranslated region of said about 340 bases is not substantially expressed as compared with the core protein, and therefore, the HCV-derived protein is believed to be expressed by the IRES activity. Accordingly, it is preferable to try to find out antisense compounds capable of inhibiting the IRES activity or destraying the tertiary structure of IRES, which results in the inhibition of the expression of the core protein in in vitro translation system or cell assay system.

Usable antisense compounds include phosphorothioate types wherein the oxide atom, double bonded with the phosphorus atom in the phosphodiester moiety which links adjacent two deoxyribonucleosides phosphorothioate type wherein the oxide atom is substituted with a sulfur atom; phosphonate types wherein the sulfur atom is substituted with a methyl group; unsubstituted phosphonate types; α oligonucleoside types, and the like (Crooke, R M, Anticancer Drug Des., 6, 6, 606-646, 1991; Tidd, D. M., Anticancer Research, 10, 1169-1182, 1990). Compounds other than nucleoside derivatives may be used as long as they can form a hybrid with mRNA target. Further, all of the antisense compounds which were introduced by Chrisey, L A. et al. in Antisense Research and Development, 1, 65-113, 1991, are also usable.

It will be easily understood that preferable antisense compounds of the present invention are those which are resistant to DNase, and those which form a hybrid to degradate with RNase H activity in cells (Tidd, D M., Anticancer Research, 10, 1169-1182, 1990).

Further, in order to increase a hybrid-forming ability of the antisense compounds without significantly decreasing a decomposing activity of the antisense compounds per se, it is advisable to convert a few phosphodiester bonds present at 3' and 5' terminal to phosphorthioate type or methylphosphonate type, while phosphodiester bonds in the internal sequence are remained unmodified.

Although any antisense compounds which meet the above criteria are satisfactory, preferred antisense compounds are those having a sequence complementary to a base sequence which consists of 10-34 bases and which is extracted from:
(a) 54 bases from guanine at position 127 to guanine at position 180;
(b) 34 bases from adenine at position 284 to thymine at position 317; or
(c) 34 bases from cytosine at position 343 to cytosine at position 376.
(Note: Any base number used herein corresponds to that in SEQ ID NO: 1).

More preferred antisense compounds are those having a sequence complementary to one or more base sequences which are selected from the sequences listed in the following items (1)-(3).
(1) A base sequence which is included within 54 bases from guanine at position 127 to guanine at position 180, and which contains 16 bases from cytosine at position 131 to adenine at position 146, 7 bases from cytosine at position 147 to cytosine at position 153, 6 bases from cytosine at position 151 to cytosine at position 156, or 6 bases from cytosine at position 175 to guanine at position 180.
(2) A base sequence which is included within 34 bases from adenine at position 284 to thymine at position 317, and which contains 5 bases from guanine at position 285 to thymine at position 289, or 6 bases from thymine at position 309 to thymine at position 314.
(3) A base sequence which is included within 34 bases from cytosine at position 343 to cytosine at position 376, and which contains 5 bases from guanine at position 355 to adenine at position 359, or 5 bases from adenine at position 369 to guanine at position 373.
   Above all, the antisense compounds which have a sequence complementary to one or more base sequences selected from the base sequences listed in the following items (4)-(13) are particularly preferred.
(4) A base sequence consisting of 16-24 bases which is included within 24 bases from guanine at position 127 to cytosine at position 150, and which contains at least 16 bases from cytosine at position 131 to adenine at position 146 (for example SEQ ID Nos: 2-26).
(5) A base sequence consisting of 15-30 bases which is included within 49 bases from guanine at position 127 to cytosine at position 175, and which contains at least 7 bases from cytosine at position 147 to cytosine at position 153 (for example SEQ ID Nos: 114-369).
(6) A base sequence consisting of 15-30 bases which is included within 31 bases from cytosine at position 150 to guanine at position 180, and which contains at least 6 bases from cytosine at position 151 to cytosine at position 156 (for example SEQ ID Nos: 27-38).
(7) A base sequence consisting of 15-30 bases which is included within 31 bases from cytosine at position 150 to guanine at position 180, and which contains at least 6 bases from cytosine at position 175 to guanine at position 180 (for example SEQ ID Nos: 38-43).
(8) A base sequence consisting of 15-33 bases which is included within 34 bases from adenine at position 284 to thymine at position 317, and which contains at least 5 bases from guanine at position 285 to thymine at position 289 (for example SEQ ID Nos: 44-49).
(9) A base sequence consisting of 15-33 bases which is included within 34 bases from adenine at position 284 to thymine at position 317, and which contains at least 6 bases from thymine at position 309 to thymine at position 314 (for example SEQ ID Nos: 50-58).
(10) A base sequence consisting of 15-30 bases which is included within 34 bases from cytosine at position 343 to cytosine at position 376, and which contains at least 5 bases from guanine at position 355 to adenine at position 359 (for example SEQ ID Nos: 59-99).
(11) A base sequence consisting of 15-30 bases which is included within 34 bases from cytosine at position 343 to cytosine at position 376, and which contains at least 5 bases from adenine at position 369 to guanine at position 373 (for example SEQ ID Nos: 71, 72, 78-80, 85-87, 91-93, and 97-105).
(12) A base sequence consisting of 15-26 bases which is included within 26 bases from thymine at position 351 to cytosine at position 376, and which contains at least 5 bases from guanine at position 355 to adenine at position 359 (for example SEQ ID Nos: 81-99)
(13) A base sequence consisting of 15-26 bases which is included within 26 bases from thymine at position 351 to cytosine at position 376, and which contains at least 5 bases from adenine at position 369 to guanine at position 373 (for example SEQ ID Nos: 85-87, 91-93, 97-105).
   Examples of most preferred antisense compounds of the present invention include:
(14) if antisense compounds meet the criterion of the above item (6) or (7), then those which satisfy both criteria;
(15) if antisense compounds meet the criterion of the above item (8) or (9), then those which consists of 20 or less bases;
(16) if antisense compounds meet the criterion of the above item (10) or (11), then those complementary to a base sequence consisting of 15-26 bases which is included within 26 bases from thymine at position 351 to cytosine at position 376; and
(17) those which satisfy both criteria of the above items (10) and (11).
   Further examples of the most preferred antisense compounds are:
(18) the compounds complementary to a base sequence consisting of 15-20 bases which is selected from 20 bases from cytosine at position 139 to guanine at position 158 (for example SEQ ID Nos: 244-249, 260-263, 275-277, 291, 292, 307);
(19) the compounds complementary to the base sequence consisting of 30 bases from cytosine at position 151 to guanine at position 180 (SEQ ID No: 38);
(20) the compounds complementary to the base sequence consisting of 20 bases from cytosine at position 131 to cytosine at position 150 (SEQ ID No: 6);
(21) the compounds complementary to the base sequence consisting of 19 bases from cytosine at position 141 to guanine at position 159 (SEQ ID No: 106);
(22) the compounds complementary to the base sequence consisting of 20 bases from guanine at position 355 to cytosine at position 374 (SEQ ID No: 98); and
(23) the compounds complementary to the base sequence consisting of 20 bases from thymine at position 353 to adenine at position 372 (SEQ ID No.: 90).

Although the antisense compounds of the present invention are shown for convenience as "nucleic acid" in Sequence Listing, the compounds are not necessarily nucleoside derivatives as far as they are capable of hybridizing to the target sequences, as discussed above. Furthermore, part of the sequence (preferably 5 or less bases) may be replaced by any non-complementary bases to such an extent that their hybridization ability are not spoiled.

It may be possible to introduce the antisense compounds of the present invention into cultured cells, for example, by incorporating said antisense compounds as such into the culture medium. The antisense compounds consisting of about 15-28 bases in the form of phosphorothioate-type or methylphosphonate-type are readily introduced into cells by such a method. In order to effect an active introduction of the antisense compounds, the transfection methods which are commonly applied to animal cells, such as calcium phosphate method, electroporation, or liposome method, may also be used preferably.

When intravenously administered to human subjects, it appears that about half of the antisense compounds administered will be absorbed by liver, judging from the results of experiments in animals. Depending on the structure and property of an antisense compound, the uptake efficiency can be increased by, for example, protecting the antisense compound with liposomes or attaching a substance capable of recognizing cells to the antisense compound.

The process for preparing the antisense compounds of the present invention is described in more detail below.

### (1) Preparation of mRNA T7N1-19

For example, plasmid pUCT71-19 (European Patent Publication 518,313) is firstly prepared by the alkaline method and subsequent CsCl density gradient ultracentrifugation. Then, the plasmid is digested completely with EcoRI to obtain a linear DNA which has been cut at a site 3' to the clone T7N1-19. About 80-100 µg of HCV mRNA T7N1-19 may be obtained from about 1 µg of this linear DNA by in vitro transcription using T7 RNA polymerase. This reaction may be effected by means of RNA TRANSCRIPTION Kit (Stratagene), although the reagents separately prepared may also be used under the condition in which T7 RNA polymerase is active. The resultant mRNA may be identified by northern hybridization. The probe may be prepared by the labelling method using a DNA fragment of 3'-terminal region of the clone T7N1-19. The amount of mRNA may be calculated from the absorbance at 260 nm.

### (2) Synthesis of antisense compounds

Phosphodiester-type oligonucleotides and phosphorothioate-type oligonucleotides may be synthesized by means of, for example, a DNA Synthesizer Model 394 (Applied Biosystems). The reaction is carried out under the condition of dimethoxytrityl-ON. The desired antisense compound may be obtained after the purification with HPLC (all of the diastereomers of the desired product are combined) and the subsequent treatment with acetic acid.

### (3) Measurement of the inhibitory effects of the antisense compounds on the translation of HCV-derived proteins using the in vitro translation method

The in vitro translation is carried out using the mRNA obtained in the above step (1) to express the HCV-derived proteins encoded by the mRNA under the IRES activity. The in vitro translation uses, for example, Rabbit Reticulocyte Lysate and Canine Microsomal Membranes (Promega). The microsomal membrane is considered to be necessary for the cutting, by signal peptidase, the junctions between the core protein and the envelope (E1) as well as the envelope (E1) and E2 (NS1). [³⁵S]-methionine is incorporated into the translated polypeptide. The polypeptides containing the HCV core protein may be immunoprecipitated with anti-HCV core antibody, electrophoresed on SDS-PAGE, and analyzed on BIO-IMAGE ANALYZER BAS 2000 (Fuji Film).

In order to determine the inhibitory effect on the translation, the antisense compound is preferably mixed with in vitro translation reagents immediately before the mRNA and in vitro translation reagents are mixed. As the result of such studies, it is confirmed that the antisense compounds of the present invention consisting of 10-34 bases (preferably about 15-30 bases) which may be designed on the basis of the HCV gene sequence are closely associated with the inhibitory effects.

### (4) Translation inhibition of HCV gene by antisense compounds in cell evaluation system using recombinant vaccinia virus

It is known that a homologous recombination occurs between a particular sequence found in vaccinia virus gene, which is connected with both termini of a foreign gene, and the corresponding sequence to said particular sequence in the vaccinia virus gene. Taking advantage of this homologous recombination, a recombinant vaccinia virus can be prepared, into which HCV gene has been inserted. The resultant vaccinia virus can be used to infect an appropriate cell, and the HCV gene is allowed to express in the cell. Accordingly, translation inhibitory effect of the antisense compounds of the present invention can be measured by the use of a cell evaluation system which permits assay of expressed HCV protein.

Specifically, HCV-derived gene is inserted into hemaglutinine (HA) gene of vaccinia virus, as described hereinafter in the working example. HA is not essential for the growth of vaccinia virus. However, loss of HA gene function results in vaccinia virus which is deficient in hemagglutination ability, and can be detected by virus plaque stain by chick erythrocyte. Accordingly, said HA gene is conveniently used as an inserting site of a foreign gene. However, said inserting site is not limited to the HA gene as far as the growth of the virus is not adversely affected and the virus containing a foreign gene can easily be detected after the insertion. The HCV-derived gene to be inserted into the vaccinia virus must be a gene which contains IRES region locating at 5' untranslated region. As previously stated, it is said that a polypeptide coded by the HCV genome is expressed as a single polypeptide (precursor protein) comprising about 3,000 amino acid residues, and the polypeptide results in various functional proteins 24 through processing. The precursor proteins consists of core protein, E1 (envelope) protein, E2 (NS1 or envelope 2) protein, etc. aligned from N-terminus in this order. This means that the HCV genome is composed of untranslated region, core protein-encoding region, E1 protein-encoding region, etc. aligned from 5' terminus in this order. In order to determine the magnitude of the translation inhibitory effect of HCV polypeptide, it is essential that HCV-derived polypeptide is normally produced. Accordingly, the HCV-derived gene to be inserted must be a gene which contains at least the IRES region locating at 5' untranslated region and core protein-encoding region locating at 3' side thereof. More specifically, such HCV-derived gene may be a gene comprising the base sequence beginning form the base at position 25-30 in SEQ ID No. 1 and containing subsequent ^{∼}910 bp. Since this gene encodes the core protein, the expression of the gene can be measured by detecting a protein of about 22KDa through western blotting.

The HCV-derived gene is inserted into a vector such as pUC19, after linked with a promoter at the 5' terminal. The promoter may be anything as far as it functions in vaccinia virus. High-expression promoter is preferable, such as an early promoter derived from vaccinia virus. More specifically, it is preferred to use 7.5K promoter from vaccinia virus (cell 125 805-813, 1981) and its variant which contains point mutation (J. Mol. Biol., 210) 749-769, 1988). It is one of preferred embodiments of the present invention to use a combination of a synthetic DNA represented by SEQ ID No. 406 and the above-noted promoter. When a reporter gene of luciferase gene is inserted at 3' side of HCV gene, the fused gene yields a fused protein. Said fused protein consists of HCV-derived polypeptide and a polypeptide encoded by the reporter gene, and therefore, the HCV-derived polypeptide is indirectly measured by measuring the polypeptide encoded by the reporter gene after processing under appropriate conditions. The HCV gene contains a signal sequence at which the core protein and E1 protein undergo processing under an appropriate condition. Accordingly, where translation inhibitory activity of HCV core protein is measured, it is desirous to make design so that the core protein is cut at its C-terminal, taking advantage of the signal sequence. Construction of a vector can be conducted in conventional manners.

A vector DNA is prepared in conventional manner using the transfer vector thus obtained. The DNA and vaccinia virus are combined so that homologous recombination may occur between them. A cell line derived from human beings is infected with the recombinant vaccinia virus thus obtained. The recombinant protein expressed in the infected cells is recovered according to any one of conventional methods, and the amount of the HCV-derived polypeptide is measured by a known method such as western blotting.

The antisense compound of the present invention is added before and/or after the infection of cells with the recombinant vaccinia virus. The translation inhibitory effect of the antisense compounds of the invention is determined after comparison of the amount of expressed polypeptide with that obtained when the antisense compound is not added, or when there is added other antisense compound which has low homology with a complementary stand of a HCV or reporter gene and therefore hardly forms a hybrid with the HCV gene. Many groups including American bio-venture companies have described about the dose of antisense compounds. According to such information, it has been shown in incurable diseases such as HIV patients that an antisense compound which exhibits its effect on cultured cells (animal cells) at 10-100 µM also exhibits its effect on human subjects to some extent. Based on those values, we aimed for the antisense compounds which exhibit their effects in the in vitro translation study at 10 µM or less, and preferably at 1 µM or less, so that they may exhibit their effects on cultured cells at 50 µM or below after taking the contribution of the factors such as permeability and uptake efficiency into consideration.

As a result, the antisense compounds which realize the above aim have been found as described in the following examples. These compounds are expected to exhibit their effects on cultured cells expressing the HCV gene and even on HCV patients.

### Brief Description of the Drawings

Fig. 1 is an electrophoretic pattern which shows the translational inhibitory effects of antisense compounds of the present invention, Anti 1, SMS 13, SMS 14, SMS 16, SMS 17, and SMS 18 (the final concentration = 1.18 µM) as measured in in vitro translation system.

Fig. 2 is an electrophoretic pattern which shows the correlation between the concentration of the antisense compounds of the present invention, SMS 16, SMS 17, and SMS 18, and their translational inhibitory effects.

Fig 3. shows Western Blotting of HCV core protein expressed by recombinant vaccinia virus. Lane 1 represents recombinant vaccinia virus rVV5CL and Lanes 2 and 3 represent wild-type vaccinia virus.

Fig. 4 shows an enzymatic activity of luciferase expressed by WRL 68 cell infected by the recombinant vaccinia virus rVV5CL in the presence of antisense compounds of the present invention at concentrations of 0.25, 0.5, and 2.5 µM. The ordinate indicates an enzymatic activity of luciferase (x10⁻²⁰ mol/8µM). The legend "antisense(-)" means an enzymatic activity of luciferase in the absence of the antisense compounds of the invention.

Fig. 5 shows relative values of the enzymatic activity of the expressed luciferase when an average enzymatic activity of antisense(-) is stipulated as 100.

Fig. 6 shows an enzymatic activity of luciferase expressed by WRL68 cell infected by the recombinant vaccinia virus rVV5CL in the presence of antisense compounds of the present invention at concentrations of 0.25, 0.5, and 1.5 µM. The activity is a relative value to the antisense(-).

The following examples further illustrate the present invention. The examples are illustrative only and are not intended to limit the scope of the invention in any way.

### Example 1: Preparation of mRNA T7N1-19

A hundred µg of plasmid pUCT7119 (European Patent Publication 518,313) which contains the clone T7N1-19 shown as SEQ ID NO: 1 in the cloning sites of pUC19 was prepared by the alkaline method and the subsequent density gradient ultracentrifugation using CsCl (Molecular Cloning: A Laboratory Manual, 2nd ed., 1.33-1.52, 1989).

Ten µg of this highly purified plasmid was digested completely with EcoRI to obtain a linear DNA which had been cut at a site 3' to the clone T7N1-19. One µg of the linear DNA was subjected to a reaction in 50 µl of a reaction mixture consisting of 40 mM Tris-HCl (pH 8.0), 5 mM DTT, 50 µg/ml BSA, 2 mM each NTP, 40 mM MgCl₂, 1 mM spermidine, 50 units of RNase inhibitor, and 1.42 µg of T7 RNA polymerase. After 20 min at 37 °C, 10 unit of T7 RNA polymerase was added and the mixture was further incubated for 20 min at 37 °C. Finally, 10 units (1 µl) of DNase I (Stratagene) was added, and the mixture was incubated for 10 min at 30 °C. The reaction was then terminated by adding 50 µl of phenol/chloroform (1/1) mixture. After mixing, 50 µl of the aqueous phase was recovered. In order to precipitate RNA, the aqueous phase was mixed with 5.5 µl of 3M sodium acetate (pH5.5) and then with 150 µl of ethanol. The mixture was then centrifuged at 15,000 rpm for 15 min, and the resultant RNA (transcript) was dried.

The RNA thus obtained was dissolved in 30 µl of DEPC-treated sterile water (Molecular Cloning: A Laboratory Manual, 2nd ed., 7.26, 1989). Three µl aliquot of the resultant solution was used to measure the absorbance at 260 nm, and the amount of RNA was calculated from the absorbance on the assumption that 1 OD = 40 µg/ml. The amount of RNA thus calculated was about 80 µg. In order to examine the length of the transcript, an agarose electrophoresis using formamide was carried out (Molecular Cloning: A Laboratory Manual, 2nd ed., 7.43, 1989). On the gel, the RNA was shown as a single band, and its length was proper as compared with the molecular markers (GIBCO BRL: 0.24-9.5 Kb RNA Ladder). Furthermore, the band on the agarose gel was transferred onto a membrane, and northern hybridization (Molecular Cloning: A Laboratory Manual, 2nd ed., 7.39-7.52, 1989) was carried out to confirm that the transcripted RNA was surely derived from the clone T7N1-19. The probe used in this hybridization was prepared according to the labelling method (Molecular Cloning: A Laboratory Manual, 2nd ed., 10.13-10.17, 1989) from a DNA fragment which has a sequence of the clone N19 at the 3'-region of the clone T7N1-19 .

### Example 2: In vitro synthesis of HCV-derived proteins and analysis thereof

The mRNA T7N1-19 synthesized in Example 1 has almost the same structure as 2,007 bases of the 5'-region of the HCV genome gene (European Patent Publication 518,313) which is a single stand RNA. The difference between the above two RNAs resides in that the promoter enhancing sequence of T7 which acts on T7 RNA polymerase has been attached to the 5'-terminal of the HCV gene in the former RNA. The in vitro translation was initiated by adding a mixture consisting of 11.375 µl of Rabbit Reticulocyte Lysate (Promega), 1.17 µl of Canine Microsomal Membranes (Promega), 5.2 µl of Amino Acid Mixture (Promega), 1.3 µl (729 KBq) of L-[³⁵S]-methionine (Amersham), and 0.2 µl of RNase Inhibitor (Takara Shuzo) to about 3.5 µg of the transcript obtained above so as to obtain the final volume of 14.37 µl. The reaction was accomplished substantially according to the protocol described in "Translation in vitro Technical Manual" (Promega). Similar reaction was carried out without the RNA (transcript) in order to check the reagents used, whereby nothing was synthesized. In the control, 0.5 µg of E. coli β-lactamase mRNA (supplied by Promega along with Canine Microsomal Membranes) was substituted for about 3.5 µg of the transcript.

After incubating for 1 hour and 15 min at 30 °C, only polypeptides including the HCV core protein were separated by the immunoprecipitate method, and then subjected to SDS-PAGE. This is because it was expected that the amount of proteins per lane may become too plenty to analyze synthesized proteins, if the whole reaction mixture is used for the electrophoresis.

Thus, 2.5% SDS was added to the whole translation reaction mixture so that the final concentration of SDS became 0.5%. Four volume of RIPA buffer 1 (1% Triton X-100, 1% sodium deoxycholate, 0.15 M NaCl and 50 mM Tris-HCl (pH7.5)) was then added, and the mixture was cooled on ice. One µl of anti-HCV core antibody (purified from rabbit serum, polyclonal antibody, 1 µg/1 µg) was then added and the resultant mixture was allowed to stand for 1 hour at 0 °C. The mixture was then mixed with 3.125 µl of zysorbin (Zymet, 10% w/v), and allowed to stand for 1 hour at 0 °C. Then, the mixture was centrifuged at 3000 rpm for 3 min. The precipitate was washed by adding 100 µl of RIPA buffer 2 (1% Triton X-100, 1% sodium deoxycholate, 0.1% SDS, 0.15 M NaCl and 50 mM Tris-HCl (pH7.5)), and the same procedure was repeated with 100 µl of RIPA buffer 3 (1% Triton X-100, 1% sodium deoxycholate, 0.1% SDS, 0.15 M NaCl, 50 mM Tris-HCl (pH7.5) and 1 mg/ml BSA). After the final washing with RIPA buffer 2, the resultant precipitate was suspended in 8 µl of SDS loading buffer (9.1% Tris-HCl (pH6.8), 16.1% (v/v) glycerine, 4.2 M urea, 3.15% SDS, 12.7% (v/v) β-mercaptoethanol and 0.04% BPB).

This sample was then boiled at 95 °C for 5 min. Eight µl of the sample thus obtained was applied to 0.1% SDS-15.0% polyacrylamide gel (70 x 85 x 1 mm). In this electrophoresis, Rainbow [¹⁴C] methylated protein molecular weight markers (Amersham, molecular weight range: 14,300-200,000) was used as marker proteins. The electrode buffer utilized was a Tris buffer (25 mM Tris (pH8.3), 192 mM glycine and 0.1% SDS). The electrophoresis was carried out with a constant electric current of 30 mA for 45 min. The gel was then placed on a Whatman 3MM filter, covered with a transparent wrapping film (Saran wrap), and dried with a gel drier. The dried gel was held between imaging plates (Fuji Film, Type BAS-III) and put into a designated cassette, and allowed to stand at room temperature for 12 hours (these procedures were done according to the protocol for BIO-IMAGE ANALYZER BAS 2000 of Fuji Film). By analyzing the imaging plate on BIO-IMAGE ANALYZER, about 22 KDa HCV-derived core protein and its about 61 KDa precursor (polypeptide consisting of 555 amino acids) labelled with ³⁵S-methionine were detected as sharp bands.

### Example 3: Synthesis of antisense compounds

From the region beginning from thymine at position 27 and ending at cytosine at position 410, a lot of specific sequences consisting of about 10-34 bases to which antisense compounds are to be hybridized were set up, and the complementary sequences determined by such specified base sequences were used as the sequences of antisense oligonucleotides. The antisense oligonucleotides were synthesized using Applied Biosystems DNA Synthesizer Model 394. The reaction was carried out under the condition of dimetoxytrityl-ON, and the protective groups on the bases which were added during the synthesis were removed according to the protocol provided by the manufacturer. The synthesized oligonucleotides were purified by HPLC. Although, in the case of phosphorothioate-type oligonucleotides, they are not separated in a single peak as in the case of phosphodiester-type oligonucleotides, all of the diastereomers were combined into one lot. The protective group on the hydroxy group at the 5'-terminal (dimethoxytrityl group) was deprotected with acetic acid aqueous solution according to the conventional method to obtain a desired antisense compound. Such antisense compounds were further treated with phenol and quantified from the absorbance at 260 nm on the assumption that 1 OD = 35 µg/ml. The sequences of the antisense compounds thus synthesized are shown below.

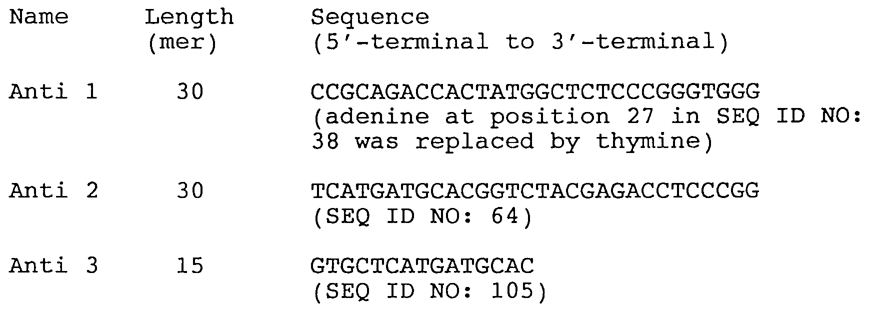

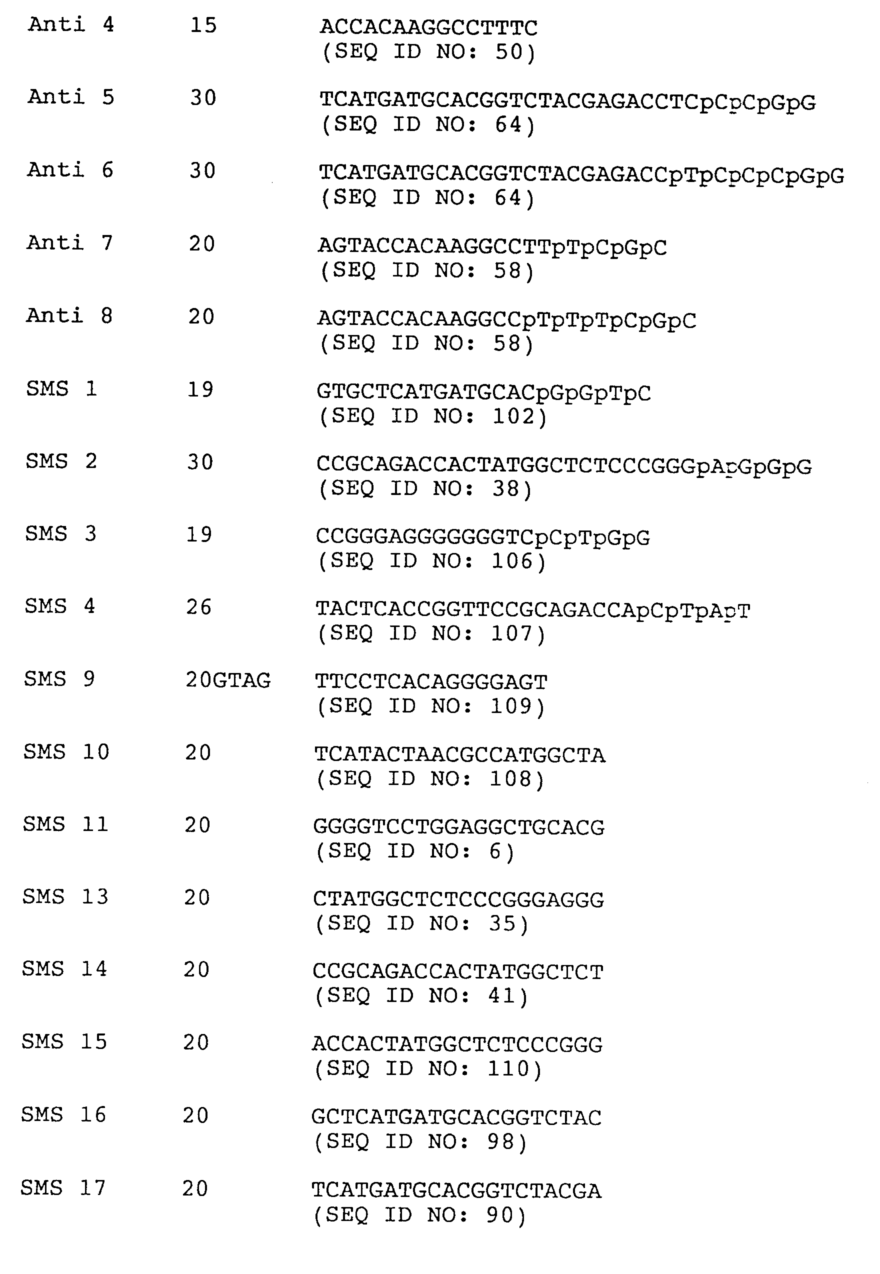

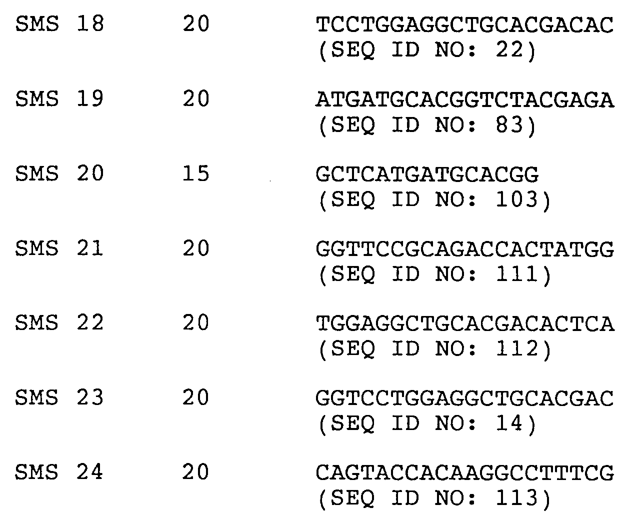

In the above sequences, the letter "p" inserted between two bases indicates that the phosphodiester linkage at that position is not phosphorothioate-type but is an unmodified phosphodiester linkage. The phosphate linkages between the other bases are all phosphorothioate-type.

### Example 4: Inhibitory effects of antisense compounds on the synthesis of HCV-derived proteins

The experiments were carried out as described below using antisense compounds synthesized in Example 3.

The in vitro translation was accomplished as described in Example 2 by adding a lysate mixture consisting of 11.375 µl of Rabbit Reticulocyte Lysate (Promega), 1.17 µl of Canine Microsomal Membranes (Promega), 5.2 µl of Amino Acid Mixture (Promega), 1.3 µl (729 KBq) of L-[³⁵S]-methionine (Amersham), and 0.2 µl of RNase Inhibitor (Takara Shuzo) into an Eppendorf tube containing mRNA T7N1-19 so as to obtain the final volume of 14.37 µl. The Eppendorf tube contained also an antisense compound on its wall so that the lysate mixture was mixed with the antisense compound prior to the mixing with mRNA. All of the procedures after the reaction were carried out as described in Example 2.

In these experiments, at least three in vitro translation reactions per experiment were carried out in the absence of an antisense compound and those three reactions were arranged on an electrophoresis gel disconnectedly. Furthermore, each of the reagents used in one experiment such as Rabbit Reticulocyte Lysate, Amino Acid Mixture, Canine Microsomal Membranes, and L-[³⁵S]-methionine was taken from the same lot, and combined together to make a mixture which was then divided into aliquots.

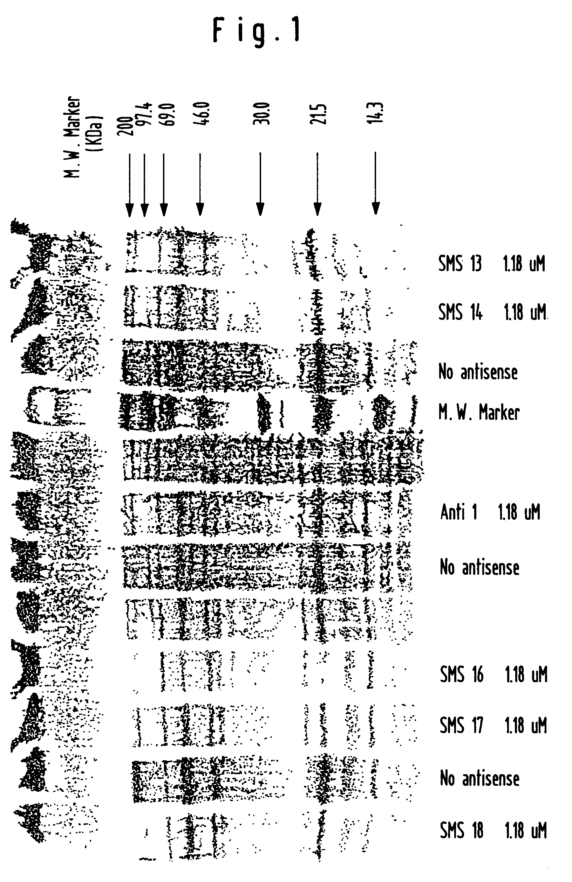

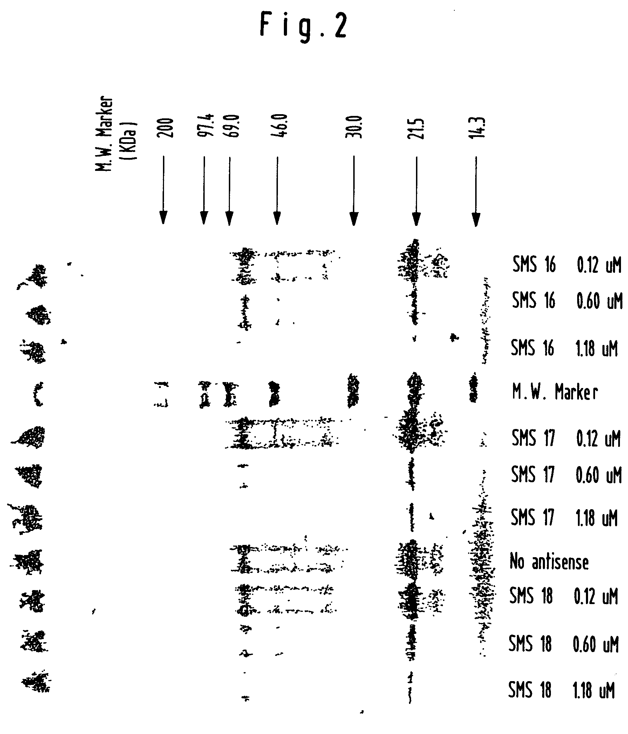

The inhibitory effects of antisense compounds on the translation of the HCV core protein were analyzed on BIO-IMAGE ANALYZER BAS 2000 (Fuji Film), and the results were printed out by Pictrography (Figures 1 and 2).

Among the numerous antisense compounds designed in the present invention, those particularly effective are antisense compounds which are directed to the regions positioned at 131-146, 151-156, 175-180, 285-289, 309-314, 355-359, or 369-373 in SEQ ID NO: 1.

These antisense compounds were examined in the in vitro translation system at a final concentration of, for example, about 0.12 µM, about 0.6 µM, about 1.2 µM, about 2.9 µM, or about 5.8 µM. In the experiment carried out with a concentration of about 1.2 µM, the amount of the produced HCV core protein has decreased, in comparison with the case without the antisense compound, to about 1/5 to about 1/10 or less for Anti 1, SMS 1, SMS 11, SMS 13 and SMS 14, and to about 1/10 to about 1/40 or less for SMS 16, SMS 17, and SMS 18 (Figures 1 and 2). Antisense compounds, Anti 1, Anti 4, Anti 7, SMS 1, SMS 2, SMS 11, SMS 13, SMS 14, SMS 16, SMS 17, and SMS 18 at a final concentration of from about 2.9 µM to about 5.8 µM did not affect the translation of E. coli β-lactamase mRNA. In the reaction carried out in the presence of SMS 9 (an antisense compound directed to the sequence consisting of 20 bases from adenine at position 66 to cytosine at position 85: SEQ ID NO: 109) which was evaluated for the purpose of comparison, the amount of the produced HCV core protein has decreased only slightly. Although the amount of the produced HCV core protein was decreased by SMS 3, this antisense compound has affected also the translation of E. coli β-lactamase mRNA to some extent.

Thus, it was confirmed that the antisense compounds of the present invention act specifically on the mRNA of HCV to inhibit the translation of HCV gene without adversely affecting the translation system as such.

### Example 5: Construction of a recombinant vaccinia virus rVV5CL

### (1) Preparation of a transfer vector for constructing a recombinant vaccinia virus

The HA protein gene was purified from vaccinia virus strain WR according to the procedure described in Example 1 of Japanese Patent Publication (kokai) 63-63380. Vaccinia virus strain WR was purified and suspended in 50mM Tris-HCl (pH 7.4) containing 1mM EDTA and 0.5% sodium dodecylsulfate. To this suspension was added proteinase K at 250-1000 µg/ml. The resultant mixture was incubated overnight at 37 °C, and then extracted thrice with buffer-saturated phenol-chloroform (1:1). Then, viral DNA was precipitated with ethanol. (Hereinafter, the term "ethanol precipitation" refers to a procedure in which an aqueous phase is mixed with one tenth volume of 3M sodium acetate or equal volume of 4M ammonium acetate and 2.5 fold volume of ethanol, then subjected to centrifugation using a rotor having about 5 cm of radius at 15,000 rpm for 15min at 4 °C, and the resultant precipitate is dried.) The DNA thus obtained was dissolved in 10 mM Tris-HCl (pH 8.0) containing 1mM EDTA, digested with HindIII, and subjected to agarose gel electrophoresis to isolate an about 50 kb HindIII A fragment. This HindIII A fragment was digested with SalI in high-salt buffer (50mM Tris-HCl, 100mM NaCl, 10mM MgCl₂, 1mM DTT (pH 7.5)), and then subjected to agarose electrophoresis to isolate an about 1.8kb HindIII-SalI fragment which is present at 3' terminal of the HindIII A fragment. This DNA fragment was blunt-ended with T4 DNA polymerase. By means of DNA Ligation Kit (Takara Shuzo), this DNA fragment was incorporated into pUC 19 cloning vector which had been digested with HindIII and EcoRI, and then blunt-ended with T4 DNA polymerase.

A 7.5k protein promotor fragment was also purified from vaccinia virus strain WR according to the procedure described in Example 4 of Japanese Patent Publication (kokai) 63-63380. Viral DNA prepared as described above was digested with SalI in high-salt buffer, and subjected to agarose electrophoresis to obtain an about 0.9kb SalI fragment. Separately, plasmid pUC 18 was digested with SalI in high-salt buffer, and subjected to extraction with phenol and ethanol precipitation to obtain a linear plasmid. This linear plasmid was then ligated to the about 0.9kb SalI fragment described above in ligation buffer (66mM Tris-HCl, 1mM ATP, 5mM MgCl₂, 5mM DTT (pH 7.6)) by means of T4 DNA ligase. The ligation mixture was used to transform E. coli strain JM103. Plasmid p0901 was obtained by screening in which each of the plasmids from transformed clones was digested with SalI to obtain the above DNA fragment which was then digested with RsaI, AluI, HapII and DdeI for analysis. This plasmid was digested with RsaI and HincII in medium-salt buffer (10mM Tris-HCl, 50mM NaCl, 10mM MgCl₂, 1mM DTT (pH 7.5)), and then subjected to agarose electrophoresis to isolate a 0.26kb blunt-ended RsaI-HincII fragment. This fragment includes 7.5k protein promotor. This DNA fragment was incorporated by means of DNA Ligation Kit (Takara Shuzo) into pUC 19 cloning vector which had been digested with HincII.

In the ligation reaction described above, 5-10 ng of vector DNA which had been prepared as described below was used. The pUC 19 cloning vector was cut with restriction enzymes HindIII and EcoRI or HincII (Toyobo), treated with phenol/chloroform, and subjected to ethanol precipitation. The resultant linear DNA was dephosphorylated at its 5' end using alkaline phosphatase (Boehringer-Mannheim) (Molecular Cloning: A Laboratory Manual, 1982, Cold Spring Harbor Laboratory Press), treated with phenol/chloroform, and then subjected to ethanol precipitation.

DNA thus constructed was used to transform E. coli JM109 using competent cells (COMPETENT HIGH) supplied by Toyobo according to the manufacturer's instruction.

From transformants thus obtained, a plasmid in which the 5' side of the HA protein gene is present at the same side as the EcoRI site in the multicloning site of pUC was selected by conventional miniscreening (Molecular Cloning: A Laboratory Manual, 1982, Cold Spring Harbor Laboratory Press), and designated as pUCHA. In addition, a plasmid in which the 5' side of the 7.5k promotor is present at the same side as the HindIII site in the multicloning site of pUC 19 was also selected and designated as pUC7.5.

Plasmid DNAs of pUCHA and pUC7.5 were prepared from corresponding transformants and sequenced by means of a fluorescence sequencer GENESIS 2000 system (DuPont). Synthetic sequence primers used in this sequencing were 5'd(GTAAAACGACGGCCAGT)3' (SEQ ID NO: 399) and 5'd(CAGGAAACAGCTATGAC)3' (SEQ ID NO: 400).

One µg of plasmid pUC7.5 was cut with a restriction enzyme SmaI, treated with phenol/chloroform, subjected to ethanol precipitation, dephosphorylated at its 5' end using alkaline phosphatase (Boehringer-Mannheim) (Molecular Cloning: A Laboratory Manual, 1982, Cold Spring Harbor Laboratory Press), treated with phenol/chloroform, and subjected to ethanol precipitation. Into 10 ng of DNA thus obtained, 5 ng of synthetic linker 5'd(pCAGATCTGCAAGCTTG)3' (SEQ ID NO: 401) was inserted by means of DNA Ligation Kit (Takara Shuzo). The DNA thus constructed was used to transform E. coli DH5 using competent cells (COMPETENT HIGH) supplied by Toyobo according to the manufacturer's instruction. From transformants thus obtained, plasmid pUC7.5GH in which the above synthetic linker has been incorporated so that BglII and HindIII sites align in this order in the same direction as the 7.5k promotor was obtained by conventional miniscreening.

In order to modify the 7.5k promotor, this plasmid was used to amplify a DNA fragment having a specific sequence by PCR method according to the method of Saiki et al. [Nature, 324, 126, (1986)].

To a mixture of 10 ng of plasmid pUC7.5GH, 10 µl of 10xPCR buffer (100mM Tris-HCl, pH 8.3, 500mM KCl, 15mM MgCl₂, 1% gelatin), 16 µl of 1.25mM 4dNTP, and each 5 µl (20 µM) of synthetic DNA primers 5'd(CAGGAAACAGCTATGAC)3' (SEQ ID NO: 402) and 5'd(GAATAGTTTTTCAATTTTTACG)3' (SEQ ID NO: 403), or each 5 µl (20 µM) of synthetic primers 5'd(CGTAAAAATTGAAAAACTATTC)3' (SEQ ID NO: 404) and 5'd(GTAAAACGACGGCCAGT)3' (SEQ ID NO: 405) was added water so as to obtain the final volume of 100 µl. The mixture was firstly heated at 95 °C for 5 min and then cooled rapidly to 0 °C. After 1min, 0.5 µl of Taq DNA polymerase (7 unit/µl, AmpliTaq™, Takara Shuzo) was added and the mixture was covered with mineral oil. This sample was subjected to 30 cycles of 1 min at 95 °C, 1 min at 48 °C, and 1 min at 72 °C on DNA Thermal Cycler (Perkin-Elmer Cetus Instruments). At the end of this period, the reaction mixture was maintained at 72 °C for 7 min, and then treated with phenol/chloroform. After ethanol precipitation, two amplified DNA fragments which are 250 bp and 110 bp in length were obtained. These fragments were purified on 5% acrylamide gel. To a mixture of each 5 ng of DNA fragments obtained above, 10 µl of 10xPCR buffer (100mM Tris-HCl, pH 8.3, 500mM KCl, 15mM MgCl₂, 1% gelatin), 16 µl of 1.25mM 4dNTP, and each 5 µl (20 µM) of synthetic DNAs 5'd(CAGGAAACAGCTATGAC)3' (SEQ ID NO: 402) and 5'd(GTAAAACGACGGCCAGT)3' (SEQ ID NO: 405) was added water so as to obtain the final volume of 100 µl. The mixture was firstly heated at 95 °C for 5 min and then cooled rapidly to 0 °C. After 1 min, 0.5 µl of Taq DNA polymerase (7 unit/µl, AmpliTaq™, Takara Shuzo) was added, and the mixture was covered with mineral oil. This sample was subjected to 30 cycles of 1 min at 95 °C, 1 min at 48 °C, and 1 min at 72 °C on DNA Thermal Cycler (Perkin-Elmer Cetus Instruments). At the end of this period, the reaction mixture was maintained at 72 °C for 7 min, and then treated with phenol/chloroform. After ethanol precipitation, an amplified DNA fragment which is 330bp in length was obtained. This amplified fragment was digested with restriction enzymes EcoRI and PstI, and purified on 5% acrylamide gel. Five ng of the DNA fragment thus obtained was incorporated by means of DNA Ligation Kit (Takara Shuzo) into pUC 19 which had been digested with EcoRI and PstI. The resultant vector was used to transform E. coli DH5 using competent cells (COMPETENT HIGH) supplied by Toyobo according to the manufacturer's instruction. From transformants thus obtained, plasmid pUCSP which has a potent promotor of vaccinia virus was isolated by conventional miniscreening (Molecular Cloning: A Laboratory Manual, 1982, Cold Spring Harbor Laboratory Press). The DNA fragment inserted into the multicloning site of the above plasmid was sequenced using a fluorescence sequencer GENESIS 2000 system (DuPont).

A synthetic DNA (SEQ ID NO: 406) which was designed to have BamHI and BglII sites at the ends of the promotor described in the 40th General Meeting of Japan Virology Society Abstract 4075, 
5'd(GATCCAAAAATTGAAAAACTAGTCTAATTTATTGCACGGA)3'
3'(GTTTTTAACTTTTTGATCAGATTAAATAACGTGCCTCTAG)5'
was inserted into BamHI and BglII sites of plasmid pUCSP by conventional method using DNA Ligation Kit (Takara Shuzo) according to the manufacturer's instruction. The resultant plasmids were used to transform E. coli DH5. A plasmid pUCSE in which six synthetic DNAs had been inserted tandem in correct direction was then isolated by miniscreening.

The plasmid pUCSE thus obtained was digested with restriction enzymes PstI and EcoRI. The reaction mixture was treated with phenol/chloroform and subjected to ethanol precipitation. The precipitated DNA was blunt-ended with T4 DNA polymerase, and then purified on 5% acrylamide gel to obtain a 550bp DNA fragment. Five ng of the DNA fragment thus obtained was ligated to 10 ng of plasmid pUCHA which had been digested with NruI. The resultant plasmids were used to transform E. coli DH5 using competent cells (COMPETENT HIGH) supplied by Toyobo according to the manufacturer's instruction. From the transformants thus obtained, plasmid pHASE in which the vaccinia viral promotor had been inserted in the same direction as the HA gene was isolated by conventional miniscreening (Molecular Cloning: A Laboratory Manual, 1982, Cold Spring Harbor Laboratory Press). The DNA fragment which had been inserted in the multicloning site of the above plasmid was sequenced by means of a fluorescence sequencer GENESIS 2000 system (DuPont). DNA sequence thus determined which begins from the SalI site and ends at the HindIII site of the multicloning site of that plasmid is shown as SEQ ID NO: 409 in Sequence Listing.

The segment of the HCV gene beginning from its 5' end and ending at the core protein gene was amplified by PCR method. To a mixture of five ng of DNA of clone T7N119 described in Example 28 [2] of European Patent Publication 518,313, 10 µl of 10x PCR buffer (100mM Tris-HCl, pH 8.3, 500mM KCl, 15mM MgCl₂, 1% gelatin), 16 µl of 1.25mM 4dNTP, and each 5 µl (20 µM) of synthetic DNAs 5'd(CGAAGCTTGCCAGCCCCCTGATGGG)3' (SEQ ID NO:407) and 5'd(CCGGATCCCGGAAGCTGGGATGGTCAAC)3' (SEQ ID NO:408) was added water so as to obtain the final volume of 100 µl, and the mixture was firstly heated to 95 °C for 5 min, and then cooled rapidly to 0 °C. After 1 min, 0.5 µl of Taq DNA polymerase (7 unit/µl, AmpliTaq™, Takara Shuzo) was added, and the mixture was covered with mineral oil. This sample was subjected to 30 cycles of 1 min at 95 °C, 1 min at 58 °C, and 1 min at 72 °C on DNA Thermal Cycler (Perkin-Elmer Cetus Instruments). At the end of this period, the reaction mixture was maintained at 72 °C for 7 min, and then treated with phenol/chloroform. After ethanol precipitation, an amplified DNA fragment which is 910bp in length was obtained. This amplified DNA fragment was digested with restriction enzymes HindIII and BamHI, and then purified on 5% acrylamide gel. The purified DNA fragment was inserted into HindIII and BamHI sites of PicaGene™ cassette vector (Toyo Ink) for luciferase assay. Plasmid pCS5CL in which the segment of the HCV gene begining from its 5' end and ending at the core protein gene had been inserted upstream of the luciferase gene in the same direction was obtained by miniscreening.

Plasmid pCS5CL was partially digested with EcoRI, and then completely digested with HindIII. The reaction mixture was subjected to agarose electrophoresis to isolate a 2.6 kbp fragment. This fragment was inserted into HindIII and EcoRI sites of plasmid pHASE. Then, plasmid pHA5CL which contains, in the vaccinia viral HA protein gene, the vaccinia viral promotor, the segment of the HCV gene begining from its 5' end and ending at the core protein gene, and the luciferase gene in this order was obtained by miniscreening.

### (2) Construction of recombinant vaccinia virus rVV5CL

African green monkey kidney-derived cell line CV-1 (Rikagaku Kenkyusho Saibou Kaihatu Ginko RCB0160) which had been cultivated to semi-confluent in a 3.5 cm petri dish was infected with vaccinia virus strain LC16mO (Rinsho-to-uirus, 3(3), 229-235, 1975) at MOI (multiplicity of infection) = 0.1 PFU/cell for 1 hour at room temperature. Separately, plasmid pHA5CL constructed in (1) was isolated and purified from the recombinant E. coli according to the method of Maniatis et al. [Molecular Cloning: A Laboratory Manual, Cold Spring Harbor Laboratory, 86-96(1982)] to obtain a large amount of the transfer vector pHA5CL DNA. Ten µg of pHA5CL DNA thus obtained was mixed with 30 µl of Lipofectin (Life Technology) in 170 µl of Opti-MEM medium (Life Technology), allowed to stand for 10 min, and used as a transfection solution.

Then, the viral solution was removed from the petri dish, and the cells were washed twice with Opti-MEM medium. The aforementioned transfection solution was mixed with 800 µl of Opit-MEM, and then added to the washed cells. The cells were cultivated in a 5% CO₂ incubator at 37 °C. After 4 hours, the medium was removed, and MEM medium containing 10% fetal bovine serum was added to the petri dish. After incubating in a 5% CO₂ incubator at 37 °C for 2 days, these infected cells were subjected thrice to freeze-thawing to harvest the virus.

The harvested virus solution contained about 10⁶ virus per ml and about 0.1% of which was the recombinant virus. Plaque isolation method described below was used for isolating the recombinant virus. The virus solution was diluted 10⁵ times. Separately, rabbit kidney-derived cell line RK-13 (Rikagaku Kenkyusho Saibou Kaihatu Ginko RCB0183) was plated at 2 x 10⁵ cells per 10 cm petri dish, and cultivated. After the medium was removed completely, 1 ml of the above virus solution diluted 10⁵ times was added to each petri dish. In order to prevent the drying of the cells, the petri dish was slanted at every 15 min so that the surface was covered with the virus solution. After the cells were thus infected with the virus for 1 hour, MEM medium containing 2% fetal bovine serum was added to each petri dish, and the cells were cultivated in a 5% CO₂ incubator at 37 °C.

After two days, the medium was aspirated to remove the virus solution completely. Three ml of 1% domestic fowl erythrocyte solution was then added slowly to each petri dish, allowed to adsorb for 1 hour at room temperature, and then aspirated completely. The plaques which did not adsorb the domestic fowl erythrocyte were aspirated with pipette, and suspended in 1 ml of PBS by pipetting. This procedure (comprising infection, cultivation for 2 days, and isolation of recombinant virus) is referred herein as the plaque purification procedure. Two µl of the above virus suspension was subjected to the same plaque purification procedure. This procedure was repeated thrice to obtain a recombinant virus rVV5CL containing the HCV-derived gene and the luciferase gene which is free of contamination from a wild strain.

### (3) Expression of the hepatitis virus C gene and the luciferase gene by the recombinant vaccinia virus rVV5CL

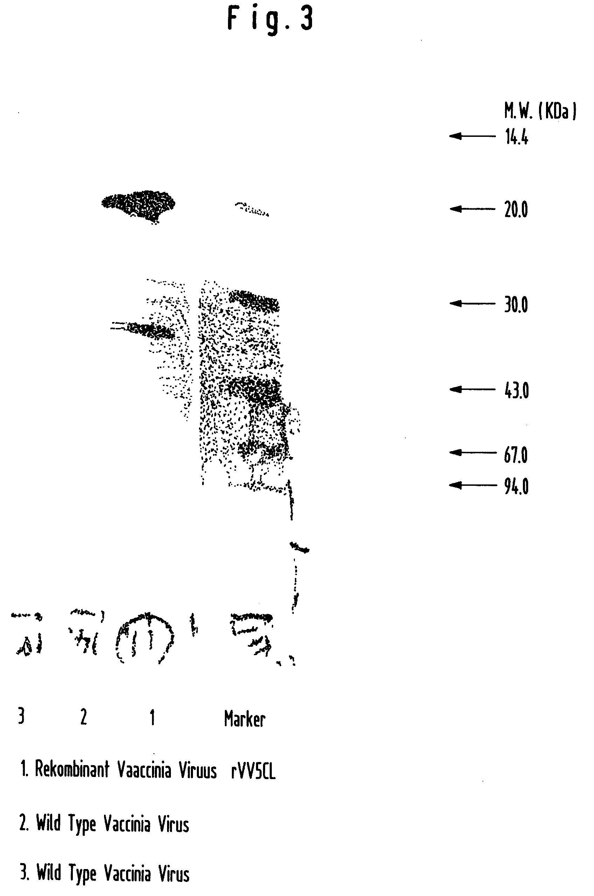

Human liver-derived cell line WRL68 (fetal human liver cell, ATCC CL68) which had been cultivated on a 24-well plate to about 60% confluent in TS-2 medium containing 10% fetal bovine serum was infected at MOI = 4 PFU/cell for 1 hour at room temperature with the recombinant vaccinia virus rVV5CL which was mixed homogeneously with PBS containing 2% fetal bovine serum. At the end of this period, the cells were washed twice with 500 µl of Opti-MEM medium, and then cultivated in 500 µl of Opti-MEM medium in a 5% CO₂ incubator at 37 °C for 16 hours. The medium was then removed, and the infected cells were lysed by adding 100 µl of SDS loading buffer described above. Twenty µl of the lysate was boiled, and then subjected to electrophoresis on 12.5% SDS-PAGE according to the conventional technique. Western blotting onto a nitrocellulose filter was then carried out according to the conventional technique. Color development was accomplished by using anti-HCV core antibody in the similar manner to that described in European Patent Publication 518,313. The result is shown in Fig. 3. As can be seen from the figure, the about 22KDa HCV core protein was detected as a major band, indicating that the fusion protein between the HCV core protein and the luciferase protein was expressed in the infected cells and processed by intracellular signal peptidase which recognizes the signal sequence present at the C terminal of the HCV core protein. The protein bands larger than 22 KDa are considered to be derived from said fusion protein which were not processed sufficiently, because a control run using non-recombinant wild type vaccinia virus didn't show such bands. Thus, the bands detected herein are believed to be derived from a fusion protein between the HCV core protein and the luciferase protein which was expressed in the cells by the recombinant vaccinia virus rVV5CL.

Furthermore, the cell lysate and the color development solution which are supplied along with PicaGene™ kit (Toyo Ink) were used in order to detect the expression of the luciferase protein in the infected cells. To infected cells cultivated as described above was added 500 µl of said cell lysate solution instead of the SDS loading buffer, and the mixture was allowed to stand for 30 min at room temperature. Five µl of the above mixture was then added to 80 µl of said color development solution, and 10 seconds after, the mixture was measured on MULTI-BIOLUMAT LB9505C (Berthold Japan). As a result, the protein more than about 10⁵ per 5 µl of the cell lysate was expressed as compared to that with uninfected cells (background).

### Example 6: Inhibitory effect on intracellular translation of the HCV gene by antisense compounds

### (1) Synthesis of antisense compounds

Antisense DNAs prepared as described below were used in this experiment.

From the region begining from thymine at position 27 and ending at adenine at position 859, a lot of specific sequences consisting of about 15-30 bases to which antisense compounds are to be hybridized were set up, and the complementary sequences determined by such specified base sequences were used as the sequences of antisense oligonucleotides. The antisense oligonucleotides were synthesized in phosphorothioate type using Applied Biosystems DNA Synthesizer Model 394. The protective groups on the bases which were added during the synthesis were removed according to the protocol provided by the manufacturer. The synthesized oligonucleotides of intended length were purified by HPLC. Although they are not separated in a single peak as in the case of phosphodiester-type oligonucleotides, all of the phosphorothioate type diastereomers of intended length were combined into one lot. The protective group on the hydroxy group at the 5'-terminal (dimethoxytrityl group) was then deprotected with acetic acid aqueous solution according to the conventional method to obtain a desired antisense compound.

Such antisense compounds were dissolved in sterile water which was prepared by subjecting ultrapure water (Milli-XQ, Millipore, water of about 18.3MΩ^{·}cm) to autoclave. The concentration was quantified from absorbance at 260 nm using the nearest-neighbor method (Methods in Enzymology, 1989, Academic Press, Vol.180, 304-325). The solutions of antisense compounds were further sterilized with UFC3 OGVOS (Millipore).

The sequences of the antisense compounds thus synthesized are shown below.

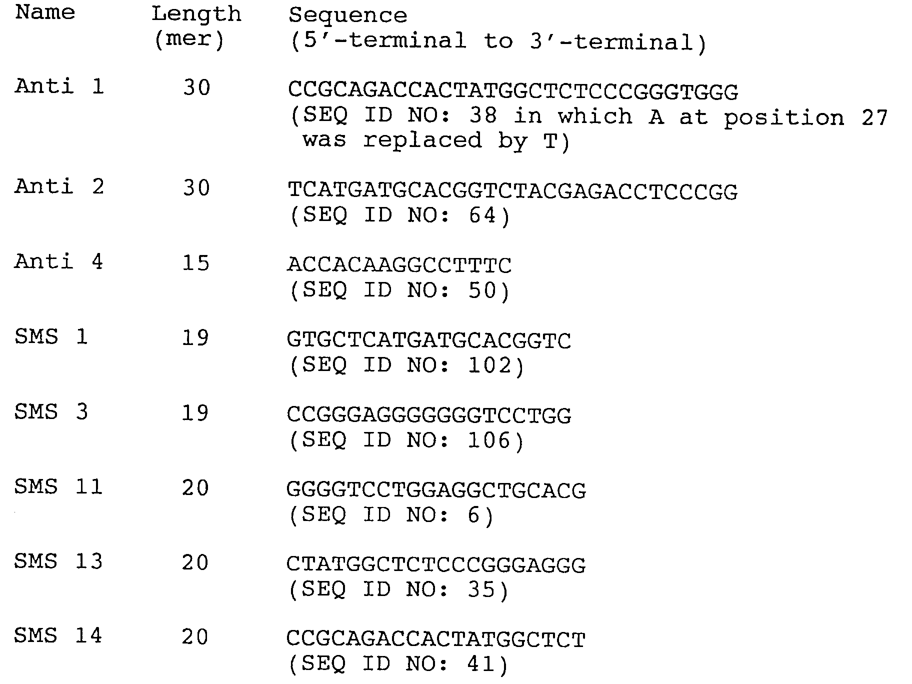

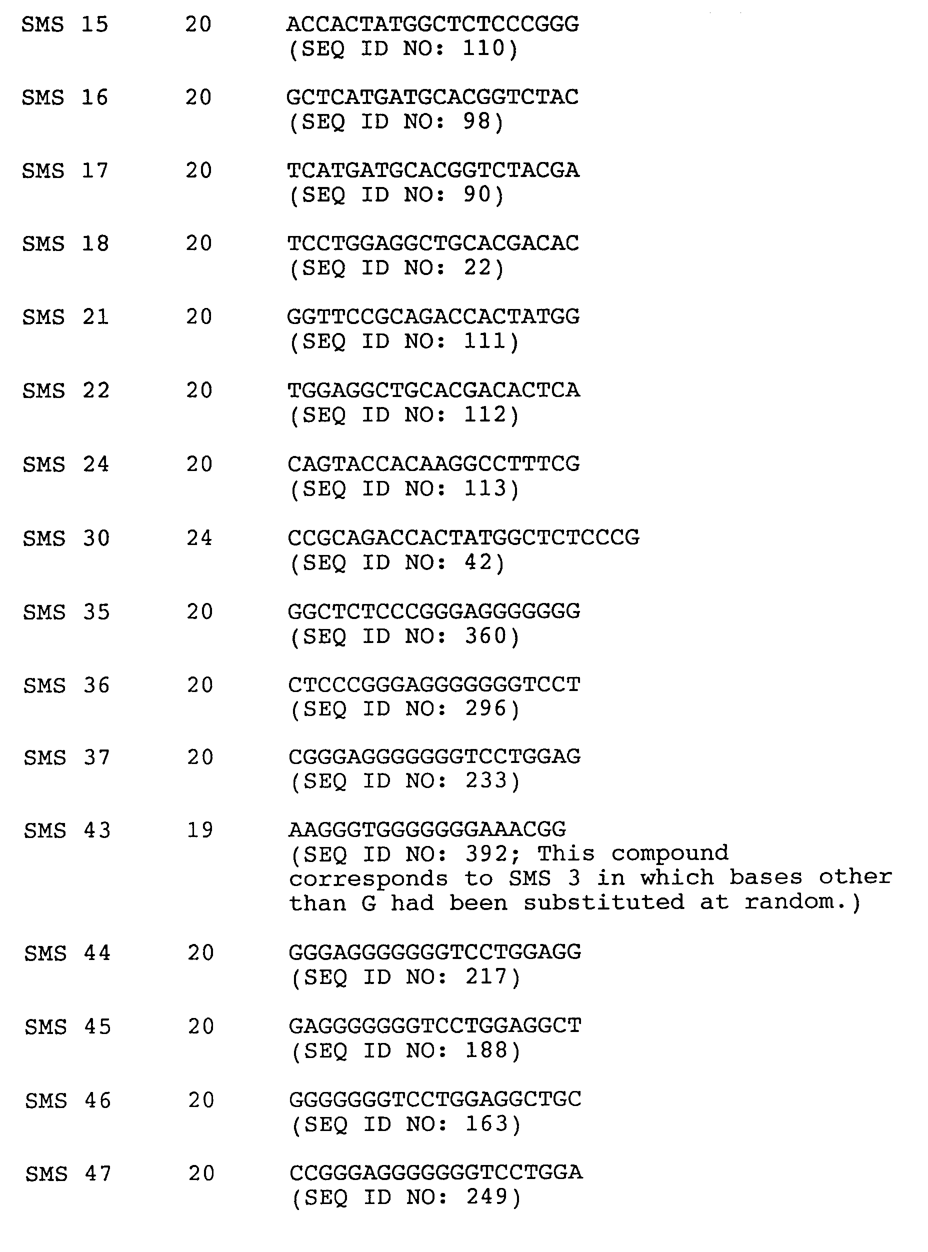

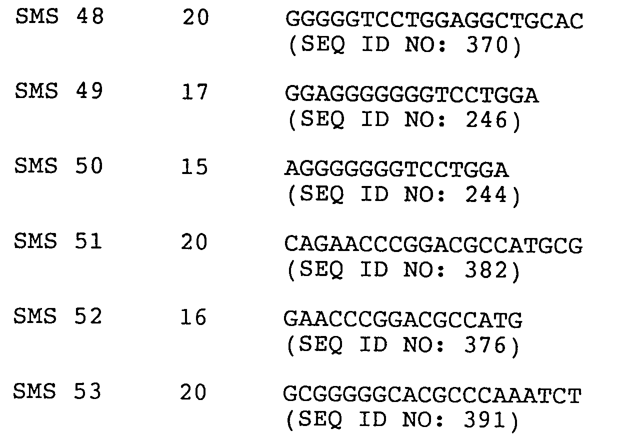

In addition, the following antisense compounds were prepared as controls.

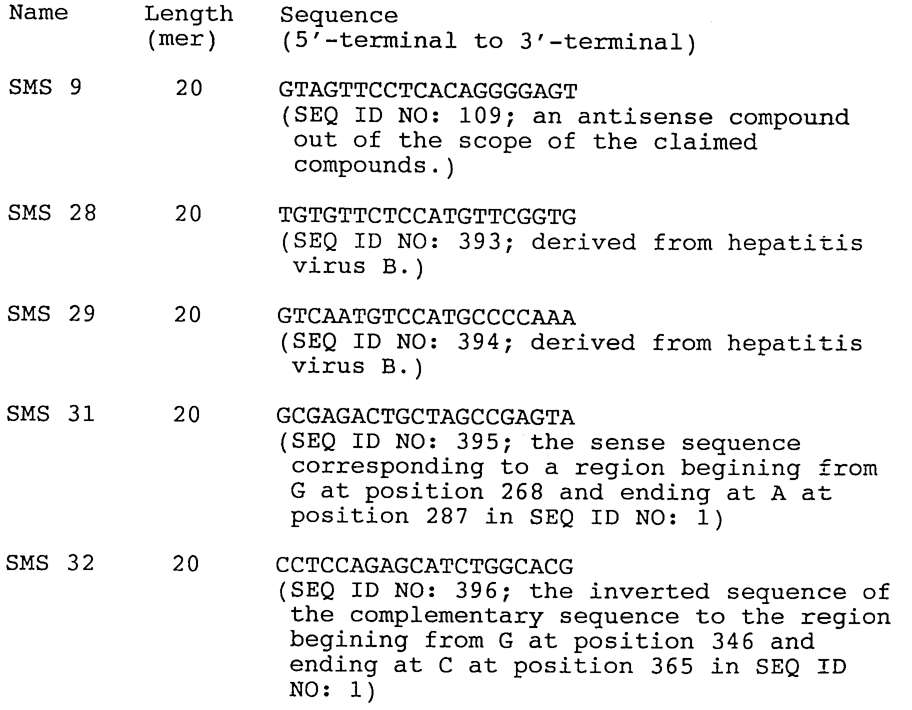

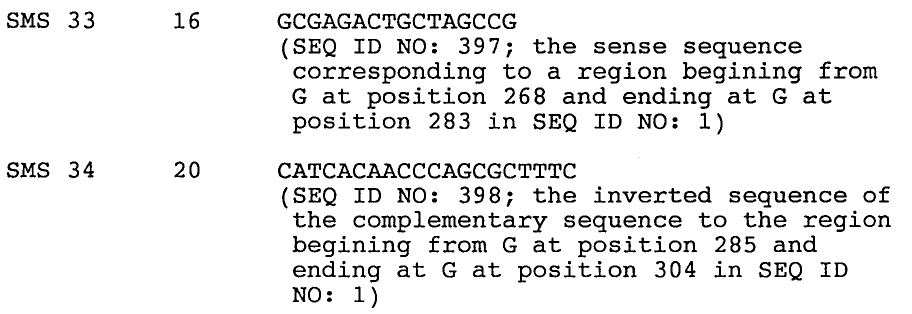

The phosphate diester linkages between bases in the above listed compounds are all phosphorothioate type.

### (2) Measurement of inhibitory effect on intracellular translation of the HCV-derived protein by antisense DNAs

Human liver-derived cell line WRL68 which had been cultivated on a 24-well plate to about 60% confluent in TS-2 medium containing 10% fetal bovine serum was infected at MOI = 0.01 PFU/cell for 1 hour at room temperature with the recombinant vaccinia virus rVV5CL which was mixed homogeneously with PBS containing 2% fetal bovine serum. At the end of this period, the cells were washed twice with 500 µl of Opti-MEM medium, and then cultivated in 500 µl of Opti-MEM medium supplemented with an antisense compound in a 5% CO₂ incubator at 37 °C for 16 hours. As described in Example 5 (3), after removal of the medium, the infected cells were mixed with 500 µl of PicaGene™ cell lysate solution, and allowed to stand for 30 min at room temperature. After mixing thoroughly, 8 µl of the mixture was added to 80 µl of the color development solution, and ten seconds after, measured on MULTI-BIOLUMAT LB9505 (Berthold Japan) for 2.5 min at 27 °C. In order to create a calibration curve, a series of luciferase solutions diluted with PBS containing 1% BSA was prepared to have a concentration of 10⁻¹⁵, 10⁻¹⁶, 10⁻¹⁷, 10⁻¹⁸, or 10⁻¹⁹ mol/µl, and used as standard reagents. Each 8 µl of these standard reagents was mixed with 80 µl of the color development solution and measured as described above.

Since common logarithm of each luciferase concentration of standard reagents was a linear function of common logarithm of corresponding measurement (integrated value of the fluorescence), the linear line was used as a calibration curve.

The amount of luciferase expressed in the infected cells was determined from the measurement (integrated value of the fluorescence) using the calibration curve. The amount of luciferase thus determined was regarded as the amount of the fusion protein expressed from the fusion protein gene between the HCV-derived core protein and luciferase genes.

The expression of this fusion protein depends on the action of the region present in the 5' untranslated region of the HCV-derived gene which plays a role in HCV specific translation. IRES (Internal Ribosome Entry Site) is believed to reside in this region. Ribosome may recognize the HCV-specific sequence and structure so that it binds at inner part, but not at the 5' end, of the mRNA to initiate the translation of the HCV protein (this function is referred as the IRES function). The fusion protein gene used herein contains sufficient region to express in infected cells the fusion protein between the HCV-derived core protein and the luciferase via such a function. Accordingly, the target region of antisense compounds is a HCV-derived gene sequence which takes part in the IRES function. Taking into account the fact that the IRES function arises from the mechanism by which the higher structure of RNA of the HCV gene is recognized, antisense compounds which may be capable of destroying such a higher structure were also selected.

In order to deduce the IRES of said gene, the secondary structure was analyzed with the analysis program FOLD (UWGCG Software, Univ. Wisconsin) on the basis of the RNA sequence begining from the 5' untranslated region and ending at envelope 1 region of the HCV gene (corresponding to the base sequence from position 1 to position 1200 in SEQ ID NO: 1).

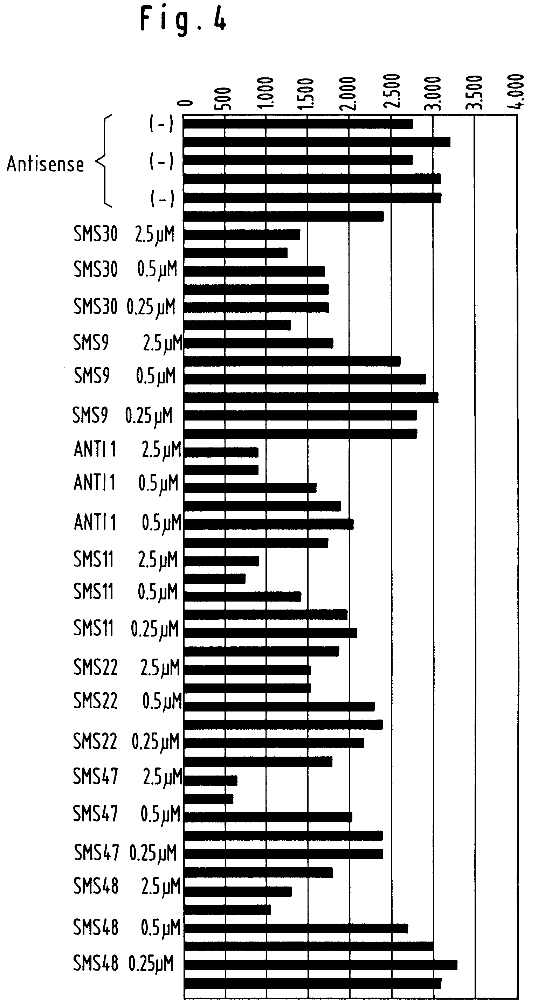

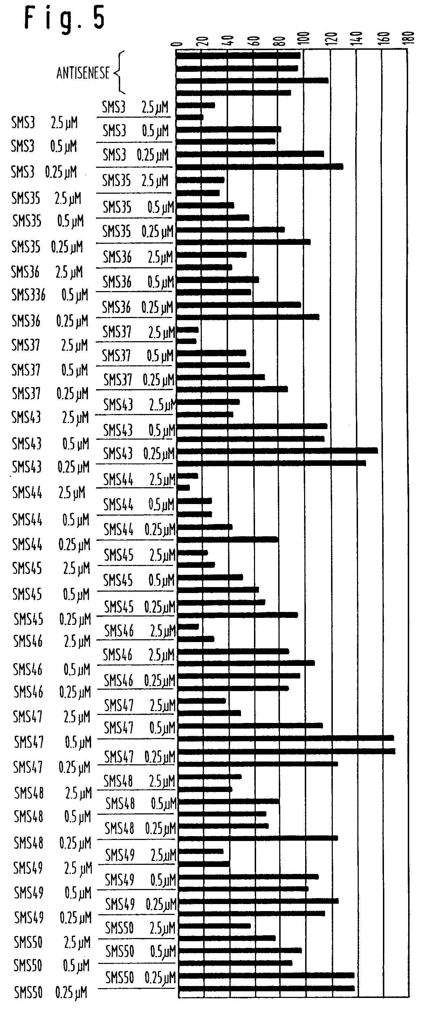

The results are shown in Figs. 4-6.

Among many antisense compounds designed herein, the antisense compounds particularly effective were those directed to the sequences in the region begining from thymine at position 107 and ending at adenine at position 199, such as Anti 1, SMS 3, SMS 11, SMS 18, SMS 22, SMS 30, SMS 35-37, SMS 44-50, and the like.

These antisense compounds were added to the medium of the infected cells at a final concentration of 5 µM, 2.5 µM, 1 µM, 0.5 µM, 0.25 µM, 0.1 µM, or 0.01 µM.

The number of samples which can be assayed under the same conditions is limitary. Accordingly, in a signal run, 6 plates (24 well) were used at the most so that experimental conditions may be kept identical. The number of the antisense compounds and the number of concentration levels to be assayed at a time is limitative for this reason, and therefore, every run was conducted with some wells (normally four wells) which are free from the antisense compound and a well which contains, in place of the antisense compound, a control compound (see Table 3) free from an activity possessed by the antisense compound. Although there was slight difference or variation among experiments with respect to cell density, infection time, and cultivation time after infection, the amount of luciferase expressed in the presence of Anti 1, SMS 1, SMS 11, SMS 35, SMS 36, or SMS 37, was about from one tenth to about one twelfth of that expressed in the presence of an antisense compound (SMS 9) which contains the sequence derived from HCV, but which is hardly effective, or an antisense compound which dose not contain HCV-derived sequence, such as SMS 28 or SMS 29. At the final concentration of 0.5 µM, Anti 1, SMS 3, SMS 11, SMS 35, SMS 36, and SMS 37 reduced the expression about 30 to 50%.

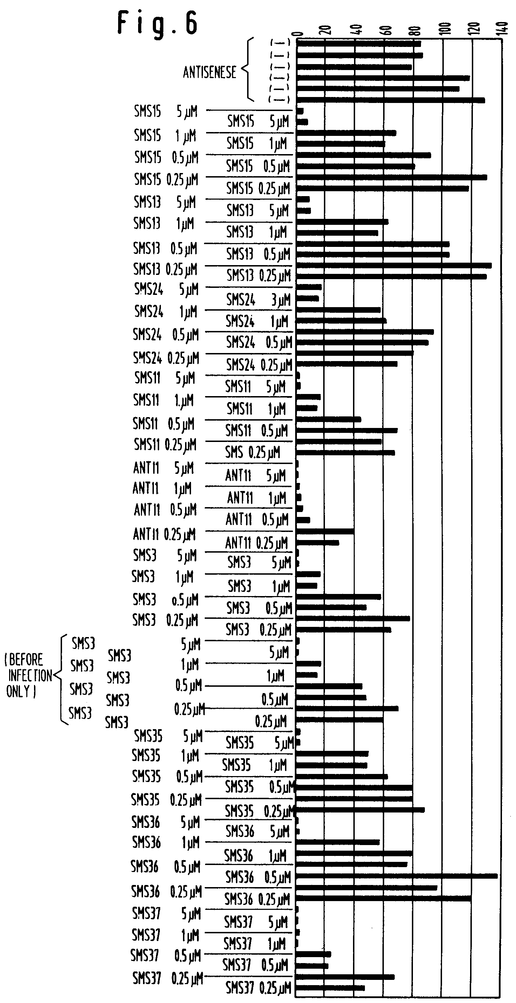

When WRL 68 cells were cultured before infection with the recombinant vaccinia virus for 1.5-2.0 hours in OPTI-MEM medium (500µℓ), to which the antisense compound of the invention had been added so that the amount of the compound was identical to that used in the case where the antisense compound was added to the medium after the WRL 68 cells were infected with the recombinant vaccinia virus, the expression inhibition was increased. Thus, the antisense compounds, such as Anti 1, SMS 3, SMS 11, SMS 35, SMS 36, and SMS 37, showed about 90-100% translation inhibition at concentrations of 5 µM, 2.5 µM, and 1 µM. In particular, Anti 1 was most effective (Fig. 6).

In summary, antisense compounds which require less than 1 µM or even less than 0.5 µM in order to exhibit about 50% or more inhibition of protein expression were discovered. It was also found that antisense compounds corresponding to a region other than a particular region in HCV polypeptide are definitely ineffective. It has been determined that said particular region corresponds to the base sequence from positions 107 to 199, preferably from 127 to 180, of the SEQ ID No. 1 of Sequence Listing. Thus, it is believed that all of the target sequences of antisense compounds are fallen within the above scope.

Because the antisense compounds of the present invention act specifically on the mRNA of HCV to inhibit the translation of HCV gene, they may be useful as an antiviral agent against HCV.

SEQ ID NO:1

SEQUENCE TYPE: nucleic acid

SEQUENCE LENGTH: 2033 base pairs

STRANDNESS: double

TOPOLOGY: linear

ANTI-SENSE: No

ORIGINAL SOURCE:

ORGANISM: Hepatitis C virus

IMMEDIATE SOURCE:

CLONE: T7N1-19

ACTAGTTAAT ACGACTCACT ATAGGGTGCC AGCCCCCTGA TGGGGGCGAC ACTCCACCAT 60

AGATCACTCC CCTGTGAGGA ACTACTGTCT TCACGCAGAA AGCGTCTAGC CATGGCGTTA 120

GTATGAGTGT CGTGCAGCCT CCAGGACCCC CCCTCCCGGG AGAGCCATAG TGGTCTGCGG 180

AACCGGTGAG TACACCGGAA TTGCCAGGAC GACCGGGTCC TTTCTTGGAT CAACCCGCTC 240

AATGCCTGGA GATTTGGGCG TGCCCCCGCG AGACTGCTAG CCGAGTAGTG TTGGGTCGCG 300

AAAGGCCTTG TGGTACTGCC TGATAGGGTG CTTGCGAGTG CCCCGGGAGG TCTCGTAGAC 360

CGTGCATC ATG AGC ACA AAT CCA AAA CCC CAA AGA AAA ATC AAA CGT AAC 410

Met Ser Thr Asn Pro Lys Pro Gln Arg Lys Ile Lys Arg Asn

1               5                   10

ACC AAC CGC CGC CCA CAG GAC GTT AAG TTC CCG GGC GGT GGT CAG ATC 458

Thr Asn Arg Arg Pro Gln Asp Val Lys Phe Pro Gly Gly Gly Gln Ile

15                  20                  25                  30

GTT GGT GGA GTT TAC CTG TTG CCG CGC AGG GGC CCC AGG TTG GGT GTG 506
Val Gly Gly Val Tyr Leu Leu Pro Arg Arg Gly Pro Arg Leu Gly Val
                35                  40                  45
CGC GCG ACT AGG AAG ACT TCC GAG CGG CCG CAA CCT CGT GGA AGG CGA 554
Arg Ala Thr Arg Lys Thr Ser Glu Arg Pro Gln Pro Arg Gly Arg Arg
            50                  55                  60
CAA CCT ATC CCC AAG GCT CGC CAA CCC GAG GGT AGG GCC TGG GCT CAG 602
Gln Pro Ile Pro Lys Ala Arg Gln Pro Glu Gly Arg Ala Trp Ala Gln
        65                  70                  75
CCC GGG TAC CCT TGG CCC CTC TAT GGC AAT GAG GGC TTG GGG TGG GCA 650
Pro Gly Tyr Pro Trp Pro Leu Tyr Gly Asn Glu Gly Leu Gly Trp Ala
    80                  85                  90
GGA TGG CTC CTG TCA CCC CGC GGC TCC CGG CCT AGT TGG GGC CCC ACG 698
Gly Trp Leu Leu Ser Pro Arg Gly Ser Arg Pro Ser Trp Gly Pro Thr
95                  100                 105                 110
GAC CCC CGG CGT AGG TCG CGT AAT TTG GGT AAG GTC ATC GAT ACC CTC 746
Asp Pro Arg Arg Arg Ser Arg Asn Leu Gly Lys Val Ile Asp Thr Leu
                115                 120                 125
ACA TGC GGC TTC GCC GAC CTC ATG GGG TAC ATT CCG CTC GTC GGC GCC 794
Thr Cys Gly Phe Ala Asp Leu Met Gly Tyr Ile Pro Leu Val Gly Ala
            130                 135                 140
CCC CTA GGG GGC GCT GCC AGG GCT CTA GCG CAT GGC GTC CGG GTT CTG 842
Pro Leu Gly Gly Ala Ala Arg Ala Leu Ala His Gly Val Arg Val Leu
        145                 150                 155

GAG GAC GGC GTG AAC TAT GCA ACA GGG AAT CTG CCT GGT TGC TCC TTT 890
Glu Asp Gly Val Asn Tyr Ala Thr Gly Asn Leu Pro Gly Cys Ser Phe
    160                 165                 170
TCT ATC TTC CTT TTG GCT TTG CTG TCC TGT TTG ACC ATC CCA GCT TCC 938
Ser Ile Phe Leu Leu Ala Leu Leu Ser Cys Leu Thr Ile Pro Ala Ser
175                 180                 185                 190
GCC TAC CAA GTG CGC AAC GCG TCC GGG GTG TAC CAT GTC ACG AAC GAC 986
Ala Tyr Gln Val Arg Asn Ala Ser Gly Val Tyr His Val Thr Asn Asp
                195                 200                 205
TGC TCC AAC TCA AGT ATT GTG TAT GAG GCG GCG GAC GTG ATT ATG CAC 1034
Cys Ser Asn Ser Ser Ile Val Tyr Glu Ala Ala Asp Val Ile Met His
            210                 215                 220
ACC CCC GGG TGC GTG CCC TGC GTC CGG GAG AAC AAT TCC TCC CGC TGC 1082
Thr Pro Gly Cys Val Pro Cys Val Arg Glu Asn Asn Ser Ser Arg Cys
        225                 230                 235
TGG GTA GCG CTC ACT CCC ACG CTT GCG GCC AGG AAC AGC AGC ATC CCC 1130
Trp Val Ala Leu Thr Pro Thr Leu Ala Ala Arg Asn Ser Ser Ile Pro
    240                 245                 250
ACT ACG ACA ATA CGG CGT CAT GTC GAC TTG CTC GTT GGG GCA GCT GCT 1178
Thr Thr Thr Ile Arg Arg His Val Asp Leu Leu Val Gly Ala Ala Ala
255                 260                 265                 270
CTC TGT TCC GCT ATG TAT GTG GGG GAT TTT TGC GGA TCT GTT TTC CTC 1226
Leu Cys Ser Ala Met Tyr Val Gly Asp Phe Cys Gly Ser Val Phe Leu
                275                 280                 285

GTC TCC CAG CTG TTC ACT TTC TCA CCT CGC CGG TAT GAG ACG GTG CAA 1274
Val Ser Gln Leu Phe Thr Phe Ser Pro Arg Arg Tyr Glu Thr Val Gln
            290                 295                 300
GAC TGC AAT TGC TCA ATC TAT CCC GGC CAT GTA TCA GGC CAT CGC ATG 1322
Asp Cys Asn Cys Ser Ile Tyr Pro Gly His Val Ser Gly His Arg Met
        305                 310                 315
GCT TGG GAT ATG ATA ATG AAT TGG TCA CCT ACA ACA GCC CTA GTG GTA 1370
Ala Trp Asp Met Ile Met Asn Trp Ser Pro Thr Thr Ala Leu Val Val
    320                 325                 330
TCG CAG CTA CTC CGG ATC CCA CAA GCC GTG GTG GAT ATG GTG GCA GGG 1418
Ser Gln Leu Leu Arg Ile Pro Gln Ala Val Val Asp Met Val Ala Gly
335                 340                 345                 350
GCC CAC TGG GGA GTC CTG GCG GGC CTT GCC TAC TAT TCC ATG GTG GGG 1466
Ala His Trp Gly Val Leu Ala Gly Leu Ala Tyr Tyr Ser Met Val Gly
                355                 360                 365
AAC TGG GCT AAG GTC TTG GTT GTG ATG CTG CTC TTC GCC GGT GTT GAC 1514
Asn Trp Ala Lys Val Leu Val Val Met Leu Leu Phe Ala Gly Val Asp
            370                 375                 380
GGG GGG ACC CAC GTG ACA GGG GGG AAG GTA GCC TAC ACC ACC CAG GGC 1562
Gly Gly Thr His Val Thr Gly Gly Lys Val Ala Tyr Thr Thr Gln Gly
        385                 390                 395
TTT ACA TCC TTC TTT TCA CGA GGG CCG TCT CAG AAA ATC CAA CTT GTA 1610
Phe Thr Ser Phe Phe Ser Arg Gly Pro Ser Gln Lys Ile Gln Leu Val
    400                 405                 410

AAC ACT AAC GGC AGC TGG CAC ATC AAT AGG ACT GCC CTC AAT TGC AAT 1658
Asn Thr Asn Gly Ser Trp His Ile Asn Arg Thr Ala Leu Asn Cys Asn
415                 420                 425                 430
GAC TCC CTT AAC ACC GGG TTC CTT GCC GCG CTG TTC TAC ACC CAC AGC 1706
Asp Ser Leu Asn Thr Gly Phe Leu Ala Ala Leu Phe Tyr Thr His Ser
                435                 440                 445
TTC AAC GCG TCC GGA TGT CCG GAG CGT ATG GCC GGT TGC CGC CCC ATT 1754
Phe Asn Ala Ser Gly Cys Pro Glu Arg Met Ala Gly Cys Arg Pro Ile
            450                 455                 460
GAC GAG TTC GCT CAG GGG TGG GGT CCC ATC ACT CAT GTT GTG CCT AAC 1802
Asp Glu Phe Ala Gln Gly Trp Gly Pro Ile Thr His Val Val Pro Asn
        465                 470                 475
ATC TCG GAC CAG AGG CCC TAT TGC TGG CAC TAC GCG CCT CGA CCG TGT 1850
Ile Ser Asp Gln Arg Pro Tyr Cys Trp His Tyr Ala Pro Arg Pro Cys
    480                 485                 490
GGT ATC GTA CCC GCG TCG CAG GTG TGT GGT CCG GTG TAT TGC TTC ACC 1898
Gly Ile Val Pro Ala Ser Gln Val Cys Gly Pro Val Tyr Cys Phe Thr
495                 500                 505                 510
CCA AGC CCT GTT GTG GTG GGG ACG ACC GAT CGT TTC GGC GCC CCC ACG 1946
Pro Ser Pro Val Val Val Gly Thr Thr Asp Arg Phe Gly Ala Pro Thr
                515                 520                 525
TAC AAC TGG GGA AAC AAT GAG ACG GAT GTG CTA CTC CTC AAC AAC ACA 1994
Tyr Asn Trp Gly Asn Asn Glu Thr Asp Val Leu Leu Leu Asn Asn Thr
            530                 535                 540

CGG CCG CCG CAG GGC AAC TGG TTC GGT TGT ACC TGG ATG 2033
Arg Pro Pro Gln Gly Asn Trp Phe Gly Cys Thr Trp Met
        545                 550                 555

SEQ ID NO:2
SEQUENCE TYPE: nucleic acid
SEQUENCE LENGTH: 16 base pairs
STRANDNESS: single
TOPOLOGY: linear
MOLECULAR TYPE: other nucleic acid
ANTI-SENSE: Yes

TCCTGGAGGC TGCACG 16

SEQ ID NO:3
SEQUENCE TYPE: nucleic acid
SEQUENCE LENGTH: 17 base pairs
STRANDNESS: single
TOPOLOGY: linear
MOLECULAR TYPE: other nucleic acid
ANTI-SENSE: Yes

GTCCTGGAGG CTGCACG 17

SEQ ID NO:4
SEQUENCE TYPE: nucleic acid
SEQUENCE LENGTH: 18 base pairs
STRANDNESS: single
TOPOLOGY: linear
MOLECULAR TYPE: other nucleic acid
ANTI-SENSE: Yes

GGTCCTGGAG GCTGCACG 18

SEQ ID NO:5
SEQUENCE TYPE: nucleic acid
SEQUENCE LENGTH: 19 base pairs
STRANDNESS: single
TOPOLOGY: linear
MOLECULAR TYPE: other nucleic acid
ANTI-SENSE: Yes

GGGTCCTGGA GGCTGCACG 19

SEQ ID NO:6
SEQUENCE TYPE: nucleic acid

SEQUENCE LENGTH:  20 base pairs
STRANDNESS:  single
TOPOLOGY:  linear
MOLECULAR TYPE:  other nucleic acid
ANTI-SENSE:  Yes

GGGGTCCTGG AGGCTGCACG                                                    20

SEQ ID NO:7
SEQUENCE TYPE:  nucleic acid
SEQUENCE LENGTH:  17 base pairs
STRANDNESS:  single
TOPOLOGY:  linear
MOLECULAR TYPE:  other nucleic acid
ANTI-SENSE:  Yes

TCCTGGAGGC TGCACGA                                                       17

SEQ ID NO:8
SEQUENCE TYPE:  nucleic acid
SEQUENCE LENGTH:  18 base pairs
STRANDNESS:  single
TOPOLOGY:  linear

MOLECULAR TYPE: other nucleic acid

ANTI-SENSE: Yes

GTCCTGGAGG CTGCACGA 18

SEQ ID NO:9

SEQUENCE TYPE: nucleic acid

SEQUENCE LENGTH: 19 base pairs

STRANDNESS: single

TOPOLOGY: linear

MOLECULAR TYPE: other nucleic acid

ANTI-SENSE: Yes

GGTCCTGGAG GCTGCACGA 19

SEQ ID NO:10

SEQUENCE TYPE: nucleic acid

SEQUENCE LENGTH: 20 base pairs

STRANDNESS: single

TOPOLOGY: linear

MOLECULAR TYPE: other nucleic acid

ANTI-SENSE: Yes

GGGTCCTGGA GGCTGCACGA 20

SEQ ID NO:11
SEQUENCE TYPE: nucleic acid
SEQUENCE LENGTH: 21 base pairs
STRANDNESS: single
TOPOLOGY: linear
MOLECULAR TYPE: other nucleic acid
ANTI-SENSE: Yes

GGGGTCCTGG AGGCTGCACG A 21

SEQ ID NO:12
SEQUENCE TYPE: nucleic acid
SEQUENCE LENGTH: 18 base pairs
STRANDNESS: single
TOPOLOGY: linear
MOLECULAR TYPE: other nucleic acid
ANTI-SENSE: Yes

TCCTGGAGGC TGCACGAC 18

SEQ ID NO:13

SEQUENCE TYPE:  nucleic acid

SEQUENCE LENGTH:  19 base pairs

STRANDNESS:  single

TOPOLOGY:  linear

MOLECULAR TYPE:  other nucleic acid

ANTI-SENSE:  Yes

GTCCTGGAGG CTGCACGAC                                                    19

SEQ ID NO:14

SEQUENCE TYPE:  nucleic acid

SEQUENCE LENGTH:  20 base pairs

STRANDNESS:  single

TOPOLOGY:  linear

MOLECULAR TYPE:  other nucleic acid

ANTI-SENSE:  Yes

GGTCCTGGAG GCTGCACGAC                                                   20

SEQ ID NO:15

SEQUENCE TYPE:  nucleic acid

SEQUENCE LENGTH:  21 base pairs

STRANDNESS:  single
TOPOLOGY:  linear
MOLECULAR SEQUENCE TYPE:  other nucleic acid
ANTI-SENSE:  Yes

GGGTCCTGGA GGCTGCACGA C                                    21

SEQ ID NO:16
SEQUENCE TYPE:  nucleic acid
SEQUENCE LENGTH:  22 base pairs
STRANDNESS:  single
ANTI-SENSE:  Yes
TOPOLOGY:  linear
MOLECULAR SEQUENCE TYPE:  other nucleic acid

GGGGTCCTGG AGGCTGCACG AC                                   22

SEQ ID NO:17
SEQUENCE TYPE:  nucleic acid
SEQUENCE LENGTH:  19 base pairs
STRANDNESS:  single
TOPOLOGY:  linear
MOLECULAR SEQUENCE TYPE:  other nucleic acid

ANTI-SENSE: Yes

TCCTGGAGGC TGCACGACA 19

SEQ ID NO:18

SEQUENCE TYPE: nucleic acid

SEQUENCE LENGTH: 20 base pairs

STRANDNESS: single

TOPOLOGY: linear

MOLECULAR SEQUENCE TYPE: other nucleic acid

ANTI-SENSE: Yes

GTCCTGGAGG CTGCACGACA 20

SEQ ID NO:19

SEQUENCE TYPE: nucleic acid

SEQUENCE LENGTH: 21 base pairs

STRANDNESS: single

TOPOLOGY: linear

MOLECULAR SEQUENCE TYPE: other nucleic acid

ANTI-SENSE: Yes

GGTCCTGGAG GCTGCACGAC A 21

SEQ ID NO:20
SEQUENCE TYPE: nucleic acid
SEQUENCE LENGTH: 22 base pairs
STRANDNESS: single
TOPOLOGY: linear
MOLECULAR SEQUENCE TYPE: other nucleic acid
ANTI-SENSE: Yes

GGGTCCTGGA GGCTGCACGA CA 22

SEQ ID NO:21
SEQUENCE TYPE: nucleic acid
SEQUENCE LENGTH: 23 base pairs
STRANDNESS: single
TOPOLOGY: linear
MOLECULAR SEQUENCE TYPE: other nucleic acid
ANTI-SENSE: Yes

GGGGTCCTGG AGGCTGCACG ACA 23

SEQ ID NO:22
SEQUENCE TYPE: nucleic acid

SEQUENCE LENGTH:  20 base pairs
STRANDNESS:  single
TOPOLOGY:  linear
MOLECULAR SEQUENCE TYPE:  other nucleic acid
ANTI-SENSE:  Yes

TCCTGGAGGC TGCACGACAC                                                    20

SEQ ID NO:23
SEQUENCE TYPE:  nucleic acid
SEQUENCE LENGTH:  21 base pairs
STRANDNESS:  single
TOPOLOGY:  linear
MOLECULAR SEQUENCE TYPE:  other nucleic acid
ANTI-SENSE:  Yes

GTCCTGGAGG CTGCACGACA C                                                  21

SEQ ID NO:24
SEQUENCE TYPE:  nucleic acid
SEQUENCE LENGTH:  22 base pairs
STRANDNESS:  single
TOPOLOGY:  linear

MOLECULAR SEQUENCE TYPE:  other nucleic acid
ANTI-SENSE:  Yes

GGTCCTGGAG GCTGCACGAC AC                                                    22

SEQ ID NO:25
SEQUENCE TYPE:  nucleic acid
SEQUENCE LENGTH:  23 base pairs
STRANDNESS:  single
TOPOLOGY:  linear
MOLECULAR SEQUENCE TYPE:  other nucleic acid
ANTI-SENSE:  Yes

GGGTCCTGGA GGCTGCACGA CAC                                                   23

SEQ ID NO:26
SEQUENCE TYPE:  nucleic acid
SEQUENCE LENGTH:  24 base pairs
STRANDNESS:  single
TOPOLOGY:  linear
MOLECULAR SEQUENCE TYPE:  other nucleic acid
ANTI-SENSE:  Yes

GGGGTCCTGG AGGCTGCACG ACAC 24

SEQ ID NO:27

SEQUENCE TYPE: nucleic acid

SEQUENCE LENGTH: 15 base pairs

STRANDNESS: single

TOPOLOGY: linear

MOLECULAR SEQUENCE TYPE: other nucleic acid

ANTI-SENSE: Yes

CTCTCCCGGG AGGGG 15

SEQ ID NO:28

SEQUENCE TYPE: nucleic acid

SEQUENCE LENGTH: 17 base pairs

STRANDNESS: single

TOPOLOGY: linear

MOLECULAR SEQUENCE TYPE: other nucleic acid

ANTI-SENSE: Yes

GGCTCTCCCG GGAGGGG 17

SEQ ID NO:29

SEQUENCE TYPE:  nucleic acid

SEQUENCE LENGTH:  19 base pairs

STRANDNESS:  single

TOPOLOGY:  linear

MOLECULAR SEQUENCE TYPE:  other nucleic acid

ANTI-SENSE:  Yes

ATGGCTCTCC CGGGAGGGG                                                    19

SEQ ID NO:30

SEQUENCE TYPE:  nucleic acid

SEQUENCE LENGTH:  21 base pairs

STRANDNESS:  single

TOPOLOGY:  linear

MOLECULAR SEQUENCE TYPE:  other nucleic acid

ANTI-SENSE:  Yes

CTATGGCTCT CCCGGGAGGG G                                                 21

SEQ ID NO:31

SEQUENCE TYPE:  nucleic acid

SEQUENCE LENGTH:  23 base pairs

STRANDNESS: single

TOPOLOGY: linear

MOLECULAR SEQUENCE TYPE: other nucleic acid

ANTI-SENSE: Yes

CACTATGGCT CTCCCGGGAG GGG 24

SEQ ID NO:32

SEQUENCE TYPE: nucleic acid

SEQUENCE LENGTH: 25 base pairs

STRANDNESS: single

TOPOLOGY: linear

MOLECULAR SEQUENCE TYPE: other nucleic acid

ANTI-SENSE: Yes

ACCACTATGG CTCTCCCGGG AGGGG 25

SEQ ID NO:33

SEQUENCE TYPE: nucleic acid

SEQUENCE LENGTH: 15 base pairs

STRANDNESS: single

TOPOLOGY: linear

MOLECULAR SEQUENCE TYPE: other nucleic acid

ANTI-SENSE: Yes

GCTCTCCCGG GAGGG 15

SEQ ID NO:34
SEQUENCE TYPE: nucleic acid
SEQUENCE LENGTH: 17 base pairs
STRANDNESS: single
TOPOLOGY: linear
MOLECULAR SEQUENCE TYPE: other nucleic acid
ANTI-SENSE: Yes

TGGCTCTCCC GGGAGGG 17

SEQ ID NO:35
SEQUENCE TYPE: nucleic acid
SEQUENCE LENGTH: 20 base pairs
STRANDNESS: single
TOPOLOGY: linear
MOLECULAR SEQUENCE TYPE: other nucleic acid
ANTI-SENSE: Yes

CTATGGCTCT CCCGGGAGGG 20

SEQ ID NO:36

SEQUENCE TYPE:  nucleic acid

SEQUENCE LENGTH:  24 base pairs

STRANDNESS:  single

TOPOLOGY:  linear

MOLECULAR SEQUENCE TYPE:  other nucleic acid

ANTI-SENSE:  Yes

ACCACTATGG CTCTCCCGGG AGGG                                    24

SEQ ID NO:37

SEQUENCE TYPE:  nucleic acid

SEQUENCE LENGTH:  27 base pairs

STRANDNESS:  single

TOPOLOGY:  linear

MOLECULAR SEQUENCE TYPE:  other nucleic acid

ANTI-SENSE:  Yes

CAGACCACTA TGGCTCTCCC GGGAGGG                                 27

SEQ ID NO:38

SEQUENCE TYPE:  nucleic acid

SEQUENCE LENGTH: 30 base pairs
STRANDNESS: single
TOPOLOGY: linear
MOLECULAR SEQUENCE TYPE: other nucleic acid
ANTI-SENSE: Yes

CCGCAGACCA CTATGGCTCT CCCGGGAGGG 30

SEQ ID NO:39
SEQUENCE TYPE: nucleic acid
SEQUENCE LENGTH: 15 base pairs
STRANDNESS: single
TOPOLOGY: linear
MOLECULAR SEQUENCE TYPE: other nucleic acid
ANTI-SENSE: Yes

CCGCAGACCA CTATG 15

SEQ ID NO:40
SEQUENCE TYPE: nucleic acid
SEQUENCE LENGTH: 17 base pairs
STRANDNESS: single
TOPOLOGY: linear

MOLECULAR SEQUENCE TYPE:  other nucleic acid
ANTI-SENSE:  Yes

CCGCAGACCA CTATGGC                                                    17

SEQ ID NO:41
SEQUENCE TYPE:  nucleic acid
SEQUENCE LENGTH:  20 base pairs
STRANDNESS:  single
TOPOLOGY:  linear
MOLECULAR SEQUENCE TYPE:  other nucleic acid
ANTI-SENSE:  Yes

CCGCAGACCA CTATGGCTCT                                                 20

SEQ ID NO:42
SEQUENCE TYPE:  nucleic acid
SEQUENCE LENGTH:  24 base pairs
STRANDNESS:  single
TOPOLOGY:  linear
MOLECULAR SEQUENCE TYPE:  other nucleic acid
ANTI-SENSE:  Yes

CCGCAGACCA CTATGGCTCT CCCG 24

SEQ ID NO:43
SEQUENCE TYPE: nucleic acid
SEQUENCE LENGTH: 27 base pairs
STRANDNESS: single
TOPOLOGY: linear
MOLECULAR SEQUENCE TYPE: other nucleic acid
ANTI-SENSE: Yes

CCGCAGACCA CTATGGCTCT CCCGGGA 27

SEQ ID NO:44
SEQUENCE TYPE: nucleic acid
SEQUENCE LENGTH: 15 base pairs
STRANDNESS: single
TOPOLOGY: linear
MOLECULAR SEQUENCE TYPE: other nucleic acid
ANTI-SENSE: Yes

CGACCCAACA CTACT 15

SEQ ID NO:45
SEQUENCE TYPE: nucleic acid
SEQUENCE LENGTH: 17 base pairs
STRANDNESS: single
TOPOLOGY: linear
MOLECULAR SEQUENCE TYPE: other nucleic acid
ANTI-SENSE: Yes

CGCGACCCAA CACTACT 17

SEQ ID NO:46
SEQUENCE TYPE: nucleic acid
SEQUENCE LENGTH: 20 base pairs
STRANDNESS: single
TOPOLOGY: linear
MOLECULAR SEQUENCE TYPE: other nucleic acid
ANTI-SENSE: Yes

TTTCGCGACC CAACACTACT 20

SEQ ID NO:47
SEQUENCE TYPE: nucleic acid
SEQUENCE LENGTH: 15 base pairs

STRANDNESS:  single

TOPOLOGY:  linear

MOLECULAR SEQUENCE TYPE:  other nucleic acid

ANTI-SENSE:  Yes

GCGACCCAAC ACTAC                                                    15

SEQ ID NO:48

SEQUENCE TYPE:  nucleic acid

SEQUENCE LENGTH:  18 base pairs

STRANDNESS:  single

TOPOLOGY:  linear

MOLECULAR SEQUENCE TYPE:  other nucleic acid

ANTI-SENSE:  Yes

TTCGCGACCC AACACTAC                                                 18

SEQ ID NO:49

SEQUENCE TYPE:  nucleic acid

SEQUENCE LENGTH:  20 base pairs

STRANDNESS:  single

TOPOLOGY:  linear

MOLECULAR SEQUENCE TYPE:  other nucleic acid

ANTI-SENSE: Yes

CTTTCGCGAC CCAACACTAC 20

SEQ ID NO:50
SEQUENCE TYPE: nucleic acid
SEQUENCE LENGTH: 15 base pairs
STRANDNESS: single
TOPOLOGY: linear
MOLECULAR SEQUENCE TYPE: other nucleic acid
ANTI-SENSE: Yes

ACCACAAGGC CTTTC 15

SEQ ID NO:51
SEQUENCE TYPE: nucleic acid
SEQUENCE LENGTH: 17 base pairs
STRANDNESS: single
TOPOLOGY: linear
MOLECULAR SEQUENCE TYPE: other nucleic acid
ANTI-SENSE: Yes

ACCACAAGGC CTTTCGC 17

SEQ ID NO:52
SEQUENCE TYPE: nucleic acid
SEQUENCE LENGTH: 20 base pairs
STRANDNESS: single
TOPOLOGY: linear
MOLECULAR SEQUENCE TYPE: other nucleic acid
ANTI-SENSE: Yes

ACCACAAGGC CTTTCGCGAC 20

SEQ ID NO:53
SEQUENCE TYPE: nucleic acid
SEQUENCE LENGTH: 15 base pairs
STRANDNESS: single
TOPOLOGY: linear
MOLECULAR SEQUENCE TYPE: other nucleic acid
ANTI-SENSE: Yes

TACCACAAGG CCTTT 15

SEQ ID NO:54
SEQUENCE TYPE: nucleic acid

SEQUENCE LENGTH:  17 base pairs
STRANDNESS:  single
TOPOLOGY:  linear
MOLECULAR SEQUENCE TYPE:  other nucleic acid
ANTI-SENSE:  Yes

TACCACAAGG CCTTTCG                                                    17

SEQ ID NO:55
SEQUENCE TYPE:  nucleic acid
SEQUENCE LENGTH:  20 base pairs
STRANDNESS:  single
TOPOLOGY:  linear
MOLECULAR SEQUENCE TYPE:  other nucleic acid
ANTI-SENSE:  Yes

TACCACAAGG CCTTTCGCGA                                                 20

SEQ ID NO:56
SEQUENCE TYPE:  nucleic acid
SEQUENCE LENGTH:  15 base pairs
STRANDNESS:  single
TOPOLOGY:  linear

MOLECULAR SEQUENCE TYPE: other nucleic acid
ANTI-SENSE: Yes

AGTACCACAA GGCCT 15

SEQ ID NO:57
SEQUENCE TYPE: nucleic acid
SEQUENCE LENGTH: 17 base pairs
STRANDNESS: single
TOPOLOGY: linear
MOLECULAR SEQUENCE TYPE: other nucleic acid
ANTI-SENSE: Yes

AGTACCACAA GGCCTTT 17

SEQ ID NO:58
SEQUENCE TYPE: nucleic acid
SEQUENCE LENGTH: 20 base pairs
STRANDNESS: single
TOPOLOGY: linear
MOLECULAR SEQUENCE TYPE: other nucleic acid
ANTI-SENSE: Yes

AGTACCACAA GGCCTTTCGC 20

SEQ ID NO:59

SEQUENCE TYPE: nucleic acid

SEQUENCE LENGTH: 17 base pairs

STRANDNESS: single

TOPOLOGY: linear

MOLECULAR SEQUENCE TYPE: other nucleic acid

ANTI-SENSE: Yes

TCTACGAGAC CTCCCGG 17

SEQ ID NO:60

SEQUENCE TYPE: nucleic acid

SEQUENCE LENGTH: 20 base pairs

STRANDNESS: single

TOPOLOGY: linear

MOLECULAR SEQUENCE TYPE: other nucleic acid

ANTI-SENSE: Yes

CGGTCTACGA GACCTCCCGG 20

SEQ ID NO:61

SEQUENCE TYPE: nucleic acid

SEQUENCE LENGTH: 23 base pairs

STRANDNESS: single

TOPOLOGY: linear

MOLECULAR SEQUENCE TYPE: other nucleic acid

ANTI-SENSE: Yes

GCACGGTCTA CGAGACCTCC CGG 23

SEQ ID NO:62

SEQUENCE TYPE: nucleic acid

SEQUENCE LENGTH: 26 base pairs

STRANDNESS: single

TOPOLOGY: linear

MOLECULAR SEQUENCE TYPE: other nucleic acid

ANTI-SENSE: Yes

GATGCACGGT CTACGAGACC TCCCGG 26

SEQ ID NO:63

SEQUENCE TYPE: nucleic acid

SEQUENCE LENGTH: 28 base pairs

STRANDNESS: single

TOPOLOGY: linear

MOLECULAR SEQUENCE TYPE: other nucleic acid

ANTI-SENSE: Yes

ATGATGCACG GTCTACGAGA CCTCCCGG 28

SEQ ID NO:64

SEQUENCE TYPE: nucleic acid

SEQUENCE LENGTH: 30 base pairs

STRANDNESS: single

TOPOLOGY: linear

MOLECULAR SEQUENCE TYPE: other nucleic acid

ANTI-SENSE: Yes

TCATGATGCA CGGTCTACGA GACCTCCCGG 30

SEQ ID NO:65

SEQUENCE TYPE: nucleic acid

SEQUENCE LENGTH: 15 base pairs

STRANDNESS: single

TOPOLOGY: linear

MOLECULAR SEQUENCE TYPE: other nucleic acid

ANTI-SENSE: Yes

TCTACGAGAC CTCCC 15

SEQ ID NO:66
SEQUENCE TYPE: nucleic acid
SEQUENCE LENGTH: 18 base pairs
STRANDNESS: single
TOPOLOGY: linear
MOLECULAR SEQUENCE TYPE: other nucleic acid
ANTI-SENSE: Yes

CGGTCTACGA GACCTCCC 18

SEQ ID NO:67
SEQUENCE TYPE: nucleic acid
SEQUENCE LENGTH: 21 base pairs
STRANDNESS: single
TOPOLOGY: linear
MOLECULAR SEQUENCE TYPE: other nucleic acid
ANTI-SENSE: Yes

GCACGGTCTA CGAGACCTCC C 21

SEQ ID NO:68

SEQUENCE TYPE:  nucleic acid

SEQUENCE LENGTH:  24 base pairs

STRANDNESS:  single

TOPOLOGY:  linear

MOLECULAR SEQUENCE TYPE:  other nucleic acid

ANTI-SENSE:  Yes

GATGCACGGT CTACGAGACC TCCC                                        24

SEQ ID NO:69

SEQUENCE TYPE:  nucleic acid

SEQUENCE LENGTH:  26 base pairs

STRANDNESS:  single

TOPOLOGY:  linear

MOLECULAR SEQUENCE TYPE:  other nucleic acid

ANTI-SENSE:  Yes

ATGATGCACG GTCTACGAGA CCTCCC                                      26

SEQ ID NO:70

SEQUENCE TYPE:  nucleic acid

SEQUENCE LENGTH:  28 base pairs

STRANDNESS:  single
TOPOLOGY:  linear
MOLECULAR SEQUENCE TYPE:  other nucleic acid
ANTI-SENSE:  Yes

TCATGATGCA CGGTCTACGA GACCTCCC                                    28

SEQ ID NO:71
SEQUENCE TYPE:  nucleic acid
SEQUENCE LENGTH:  29 base pairs
STRANDNESS:  single
TOPOLOGY:  linear
MOLECULAR SEQUENCE TYPE:  other nucleic acid
ANTI-SENSE:  Yes

CTCATGATGC ACGGTCTACG AGACCTCCC                                   29

SEQ ID NO:72
SEQUENCE TYPE:  nucleic acid
SEQUENCE LENGTH:  30 base pairs
STRANDNESS:  single
TOPOLOGY:  linear
MOLECULAR SEQUENCE TYPE:  other nucleic acid

ANTI-SENSE: Yes

GCTCATGATG CACGGTCTAC GAGACCTCCC 30

SEQ ID NO:73
SEQUENCE TYPE: nucleic acid
SEQUENCE LENGTH: 15 base pairs
STRANDNESS: single
TOPOLOGY: linear
MOLECULAR SEQUENCE TYPE: other nucleic acid
ANTI-SENSE: Yes

CGGTCTACGA GACCT 15

SEQ ID NO:74
SEQUENCE TYPE: nucleic acid
SEQUENCE LENGTH: 18 base pairs
STRANDNESS: single
TOPOLOGY: linear
MOLECULAR SEQUENCE TYPE: other nucleic acid
ANTI-SENSE: Yes

GCACGGTCTA CGAGACCT 18

SEQ ID NO:75
SEQUENCE TYPE: nucleic acid
SEQUENCE LENGTH: 21 base pairs
STRANDNESS: single
TOPOLOGY: linear
MOLECULAR SEQUENCE TYPE: other nucleic acid
ANTI-SENSE: Yes

GATGCACGGT CTACGAGACC T 21

SEQ ID NO:76
SEQUENCE TYPE: nucleic acid
SEQUENCE LENGTH: 23 base pairs
STRANDNESS: single
TOPOLOGY: linear
MOLECULAR SEQUENCE TYPE: other nucleic acid

ANTI-SENSE: Yes

ATGATGCACG GTCTACGAGA CCT 23

SEQ ID NO:77

SEQUENCE TYPE:  nucleic acid
SEQUENCE LENGTH:  25 base pairs
STRANDNESS:  single
TOPOLOGY:  linear
MOLECULAR SEQUENCE TYPE:  other nucleic acid
ANTI-SENSE:  Yes

TCATGATGCA CGGTCTACGA GACCT                                    25

SEQ ID NO:78
SEQUENCE TYPE:  nucleic acid
SEQUENCE LENGTH:  26 base pairs
STRANDNESS:  single
TOPOLOGY:  linear
MOLECULAR SEQUENCE TYPE:  other nucleic acid
ANTI-SENSE:  Yes

CTCATGATGC ACGGTCTACG AGACCT                                   26

SEQ ID NO:79
SEQUENCE TYPE:  nucleic acid
SEQUENCE LENGTH:  27 base pairs
STRANDNESS:  single

TOPOLOGY: linear

MOLECULAR SEQUENCE TYPE: other nucleic acid

ANTI-SENSE: Yes

GCTCATGATG CACGGTCTAC GAGACCT 27

SEQ ID NO:80

SEQUENCE LENGTH: 29 base pairs

SEQUENCE TYPE: nucleic acid

STRANDNESS: single

TOPOLOGY: linear

MOLECULAR SEQUENCE TYPE: other nucleic acid

ANTI-SENSE: Yes

GTGCTCATGA TGCACGGTCT ACGAGACCT 29

SEQ ID NO:81

SEQUENCE TYPE: nucleic acid

SEQUENCE LENGTH: 15 base pairs

STRANDNESS: single

TOPOLOGY: linear

MOLECULAR SEQUENCE TYPE: other nucleic acid

ANTI-SENSE: Yes

GCACGGTCTA CGAGA 15

SEQ ID NO:82

SEQUENCE TYPE: nucleic acid

SEQUENCE LENGTH: 18 base pairs

STRANDNESS: single

TOPOLOGY: linear

MOLECULAR SEQUENCE TYPE: other nucleic acid

ANTI-SENSE: Yes

GATGCACGGT CTACGAGA 18

SEQ ID NO:83

SEQUENCE TYPE: nucleic acid

SEQUENCE LENGTH: 20 base pairs

STRANDNESS: single

TOPOLOGY: linear

MOLECULAR SEQUENCE TYPE: other nucleic acid

ANTI-SENSE: Yes

ATGATGCACG GTCTACGAGA 20

SEQ ID NO:84
SEQUENCE TYPE: nucleic acid
SEQUENCE LENGTH: 22 base pairs
STRANDNESS: single
TOPOLOGY: linear
MOLECULAR SEQUENCE TYPE: other nucleic acid
ANTI-SENSE: Yes

TCATGATGCA CGGTCTACGA GA 22

SEQ ID NO:85
SEQUENCE TYPE: nucleic acid
SEQUENCE LENGTH: 23 base pairs
STRANDNESS: single
TOPOLOGY: linear
MOLECULAR SEQUENCE TYPE: other nucleic acid
ANTI-SENSE: Yes

CTCATGATGC ACGGTCTACG AGA 23

SEQ ID NO:86
SEQUENCE TYPE: nucleic acid
SEQUENCE LENGTH: 24 base pairs

STRANDNESS:  single

TOPOLOGY:  linear

MOLECULAR SEQUENCE TYPE:  other nucleic acid

ANTI-SENSE:  Yes

GCTCATGATG CACGGTCTAC GAGA                                        24

SEQ ID NO:87

SEQUENCE TYPE:  nucleic acid

SEQUENCE LENGTH:  26 base pairs

STRANDNESS:  single

TOPOLOGY:  linear

MOLECULAR SEQUENCE TYPE:  other nucleic acid

ANTI-SENSE:  Yes

GTGCTCATGA TGCACGGTCT ACGAGA                                      26

SEQ ID NO:88

SEQUENCE TYPE:  nucleic acid

SEQUENCE LENGTH:  15 base pairs

STRANDNESS:  single

TOPOLOGY:  linear

MOLECULAR SEQUENCE TYPE:  other nucleic acid

ANTI-SENSE:  Yes

ATGCACGGTC TACGA                                                    15

SEQ ID NO:89
SEQUENCE TYPE:  nucleic acid
SEQUENCE LENGTH:  18 base pairs
STRANDNESS:  single
TOPOLOGY:  linear
MOLECULAR SEQUENCE TYPE:  other nucleic acid
ANTI-SENSE:  Yes

ATGATGCACG GTCTACGA                                                 18

SEQ ID NO:90
SEQUENCE TYPE:  nucleic acid
SEQUENCE LENGTH:  20 base pairs
STRANDNESS:  single
TOPOLOGY:  linear
MOLECULAR SEQUENCE TYPE:  other nucleic acid
ANTI-SENSE:  Yes

TCATGATGCA CGGTCTACGA                                               20

SEQ ID NO:91
SEQUENCE TYPE: nucleic acid
SEQUENCE LENGTH: 21 base pairs
STRANDNESS: single
TOPOLOGY: linear
MOLECULAR SEQUENCE TYPE: other nucleic acid
ANTI-SENSE: Yes

CTCATGATGC ACGGTCTACG A 21

SEQ ID NO:92
SEQUENCE TYPE: nucleic acid
SEQUENCE LENGTH: 22 base pairs
STRANDNESS: single
TOPOLOGY: linear
MOLECULAR SEQUENCE TYPE: other nucleic acid
ANTI-SENSE: Yes

GCTCATGATG CACGGTCTAC GA 22

SEQ ID NO:93
SEQUENCE TYPE: nucleic acid

SEQUENCE LENGTH:  24 base pairs
STRANDNESS:  single
TOPOLOGY:  linear
MOLECULAR SEQUENCE TYPE:  other nucleic acid
ANTI-SENSE:  Yes

GTGCTCATGA TGCACGGTCT ACGA                                    24

SEQ ID NO:94
SEQUENCE TYPE:  nucleic acid
SEQUENCE LENGTH:  15 base pairs
STRANDNESS:  single
TOPOLOGY:  linear
MOLECULAR SEQUENCE TYPE:  other nucleic acid
ANTI-SENSE:  Yes

TGATGCACGG TCTAC                                              15

SEQ ID NO:95
SEQUENCE TYPE:  nucleic acid
SEQUENCE LENGTH:  16 base pairs
STRANDNESS:  single
TOPOLOGY:  linear

MOLECULAR SEQUENCE TYPE:  other nucleic acid
ANTI-SENSE:  Yes

ATGATGCACG GTCTAC                                                16

SEQ ID NO:96
SEQUENCE TYPE:  nucleic acid
SEQUENCE LENGTH:  18 base pairs
STRANDNESS:  single
TOPOLOGY:  linear
MOLECULAR SEQUENCE TYPE:  other nucleic acid
ANTI-SENSE:  Yes

TCATGATGCA CGGTCTAC                                              18

SEQ ID NO:97
SEQUENCE TYPE:  nucleic acid
SEQUENCE LENGTH:  19 base pairs
STRANDNESS:  single
TOPOLOGY:  linear
MOLECULAR SEQUENCE TYPE:  other nucleic acid
ANTI-SENSE:  Yes

CTCATGATGC ACGGTCTAC 19

SEQ ID NO:98

SEQUENCE TYPE: nucleic acid

SEQUENCE LENGTH: 20 base pairs

STRANDNESS: single

TOPOLOGY: linear

MOLECULAR SEQUENCE TYPE: other nucleic acid

ANTI-SENSE: Yes

GCTCATGATG CACGGTCTAC 20

SEQ ID NO:99

SEQUENCE TYPE: nucleic acid

SEQUENCE LENGTH: 22 base pairs

STRANDNESS: single

TOPOLOGY: linear

MOLECULAR SEQUENCE TYPE: other nucleic acid

ANTI-SENSE: Yes

GTGCTCATGA TGCACGGTCT AC 22

SEQ ID NO:100

SEQUENCE TYPE:  nucleic acid

SEQUENCE LENGTH:  16 base pairs

STRANDNESS:  single

TOPOLOGY:  linear

MOLECULAR SEQUENCE TYPE:  other nucleic acid

ANTI-SENSE:  Yes

CTCATGATGC ACGGTC                                                    16

SEQ ID NO:101

SEQUENCE TYPE:  nucleic acid

SEQUENCE LENGTH:  17 base pairs

STRANDNESS:  single

TOPOLOGY:  linear

MOLECULAR SEQUENCE TYPE:  other nucleic acid

ANTI-SENSE:  Yes

GCTCATGATG CACGGTC                                                   17

SEQ ID NO:102

SEQUENCE TYPE:  nucleic acid

SEQUENCE LENGTH:  19 base pairs

STRANDNESS: single

TOPOLOGY: linear

MOLECULAR SEQUENCE TYPE: other nucleic acid

ANTI-SENSE: Yes

GTGCTCATGA TGCACGGTC 19

SEQ ID NO:103

SEQUENCE TYPE: nucleic acid

SEQUENCE LENGTH: 15 base pairs

STRANDNESS: single

TOPOLOGY: linear

MOLECULAR SEQUENCE TYPE: other nucleic acid

ANTI-SENSE: Yes

GCTCATGATG CACGG 15

SEQ ID NO:104

SEQUENCE TYPE: nucleic acid

SEQUENCE LENGTH: 17 base pairs

STRANDNESS: single

TOPOLOGY: linear

MOLECULAR SEQUENCE TYPE: other nucleic acid

ANTI-SENSE:  Yes

GTGCTCATGA TGCACGG                                                    17

SEQ ID NO:105
SEQUENCE TYPE:  nucleic acid
SEQUENCE LENGTH:  15 base pairs
STRANDNESS:  single
TOPOLOGY:  linear
MOLECULAR SEQUENCE TYPE:  other nucleic acid
ANTI-SENSE:  Yes

GTGCTCATGA TGCAC                                                      15

SEQ ID NO:106
SEQUENCE TYPE:  nucleic acid
SEQUENCE LENGTH:  19 base pairs
STRANDNESS:  single
TOPOLOGY:  linear
MOLECULAR SEQUENCE TYPE:  other nucleic acid
ANTI-SENSE:  Yes

CCGGGAGGGG GGGTCCTGG                                                  19

SEQ ID NO:107

SEQUENCE TYPE:  nucleic acid

SEQUENCE LENGTH:  26 base pairs

STRANDNESS:  single

TOPOLOGY:  linear

MOLECULAR SEQUENCE TYPE:  other nucleic acid

ANTI-SENSE:  Yes

TACTCACCGG TTCCGCAGAC CACTAT                                    26

SEQ ID NO:108

SEQUENCE TYPE:  nucleic acid

SEQUENCE LENGTH:  20 base pairs

STRANDNESS:  single

TOPOLOGY:  linear

MOLECULAR SEQUENCE TYPE:  other nucleic acid

ANTI-SENSE:  Yes

TCATACTAAC GCCATGGCTA                                    20

SEQ ID NO:109

SEQUENCE TYPE:  nucleic acid
SEQUENCE LENGTH:  20 base pairs
STRANDNESS:  single
TOPOLOGY:  linear
MOLECULAR SEQUENCE TYPE:  other nucleic acid
ANTI-SENSE:  Yes

GTAGTTCCTC ACAGGGGAGT                                                    20

SEQ ID NO:110
SEQUENCE TYPE:  nucleic acid
SEQUENCE LENGTH:  20 base pairs
STRANDNESS:  single
TOPOLOGY:  linear
MOLECULAR SEQUENCE TYPE:  other nucleic acid
ANTI-SENSE:  Yes

ACCACTATGG CTCTCCCGGG                                                    20

SEQ ID NO:111
SEQUENCE TYPE:  nucleic acid
SEQUENCE LENGTH:  20 base pairs
STRANDNESS:  single

TOPOLOGY: linear
MOLECULAR SEQUENCE TYPE: other nucleic acid
ANTI-SENSE: Yes

GGTTCCGCAG ACCACTATGG 20

SEQ ID NO:112
SEQUENCE TYPE: nucleic acid
SEQUENCE LENGTH: 20 base pairs
STRANDNESS: single
TOPOLOGY: linear
MOLECULAR SEQUENCE TYPE: other nucleic acid
ANTI-SENSE: Yes

TGGAGGCTGC ACGACACTCA 20

SEQ ID NO:113
SEQUENCE SEQUENCE TYPE: nucleic acid
SEQUENCE LENGTH: 20 base pairs
STRANDNESS: single
TOPOLOGY: linear
MOLECULAR SEQUENCE TYPE: other nucleic acid
ANTI-SENSE: Yes

CAGTACCACA AGGCCTTTCG 20

SEQ ID NO: 114
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

GGGGGGGTCC TGGAGGCTGC ACGACAC 27

SEQ ID NO: 115
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid
ANTI-SENSE: Yes

AGGGGGGGTC CTGGAGGCTG CACGACAC 28

SEQ ID NO: 116

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

GAGGGGGGT CCTGGAGGCT GCACGACAC 29

SEQ ID NO: 117

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

GGAGGGGGGG TCCTGGAGGC TGCACGACAC 30

SEQ ID NO: 118

SEQUENCE TYPE: nucleic acid

SEQUENCE LENGTH: 26 base pairs

STRANDEDNESS: single
TOPOLOGY: linear
MOLECULE SEQUENCE TYPE: Other nucleic acid
ANTI-SENSE: Yes

GGGGGGGTCC TGGAGGCTGC ACGACA 26

SEQ ID NO: 119
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

AGGGGGGGTC CTGGAGGCTG CACGACA 27

SEQ ID NO: 120
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid

ANTI-SENSE: Yes

GAGGGGGGGT CCTGGAGGCT GCACGACA 28

SEQ ID NO: 121
SEQUENCE TYPE: nucleic acid
SEQUENCE LENGTH: 29 base pairs
STRANDEDNESS: single
TOPOLOGY: linear
MOLECULE SEQUENCE TYPE: Other nucleic acid
ANTI-SENSE: Yes

GGAGGGGGGG TCCTGGAGGC TGCACGACA 29

SEQ ID NO: 122
SEQUENCE TYPE: nucleic acid
SEQUENCE LENGTH: 30 base pairs
STRANDEDNESS: single
TOPOLOGY: linear
MOLECULE SEQUENCE TYPE: Other nucleic acid
ANTI-SENSE: Yes

GGGAGGGGGG GTCCTGGAGG CTGCACGACA 30

SEQ ID NO: 123

SEQUENCE TYPE: nucleic acid

SEQUENCE LENGTH: 25 base pairs

STRANDEDNESS: single

TOPOLOGY: linear

MOLECULE SEQUENCE TYPE: Other nucleic acid

ANTI-SENSE: Yes

GGGGGGGTCC TGGAGGCTGC ACGAC 25

SEQ ID NO: 124

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

AGGGGGGGTC CTGGAGGCTG CACGAC 26

SEQ ID NO: 125

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

GAGGGGGGGT CCTGGAGGCT GCACGAC 27

SEQ ID NO: 126

SEQUENCE TYPE: nucleic acid

SEQUENCE LENGTH: 28 base pairs

STRANDEDNESS: single

TOPOLOGY: linear

MOLECULE SEQUENCE TYPE: Other nucleic acid

ANTI-SENSE: Yes

GGAGGGGGGG TCCTGGAGGC TGCACGAC 28

SEQ ID NO: 127

SEQUENCE TYPE: nucleic acid

SEQUENCE LENGTH: 29 base pairs

STRANDEDNESS: single

TOPOLOGY: linear

MOLECULE SEQUENCE TYPE:  Other nucleic acid
ANTI-SENSE:  Yes

GGGAGGGGGG GTCCTGGAGG CTGCACGAC                                    29

SEQ ID NO:  128
SEQUENCE TYPE:  nucleic acid
SEQUENCE LENGTH:  30 base pairs
STRANDEDNESS:  single
TOPOLOGY:  linear
MOLECULE SEQUENCE TYPE:  Other nucleic acid
ANTI-SENSE:  Yes

CGGGAGGGGG GGTCCTGGAG GCTGCACGAC                                   30

SEQ ID NO:  129
SEQUENCE TYPE:  nucleic acid
SEQUENCE LENGTH:  24 base pairs
STRANDEDNESS:  single
TOPOLOGY:  linear
MOLECULE SEQUENCE TYPE:  Other nucleic acid
ANTI-SENSE:  Yes

GGGGGGGTCC TGGAGGCTGC ACGA 24

SEQ ID NO: 130

SEQUENCE TYPE: nucleic acid

SEQUENCE LENGTH: 25 base pairs

STRANDEDNESS: single

TOPOLOGY: linear

MOLECULE SEQUENCE TYPE: Other nucleic acid

ANTI-SENSE: Yes

AGGGGGGGTC CTGGAGGCTG CACGA 25

SEQ ID NO: 131

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

GAGGGGGGGT CCTGGAGGCT GCACGA 26

SEQ ID NO: 132

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

GGAGGGGGGG TCCTGGAGGC TGCACGA 27

SEQ ID NO: 133

SEQUENCE TYPE: nucleic acid

SEQUENCE LENGTH: 28 base pairs

STRANDEDNESS: single

TOPOLOGY: linear

MOLECULE SEQUENCE TYPE: Other nucleic acid

ANTI-SENSE: Yes

GGGAGGGGGG GTCCTGGAGG CTGCACGA 28

SEQ ID NO: 134

SEQUENCE TYPE: nucleic acid

SEQUENCE LENGTH: 29 base pairs

STRANDEDNESS:  single
TOPOLOGY:  linear
MOLECULE SEQUENCE TYPE:  Other nucleic acid
ANTI-SENSE:  Yes

CGGGAGGGGG GGTCCTGGAG GCTGCACGA                                    29

SEQ ID NO:  135
SEQUENCE TYPE:  nucleic acid
SEQUENCE LENGTH:  30 base pairs
STRANDEDNESS:  single
TOPOLOGY:  linear
MOLECULE SEQUENCE TYPE:  Other nucleic acid
ANTI-SENSE:  Yes

CCGGGAGGGG GGGTCCTGGA GGCTGCACGA                                   30

SEQ ID NO:  136
SEQUENCE TYPE:  nucleic acid
SEQUENCE LENGTH:  23 base pairs
STRANDEDNESS:  single
TOPOLOGY:  linear
MOLECULE SEQUENCE TYPE:  Other nucleic acid

ANTI-SENSE:  Yes

GGGGGGGTCC TGGAGGCTGC ACG                                                    23

SEQ ID NO:  137
SEQUENCE TYPE:  nucleic acid
SEQUENCE LENGTH:  24 base pairs
STRANDEDNESS:  single
TOPOLOGY:  linear
MOLECULE SEQUENCE TYPE:  Other nucleic acid
ANTI-SENSE:  Yes

AGGGGGGGTC CTGGAGGCTG CACG                                                   24

SEQ ID NO:  138
SEQUENCE TYPE:  nucleic acid
SEQUENCE LENGTH:  25 base pairs
STRANDEDNESS:  single
TOPOLOGY:  linear
MOLECULE SEQUENCE TYPE:  Other nucleic acid
ANTI-SENSE:  Yes

GAGGGGGGGT CCTGGAGGCT GCACG                                                  25

SEQ ID NO: 139

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

GGAGGGGGGG TCCTGGAGGC TGCACG 26

SEQ ID NO: 140

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

GGGAGGGGGG GTCCTGGAGG CTGCACG 27

SEQ ID NO: 141

SEQUENCE TYPE: nucleic acid

SEQUENCE LENGTH:  28 base pairs

STRANDEDNESS:  single

TOPOLOGY:  linear

MOLECULE SEQUENCE TYPE:  Other nucleic acid

ANTI-SENSE:  Yes

CGGGAGGGGG GGTCCTGGAG GCTGCACG                                    28

SEQ ID NO:  142

SEQUENCE TYPE:  nucleic acid

SEQUENCE LENGTH:  29 base pairs

STRANDEDNESS:  single

TOPOLOGY:  linear

MOLECULE SEQUENCE TYPE:  Other nucleic acid

ANTI-SENSE:  Yes

CCGGGAGGGG GGGTCCTGGA GGCTGCACG                                   29

SEQ ID NO:  143

SEQUENCE TYPE:  nucleic acid

SEQUENCE LENGTH:  30 base pairs

STRANDEDNESS:  single

TOPOLOGY:  linear

MOLECULE SEQUENCE TYPE:  Other nucleic acid
ANTI-SENSE:  Yes

CCCGGGAGGG GGGGTCCTGG AGGCTGCACG                                30

SEQ ID NO:  144
SEQUENCE TYPE:  nucleic acid
SEQUENCE LENGTH:  22 base pairs
STRANDEDNESS:  single
TOPOLOGY:  linear
MOLECULE SEQUENCE TYPE:  Other nucleic acid
ANTI-SENSE:  Yes

GGGGGGGTCC TGGAGGCTGC AC                                        22

SEQ ID NO:  145
SEQUENCE TYPE:  nucleic acid
SEQUENCE LENGTH:  23 base pairs
STRANDEDNESS:  single
TOPOLOGY:  linear
MOLECULE SEQUENCE TYPE:  Other nucleic acid
ANTI-SENSE:  Yes

AGGGGGGGTC CTGGAGGCTG CAC 23

SEQ ID NO: 146

SEQUENCE TYPE: nucleic acid

SEQUENCE LENGTH: 24 base pairs

STRANDEDNESS: single

TOPOLOGY: linear

MOLECULE SEQUENCE TYPE: Other nucleic acid

ANTI-SENSE: Yes

GAGGGGGGGT CCTGGAGGCT GCAC 24

SEQ ID NO: 147

SEQUENCE TYPE: nucleic acid

SEQUENCE LENGTH: 25 base pairs

STRANDEDNESS: single

TOPOLOGY: linear

MOLECULE SEQUENCE TYPE: Other nucleic acid

ANTI-SENSE: Yes

GGAGGGGGGG TCCTGGAGGC TGCAC 25

SEQ ID NO: 148

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

GGGAGGGGGG GTCCTGGAGG CTGCAC 26

SEQ ID NO: 149

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

CGGGAGGGGG GGTCCTGGAG GCTGCAC 27

SEQ ID NO: 150

SEQUENCE TYPE: nucleic acid

SEQUENCE LENGTH: 28 base pairs

STRANDEDNESS: single

TOPOLOGY: linear

MOLECULE SEQUENCE TYPE: Other nucleic acid

ANTI-SENSE: Yes

CCGGGAGGGG GGGTCCTGGA GGCTGCAC 28

SEQ ID NO: 151

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

CCCGGGAGGG GGGGTCCTGG AGGCTGCAC 29

SEQ ID NO: 152

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

TCCCGGGAGG GGGGGTCCTG GAGGCTGCAC 30

SEQ ID NO: 153
SEQUENCE TYPE: nucleic acid
SEQUENCE LENGTH: 21 base pairs
STRANDEDNESS: single
TOPOLOGY: linear
MOLECULE SEQUENCE TYPE: Other nucleic acid
ANTI-SENSE: Yes

GGGGGGGTCC TGGAGGCTGC A 21

SEQ ID NO: 154
SEQUENCE TYPE: nucleic acid
SEQUENCE LENGTH: 22 base pairs
STRANDEDNESS: single
TOPOLOGY: linear
MOLECULE SEQUENCE TYPE: Other nucleic acid
ANTI-SENSE: Yes
AGGGGGGGTC CTGGAGGCTG CA 22

SEQ ID NO: 155
SEQUENCE TYPE: nucleic acid
SEQUENCE LENGTH: 23 base pairs
STRANDEDNESS: single
TOPOLOGY: linear
MOLECULE SEQUENCE TYPE: Other nucleic acid
ANTI-SENSE: Yes

GAGGGGGGGT CCTGGAGGCT GCA 23

SEQ ID NO: 156
SEQUENCE TYPE: nucleic acid
SEQUENCE LENGTH: 24 base pairs
STRANDEDNESS: single
TOPOLOGY: linear
MOLECULE SEQUENCE TYPE: Other nucleic acid
ANTI-SENSE: Yes

GGAGGGGGGG TCCTGGAGGC TGCA 24

SEQ ID NO: 157
SEQUENCE TYPE: nucleic acid
SEQUENCE LENGTH: 25 base pairs

STRANDEDNESS: single
TOPOLOGY: linear
MOLECULE SEQUENCE TYPE: Other nucleic acid
ANTI-SENSE: Yes

GGGAGGGGGG GTCCTGGAGG CTGCA 25

SEQ ID NO: 158
SEQUENCE TYPE: nucleic acid
SEQUENCE LENGTH: 26 base pairs
STRANDEDNESS: single
TOPOLOGY: linear
MOLECULE SEQUENCE TYPE: Other nucleic acid
ANTI-SENSE: Yes

CGGGAGGGGG GGTCCTGGAG GCTGCA 26

SEQ ID NO: 159
SEQUENCE TYPE: nucleic acid
SEQUENCE LENGTH: 27 base pairs
STRANDEDNESSSS: single
TOPOLOGY: linear
MOLECULE SEQUENCE TYPE: Other nucleic acid

ANTI-SENSE: Yes

CCGGGAGGGG GGGTCCTGGA GGCTGCA 27

SEQ ID NO: 160
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid
ANTI-SENSE: Yes

CCCGGGAGGG GGGGTCCTGG AGGCTGCA 28

SEQ ID NO: 161
SEQUENCE TYPE: nucleic acid
SEQUENCE LENGTH: 29 base pairs
STRANDEDNESS: single
TOPOLOGY: linear
MOLECULE SEQUENCE TYPE: Other nucleic acid
ANTI-SENSE: Yes

TCCCGGGAGG GGGGGTCCTG GAGGCTGCA 29

SEQ ID NO: 162

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

CTCCCGGGAG GGGGGGTCCT GGAGGCTGCA 30

SEQ ID NO: 163

SEQUENCE TYPE: nucleic acid

SEQUENCE LENGTH: 20 base pairs

STRANDEDNESS: single

TOPOLOGY: linear

MOLECULE SEQUENCE TYPE: Other nucleic acid

ANTI-SENSE: Yes

GGGGGGGTCC TGGAGGCTGC 20

SEQ ID NO: 164

SEQUENCE TYPE: nucleic acid

SEQUENCE LENGTH:  21 base pairs
STRANDEDNESS:  single
TOPOLOGY:  linear
MOLECULE SEQUENCE TYPE:  Other nucleic acid
ANTI-SENSE:  Yes

AGGGGGGGTC CTGGAGGCTG C                                    21

SEQ ID NO:  165
SEQUENCE TYPE:  nucleic acid
SEQUENCE LENGTH:  22 base pairs
STRANDEDNESS:  single
TOPOLOGY:  linear
MOLECULE SEQUENCE TYPE:  Other nucleic acid
ANTI-SENSE:  Yes

GAGGGGGGGT CCTGGAGGCT GC                                   22

SEQ ID NO:  166
SEQUENCE TYPE:  nucleic acid
SEQUENCE LENGTH:  23 base pairs
STRANDEDNESS:  single
TOPOLOGY:  linear

MOLECULE SEQUENCE TYPE:  Other nucleic acid
ANTI-SENSE:  Yes

GGAGGGGGGG TCCTGGAGGC TGC                                    23

SEQ ID NO:  167
SEQUENCE TYPE:  nucleic acid
SEQUENCE LENGTH:  24 base pairs
STRANDEDNESS:  single
TOPOLOGY:  linear
MOLECULE SEQUENCE TYPE:  Other nucleic acid
ANTI-SENSE:  Yes

GGGAGGGGGG GTCCTGGAGG CTGC                                   24

SEQ ID NO:  168
SEQUENCE TYPE:  nucleic acid
SEQUENCE LENGTH:  25 base pairs
STRANDEDNESS:  single
TOPOLOGY:  linear
MOLECULE SEQUENCE TYPE:  Other nucleic acid
ANTI-SENSE:  Yes

CGGGAGGGGG GGTCCTGGAG GCTGC 25

SEQ ID NO: 169

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

CCGGGAGGGG GGGTCCTGGA GGCTGC 26

SEQ ID NO: 170

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

CCCGGGAGGG GGGGTCCTGG AGGCTGC 27

SEQ ID NO: 171

SEQUENCE TYPE: nucleic acid

SEQUENCE LENGTH: 28 base pairs

STRANDEDNESS: single

TOPOLOGY: linear

MOLECULE SEQUENCE TYPE: Other nucleic acid

ANTI-SENSE: Yes

TCCCGGGAGG GGGGGTCCTG GAGGCTGC 28

SEQ ID NO: 172

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

CTCCCGGGAG GGGGGGTCCT GGAGGCTGC 29

SEQ ID NO: 173

SEQUENCE TYPE: nucleic acid

SEQUENCE LENGTH: 30 base pairs

STRANDEDNESS: single
TOPOLOGY: linear
MOLECULE SEQUENCE TYPE: Other nucleic acid
ANTI-SENSE: Yes

TCTCCCGGGA GGGGGGGTCC TGGAGGCTGC 30

SEQ ID NO: 174
SEQUENCE TYPE: nucleic acid
SEQUENCE LENGTH: 19 base pairs
STRANDEDNESS: single
TOPOLOGY: linear
MOLECULE SEQUENCE TYPE: Other nucleic acid
ANTI-SENSE: Yes

GGGGGGGTCC TGGAGGCTG 19

SEQ ID NO: 175
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid

ANTI-SENSE: Yes

AGGGGGGGTC CTGGAGGCTG 20

SEQ ID NO: 176
SEQUENCE TYPE: nucleic acid
SEQUENCE LENGTH: 21 base pairs
STRANDEDNESS: single
TOPOLOGY: linear
MOLECULE SEQUENCE TYPE: Other nucleic acid
ANTI-SENSE: Yes

GAGGGGGGGT CCTGGAGGCT G 21

SEQ ID NO: 177
SEQUENCE TYPE: nucleic acid
SEQUENCE LENGTH: 22 base pairs
STRANDEDNESS: single
TOPOLOGY: linear
MOLECULE SEQUENCE TYPE: Other nucleic acid
ANTI-SENSE: Yes

GGAGGGGGGG TCCTGGAGGC TG 22

SEQ ID NO: 178

SEQUENCE TYPE: nucleic acid

SEQUENCE LENGTH: 23 base pairs

STRANDEDNESS: single

TOPOLOGY: linear

MOLECULE SEQUENCE TYPE: Other nucleic acid

ANTI-SENSE: Yes

GGGAGGGGGG GTCCTGGAGG CTG 23

SEQ ID NO: 179

SEQUENCE TYPE: nucleic acid

SEQUENCE LENGTH: 24 base pairs

STRANDEDNESS: single

TOPOLOGY: linear

MOLECULE SEQUENCE TYPE: Other nucleic acid

ANTI-SENSE: Yes

CGGGAGGGGG GGTCCTGGAG GCTG 24

SEQ ID NO: 180

SEQUENCE TYPE: nucleic acid

SEQUENCE LENGTH:  25 base pairs
STRANDEDNESS:  single
TOPOLOGY:  linear
MOLECULE SEQUENCE TYPE:  Other nucleic acid
ANTI-SENSE:  Yes

CCGGGAGGGG GGGTCCTGGA GGCTG                                    25

SEQ ID NO:  181
SEQUENCE TYPE:  nucleic acid
SEQUENCE LENGTH:  26 base pairs
STRANDEDNESS:  single
TOPOLOGY:  linear
MOLECULE SEQUENCE TYPE:  Other nucleic acid
ANTI-SENSE:  Yes

CCCGGGAGGG GGGGTCCTGG AGGCTG                                   26

SEQ ID NO:  182
SEQUENCE TYPE:  nucleic acid
SEQUENCE LENGTH:  27 base pairs
STRANDEDNESS:  single
TOPOLOGY:  linear

MOLECULE SEQUENCE TYPE:  Other nucleic acid
ANTI-SENSE:  Yes

TCCCGGGAGG GGGGGTCCTG GAGGCTG                                    27

SEQ ID NO:  183
SEQUENCE TYPE:  nucleic acid
SEQUENCE LENGTH:  28 base pairs
STRANDEDNESS:  single
TOPOLOGY:  linear
MOLECULE SEQUENCE TYPE:  Other nucleic acid
ANTI-SENSE:  Yes

CTCCCGGGAG GGGGGGTCCT GGAGGCTG                                   28

SEQ ID NO:  184
SEQUENCE TYPE:  nucleic acid
SEQUENCE LENGTH:  29 base pairs
STRANDEDNESS:  single
TOPOLOGY:  linear
MOLECULE SEQUENCE TYPE:  Other nucleic acid
ANTI-SENSE:  Yes

TCTCCCGGGA GGGGGGGTCC TGGAGGCTG 29

SEQ ID NO: 185

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

CTCTCCCGGG AGGGGGGGTC CTGGAGGCTG 30

SEQ ID NO: 186

SEQUENCE TYPE: nucleic acid

SEQUENCE LENGTH: 18 base pairs

STRANDEDNESS: single

TOPOLOGY: linear

MOLECULE SEQUENCE TYPE: Other nucleic acid

ANTI-SENSE: Yes

GGGGGGGTCC TGGAGGCT 18

SEQ ID NO: 187
SEQUENCE TYPE: nucleic acid
SEQUENCE LENGTH: 19 base pairs
STRANDEDNESS: single
TOPOLOGY: linear
MOLECULE SEQUENCE TYPE: Other nucleic acid
ANTI-SENSE: Yes

AGGGGGGTC CTGGAGGCT 19

SEQ ID NO: 188
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

GAGGGGGGGT CCTGGAGGCT 20

SEQ ID NO: 189
SEQUENCE TYPE: nucleic acid
SEQUENCE LENGTH: 21 base pairs

STRANDEDNESS: single

TOPOLOGY: linear

MOLECULE SEQUENCE TYPE: Other nucleic acid

ANTI-SENSE: Yes

GGAGGGGGG TCCTGGAGGC T 21

SEQ ID NO: 190

SEQUENCE TYPE: nucleic acid

SEQUENCE LENGTH: 22 base pairs

STRANDEDNESS: single

TOPOLOGY: linear

MOLECULE SEQUENCE TYPE: Other nucleic acid

ANTI-SENSE: Yes

GGGAGGGGGG GTCCTGGAGG CT 22

SEQ ID NO: 191

SEQUENCE TYPE: nucleic acid

SEQUENCE LENGTH: 23 base pairs

STRANDEDNESS: single

TOPOLOGY: linear

MOLECULE SEQUENCE TYPE: Other nucleic acid

ANTI-SENSE: Yes

CGGGAGGGGG GGTCCTGGAG GCT 23

SEQ ID NO: 192
SEQUENCE TYPE: nucleic acid
SEQUENCE LENGTH: 24 base pairs
STRANDEDNESS: single
TOPOLOGY: linear
MOLECULE SEQUENCE TYPE: Other nucleic acid
ANTI-SENSE: Yes

CCGGGAGGGG GGGTCCTGGA GGCT 24

SEQ ID NO: 193
SEQUENCE TYPE: nucleic acid
SEQUENCE LENGTH: 25 base pairs
STRANDEDNESS: single
TOPOLOGY: linear
MOLECULE SEQUENCE TYPE: Other nucleic acid
ANTI-SENSE: Yes

CCCGGGAGGG GGGGTCCTGG AGGCT 25

SEQ ID NO: 194

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

TCCCGGGAGG GGGGGTCCTG GAGGCT 26

SEQ ID NO: 195

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

CTCCCGGGAG GGGGGGTCCT GGAGGCT 27

SEQ ID NO: 196

SEQUENCE TYPE: nucleic acid

SEQUENCE LENGTH: 28 base pairs
STRANDEDNESS: single
TOPOLOGY: linear
MOLECULE SEQUENCE TYPE: Other nucleic acid
ANTI-SENSE: Yes

TCTCCCGGGA GGGGGGTCC TGGAGGCT 28

SEQ ID NO: 197
SEQUENCE TYPE: nucleic acid
SEQUENCE LENGTH: 29 base pairs
STRANDEDNESS: single
TOPOLOGY: linear
MOLECULE SEQUENCE TYPE: Other nucleic acid
ANTI-SENSE: Yes

CTCTCCCGGG AGGGGGGTC CTGGAGGCT 29

SEQ ID NO: 198
SEQUENCE TYPE: nucleic acid
SEQUENCE LENGTH: 30 base pairs
STRANDEDNESS: single
TOPOLOGY: linear

MOLECULE SEQUENCE TYPE:  Other nucleic acid
ANTI-SENSE:  Yes

GCTCTCCCGG GAGGGGGGGT CCTGGAGGCT                                    30

SEQ ID NO:  199
SEQUENCE TYPE:  nucleic acid
SEQUENCE LENGTH:  17 base pairs
STRANDEDNESS:  single
TOPOLOGY:  linear
MOLECULE SEQUENCE TYPE:  Other nucleic acid
ANTI-SENSE:  Yes

GGGGGGGTCC TGGAGGC                                                 17

SEQ ID NO:  200
SEQUENCE TYPE:  nucleic acid
SEQUENCE LENGTH:  18 base pairs
STRANDEDNESS:  single
TOPOLOGY:  linear
MOLECULE SEQUENCE TYPE:  Other nucleic acid
ANTI-SENSE:  Yes

AGGGGGGGTC CTGGAGGC 18

SEQ ID NO: 201
SEQUENCE TYPE: nucleic acid
SEQUENCE LENGTH: 19 base pairs
STRANDEDNESS: single
TOPOLOGY: linear
MOLECULE SEQUENCE TYPE: Other nucleic acid
ANTI-SENSE: Yes

GAGGGGGGGT CCTGGAGGC 19

SEQ ID NO: 202
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

GGAGGGGGGG TCCTGGAGGC 20

SEQ ID NO: 203
SEQUENCE TYPE: nucleic acid
SEQUENCE LENGTH: 21 base pairs
STRANDEDNESS: single
TOPOLOGY: linear
MOLECULE SEQUENCE TYPE: Other nucleic acid
ANTI-SENSE: Yes

GGGAGGGGGG GTCCTGGAGG C 21

SEQ ID NO: 204
SEQUENCE TYPE: nucleic acid
SEQUENCE LENGTH: 22 base pairs
STRANDEDNESS: single
TOPOLOGY: linear
MOLECULE SEQUENCE TYPE: Other nucleic acid
ANTI-SENSE: Yes

CGGGAGGGGG GGTCCTGGAG GC 22

SEQ ID NO: 205
SEQUENCE TYPE: nucleic acid
SEQUENCE LENGTH: 23 base pairs

STRANDEDNESS:  single
TOPOLOGY:  linear
MOLECULE SEQUENCE TYPE:  Other nucleic acid
ANTI-SENSE:  Yes

CCGGGAGGGG GGGTCCTGGA GGC                                                    23

SEQ ID NO:  206
SEQUENCE TYPE:  nucleic acid
SEQUENCE LENGTH:  24 base pairs
STRANDEDNESS:  single
TOPOLOGY:  linear
MOLECULE SEQUENCE TYPE:  Other nucleic acid
ANTI-SENSE:  Yes

CCCGGGAGGG GGGGTCCTGG AGGC                                                   24

SEQ ID NO:  207
SEQUENCE TYPE:  nucleic acid
SEQUENCE LENGTH:  25 base pairs
STRANDEDNESS:  single
TOPOLOGY:  linear
MOLECULE SEQUENCE TYPE:  Other nucleic acid

ANTI-SENSE: Yes

TCCCGGGAGG GGGGGTCCTG GAGGC 25

SEQ ID NO: 208
SEQUENCE TYPE: nucleic acid
SEQUENCE LENGTH: 26 base pairs
STRANDEDNESS: single
TOPOLOGY: linear
MOLECULE SEQUENCE TYPE: Other nucleic acid
ANTI-SENSE: Yes

CTCCCGGGAG GGGGGGTCCT GGAGGC 26

SEQ ID NO: 209
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

TCTCCCGGGA GGGGGGGTCC TGGAGGC 27

SEQ ID NO: 210

SEQUENCE TYPE: nucleic acid

SEQUENCE LENGTH: 28 base pairs

STRANDEDNESS: single

TOPOLOGY: linear

MOLECULE SEQUENCE TYPE: Other nucleic acid

ANTI-SENSE: Yes

CTCTCCCGGG AGGGGGGTC CTGGAGGC 28

SEQ ID NO: 211

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

GCTCTCCCGG GAGGGGGGT CCTGGAGGC 29

SEQ ID NO: 212

SEQUENCE TYPE: nucleic acid

SEQUENCE LENGTH:  30 base pairs

STRANDEDNESS:  single

TOPOLOGY:  linear

MOLECULE SEQUENCE TYPE:  Other nucleic acid

ANTI-SENSE:  Yes

GGCTCTCCCG GGAGGGGGGG TCCTGGAGGC                                    30

SEQ ID NO:  213

SEQUENCE TYPE:  nucleic acid

SEQUENCE LENGTH:  16 base pairs

STRANDEDNESS:  single

TOPOLOGY:  linear

MOLECULE SEQUENCE TYPE:  Other nucleic acid

ANTI-SENSE:  Yes

GGGGGGGTCC TGGAGG                                                   16

SEQ ID NO:  214

SEQUENCE TYPE:  nucleic acid

SEQUENCE LENGTH:  17 base pairs

STRANDEDNESS:  single

TOPOLOGY:  linear

MOLECULE SEQUENCE TYPE:  Other nucleic acid
ANTI-SENSE:  Yes

AGGGGGGGTC CTGGAGG                                                    17

SEQ ID NO:  215
SEQUENCE TYPE:  nucleic acid
SEQUENCE LENGTH:  18 base pairs
STRANDEDNESS:  single
TOPOLOGY:  linear
MOLECULE T:  Other nucleic acid
ANTI-SENSE:  Yes

GAGGGGGGGT CCTGGAGG                                                   18

SEQ ID NO:  216
SEQUENCE TYPE:  nucleic acid
SEQUENCE LENGTH:  19 base pairs
STRANDEDNESS:  single
TOPOLOGY:  linear
MOLECULE SEQUENCE TYPE:  Other nucleic acid
ANTI-SENSE:  Yes

GGAGGGGGGG TCCTGGAGG 19

SEQ ID NO: 217
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

GGGAGGGGGG GTCCTGGAGG 20

SEQ ID NO: 218
SEQUENCE TYPE: nucleic acid
SEQUENCE LENGTH: 21 base pairs
STRANDEDNESS: single
TOPOLOGY: linear
MOLECULE SEQUENCE TYPE: Other nucleic acid
ANTI-SENSE: Yes

CGGGAGGGGG GGTCCTGGAG G 21

SEQ ID NO: 219
SEQUENCE TYPE: nucleic acid
SEQUENCE LENGTH: 22 base pairs
STRANDEDNESS: single
TOPOLOGY: linear
MOLECULE SEQUENCE TYPE: Other nucleic acid
ANTI-SENSE: Yes

CCGGGAGGGG GGGTCCTGGA GG 22

SEQ ID NO: 220
SEQUENCE TYPE: nucleic acid
SEQUENCE LENGTH: 23 base pairs
STRANDEDNESS: single
TOPOLOGY: linear
MOLECULE SEQUENCE TYPE: Other nucleic acid
ANTI-SENSE: Yes

CCCGGGAGGG GGGGTCCTGG AGG 23

SEQ ID NO: 221
SEQUENCE TYPE: nucleic acid
SEQUENCE LENGTH: 24 base pairs

STRANDEDNESS:  single

TOPOLOGY:  linear

MOLECULE SEQUENCE TYPE:  Other nucleic acid

ANTI-SENSE:  Yes

TCCCGGGAGG GGGGGTCCTG GAGG                                    24

SEQ ID NO:  222

SEQUENCE TYPE:  nucleic acid

SEQUENCE LENGTH:  25 base pairs

STRANDEDNESS:  single

TOPOLOGY:  linear

MOLECULE SEQUENCE TYPE:  Other nucleic acid

ANTI-SENSE:  Yes

CTCCCGGGAG GGGGGGTCCT GGAGG                                   25

SEQ ID NO:  223

SEQUENCE TYPE:  nucleic acid

SEQUENCE LENGTH:  26 base pairs

STRANDEDNESS:  single

TOPOLOGY:  linear

MOLECULE SEQUENCE TYPE:  Other nucleic acid

ANTI-SENSE: Yes

TCTCCCGGGA GGGGGGTCC TGGAGG 26

SEQ ID NO: 224
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

CTCTCCCGGG AGGGGGGTC CTGGAGG 27

SEQ ID NO: 225
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid
ANTI-SENSE: Yes

GCTCTCCCGG GAGGGGGGT CCTGGAGG 28

SEQ ID NO: 226

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

GGCTCTCCCG GGAGGGGGGG TCCTGGAGG 29

SEQ ID NO: 227

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

TGGCTCTCCC GGGAGGGGGG GTCCTGGAGG 30

SEQ ID NO: 228

SEQUENCE TYPE: nucleic acid

SEQUENCE LENGTH:  15 base pairs
STRANDEDNESS:  single
TOPOLOGY:  linear
MOLECULE SEQUENCE TYPE:  Other nucleic acid
ANTI-SENSE:  Yes

GGGGGGGTCC TGGAG                                                    15

SEQ ID NO:  229
SEQUENCE TYPE:  nucleic acid
SEQUENCE LENGTH:  16 base pairs
STRANDEDNESS:  single
TOPOLOGY:  linear
MOLECULE SEQUENCE TYPE:  Other nucleic acid
ANTI-SENSE:  Yes

AGGGGGGGTC CTGGAG                                                   16

SEQ ID NO:  230
SEQUENCE TYPE:  nucleic acid
SEQUENCE LENGTH:  17 base pairs
STRANDEDNESS:  single
TOPOLOGY:  linear

MOLECULE SEQUENCE TYPE:  Other nucleic acid
ANTI-SENSE:  Yes

GAGGGGGGT CCTGGAG                                                    17

SEQ ID NO:  231
SEQUENCE TYPE:  nucleic acid
SEQUENCE LENGTH:  18 base pairs
STRANDEDNESS:  single
TOPOLOGY:  linear
MOLECULE SEQUENCE TYPE:  Other nucleic acid
ANTI-SENSE:  Yes

GGAGGGGGGG TCCTGGAG                                                  18

SEQ ID NO:  232
SEQUENCE TYPE:  nucleic acid
SEQUENCE LENGTH:  19 base pairs
STRANDEDNESS:  single
TOPOLOGY:  linear
MOLECULE SEQUENCE TYPE:  Other nucleic acid
ANTI-SENSE:  Yes

GGGAGGGGGG GTCCTGGAG 19

SEQ ID NO: 233
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

CGGGAGGGGG GGTCCTGGAG 20

SEQ ID NO: 234
SEQUENCE TYPE: nucleic acid
SEQUENCE LENGTH: 21 base pairs
STRANDEDNESS: single
TOPOLOGY: linear
MOLECULE SEQUENCE TYPE: Other nucleic acid
ANTI-SENSE: Yes

CCGGGAGGGG GGGTCCTGGA G 21

SEQ ID NO: 235

SEQUENCE TYPE: nucleic acid

SEQUENCE LENGTH: 22 base pairs

STRANDEDNESS: single

TOPOLOGY: linear

MOLECULE SEQUENCE TYPE: Other nucleic acid

ANTI-SENSE: Yes

CCCGGGAGGG GGGGTCCTGG AG 22

SEQ ID NO: 236

SEQUENCE TYPE: nucleic acid

SEQUENCE LENGTH: 23 base pairs

STRANDEDNESS: single

TOPOLOGY: linear

MOLECULE SEQUENCE TYPE: Other nucleic acid

ANTI-SENSE: Yes

TCCCGGGAGG GGGGGTCCTG GAG 23

SEQ ID NO: 237

SEQUENCE TYPE: nucleic acid

SEQUENCE LENGTH: 24 base pairs

STRANDEDNESS:  single

TOPOLOGY:  linear

MOLECULE SEQUENCE TYPE:  Other nucleic acid

ANTI-SENSE:  Yes

CTCCCGGGAG GGGGGTCCT GGAG                                    24

SEQ ID NO:  238

SEQUENCE TYPE:  nucleic acid

SEQUENCE LENGTH:  25 base pairs

STRANDEDNESS:  single

TOPOLOGY:  linear

MOLECULE SEQUENCE TYPE:  Other nucleic acid

ANTI-SENSE:  Yes

TCTCCCGGGA GGGGGGTCC TGGAG                                   25

SEQ ID NO:  239

SEQUENCE TYPE:  nucleic acid

SEQUENCE LENGTH:  26 base pairs

STRANDEDNESS:  single

TOPOLOGY:  linear

MOLECULE SEQUENCE TYPE:  Other nucleic acid

ANTI-SENSE: Yes

CTCTCCCGGG AGGGGGGTC CTGGAG 26

SEQ ID NO: 240
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

GCTCTCCCGG GAGGGGGGT CCTGGAG 27

SEQ ID NO: 241
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid
ANTI-SENSE: Yes

GGCTCTCCCG GGAGGGGGG TCCTGGAG 28

SEQ ID NO: 242

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

TGGCTCTCCC GGGAGGGGGG GTCCTGGAG 29

SEQ ID NO: 243

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

ATGGCTCTCC CGGGAGGGGG GGTCCTGGAG 30

SEQ ID NO: 244

SEQUENCE TYPE: nucleic acid

SEQUENCE LENGTH: 15 base pairs
STRANDEDNESS: single
TOPOLOGY: linear
MOLECULE SEQUENCE TYPE: Other nucleic acid
ANTI-SENSE: Yes

AGGGGGGTC CTGGA 15

SEQ ID NO: 245
SEQUENCE TYPE: nucleic acid
SEQUENCE LENGTH: 16 base pairs
STRANDEDNESS: single
TOPOLOGY: linear
MOLECULE SEQUENCE TYPE: Other nucleic acid
ANTI-SENSE: Yes

GAGGGGGGGT CCTGGA 16

SEQ ID NO: 246
SEQUENCE TYPE: nucleic acid
SEQUENCE LENGTH: 17 base pairs
STRANDEDNESS: single
TOPOLOGY: linear

MOLECULE SEQUENCE TYPE:  Other nucleic acid
ANTI-SENSE:  Yes

GGAGGGGGGG TCCTGGA                                                    17

SEQ ID NO:  247
SEQUENCE TYPE:  nucleic acid
SEQUENCE LENGTH:  18 base pairs
STRANDEDNESS:  single
TOPOLOGY:  linear
MOLECULE SEQUENCE TYPE:  Other nucleic acid
ANTI-SENSE:  Yes

GGGAGGGGGG GTCCTGGA                                                   18

SEQ ID NO:  248
SEQUENCE TYPE:  nucleic acid
SEQUENCE LENGTH:  19 base pairs
STRANDEDNESS:  single
TOPOLOGY:  linear
MOLECULE SEQUENCE TYPE:  Other nucleic acid
ANTI-SENSE:  Yes

CGGGAGGGGG GGTCCTGGA 19

SEQ ID NO: 249

SEQUENCE TYPE: nucleic acid

SEQUENCE LENGTH: 20 base pairs

STRANDEDNESS: single

TOPOLOGY: linear

MOLECULE SEQUENCE TYPE: Other nucleic acid

ANTI-SENSE: Yes

CCGGGAGGGG GGGTCCTGGA 20

SEQ ID NO: 250

SEQUENCE LENGTH: 21 base pairs

SEQUENCE TYPE: nucleic acid

STRANDEDNESS: single

TOPOLOGY: linear

MOLECULE SEQUENCE TYPE: Other nucleic acid

ANTI-SENSE: Yes

CCCGGGAGGG GGGGTCCTGG A 21

SEQ ID NO: 251

SEQUENCE TYPE: nucleic acid

SEQUENCE LENGTH: 22 base pairs

STRANDEDNESS: single

TOPOLOGY: linear

MOLECULE SEQUENCE TYPE: Other nucleic acid

ANTI-SENSE: Yes

TCCCGGGAGG GGGGGTCCTG GA 22

SEQ ID NO: 252

SEQUENCE TYPE: nucleic acid

SEQUENCE LENGTH: 23 base pairs

STRANDEDNESS: single

TOPOLOGY: linear

MOLECULE SEQUENCE TYPE: Other nucleic acid

ANTI-SENSE: Yes

CTCCCGGGAG GGGGGGTCCT GGA 23

SEQ ID NO: 253

SEQUENCE TYPE: nucleic acid

SEQUENCE LENGTH: 24 base pairs

STRANDEDNESS: single
TOPOLOGY: linear
MOLECULE SEQUENCE TYPE: Other nucleic acid
ANTI-SENSE: Yes

TCTCCCGGGA GGGGGGTCC TGGA 24

SEQ ID NO: 254
SEQUENCE TYPE: nucleic acid
SEQUENCE LENGTH: 25 base pairs
STRANDEDNESS: single
TOPOLOGY: linear
MOLECULE SEQUENCE TYPE: Other nucleic acid
ANTI-SENSE: Yes

CTCTCCCGGG AGGGGGGTC CTGGA 25

SEQ ID NO: 255
SEQUENCE TYPE: nucleic acid
SEQUENCE LENGTH: 26 base pairs
STRANDEDNESS: single
TOPOLOGY: linear
MOLECULE SEQUENCE TYPE: Other nucleic acid

ANTI-SENSE: Yes

GCTCTCCCGG GAGGGGGGT CCTGGA 26

SEQ ID NO: 256
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

GGCTCTCCCG GGAGGGGGG TCCTGGA 27

SEQ ID NO: 257
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid
ANTI-SENSE: Yes

TGGCTCTCCC GGGAGGGGG GTCCTGGA 28

SEQ ID NO: 258

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

ATGGCTCTCC CGGGAGGGGG GGTCCTGGA 29

SEQ ID NO: 259

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

TATGGCTCTC CCGGGAGGGG GGGTCCTGGA 30

SEQ ID NO: 260

SEQUENCE TYPE: nucleic acid

SEQUENCE LENGTH: 15 base pairs
STRANDEDNESS: single
TOPOLOGY: linear
MOLECULE SEQUENCE TYPE: Other nucleic acid
ANTI-SENSE: Yes

GAGGGGGGT CCTGG 15

SEQ ID NO: 261
SEQUENCE TYPE: nucleic acid
SEQUENCE LENGTH: 16 base pairs
STRANDEDNESS: single
TOPOLOGY: linear
MOLECULE SEQUENCE TYPE: Other nucleic acid
ANTI-SENSE: Yes

GGAGGGGGGG TCCTGG 16

SEQ ID NO: 262
SEQUENCE TYPE: nucleic acid
SEQUENCE LENGTH: 17 base pairs
STRANDEDNESS: single
TOPOLOGY: linear

MOLECULE SEQUENCE TYPE:  Other nucleic acid
ANTI-SENSE:  Yes

GGGAGGGGGG GTCCTGG                                                    17

SEQ ID NO:  263
SEQUENCE TYPE:  nucleic acid
SEQUENCE LENGTH:  18 base pairs
STRANDEDNESS:  single
TOPOLOGY:  linear
MOLECULE SEQUENCE TYPE:  Other nucleic acid
ANTI-SENSE:  Yes

CGGGAGGGGG GGTCCTGG                                                   18

SEQ ID NO:  264
SEQUENCE TYPE:  nucleic acid
SEQUENCE LENGTH:  20 base pairs
STRANDEDNESS:  single
TOPOLOGY:  linear
MOLECULE SEQUENCE TYPE:  Other nucleic acid
ANTI-SENSE:  Yes

CCCGGGAGGG GGGGTCCTGG 20

SEQ ID NO: 265

SEQUENCE TYPE: nucleic acid

SEQUENCE LENGTH: 21 base pairs

STRANDEDNESS: single

TOPOLOGY: linear

MOLECULE SEQUENCE TYPE: Other nucleic acid

ANTI-SENSE: Yes

TCCCGGGAGG GGGGGTCCTG G 21

SEQ ID NO: 266

SEQUENCE TYPE: nucleic acid

SEQUENCE LENGTH: 22 base pairs

STRANDEDNESS: single

TOPOLOGY: linear

MOLECULE SEQUENCE TYPE: Other nucleic acid

ANTI-SENSE: Yes

CTCCCGGGAG GGGGGGTCCT GG 22

SEQ ID NO: 267

SEQUENCE TYPE: nucleic acid

SEQUENCE LENGTH: 23 base pairs

STRANDEDNESS: single

TOPOLOGY: linear

MOLECULE SEQUENCE TYPE: Other nucleic acid

ANTI-SENSE: Yes

TCTCCCGGGA GGGGGGTCC TGG 23

SEQ ID NO: 268

SEQUENCE TYPE: nucleic acid

SEQUENCE LENGTH: 24 base pairs

STRANDEDNESS: single

TOPOLOGY: linear

MOLECULE SEQUENCE TYPE: Other nucleic acid

ANTI-SENSE: Yes

CTCTCCCGGG AGGGGGGTC CTGG 24

SEQ ID NO: 269

SEQUENCE TYPE: nucleic acid

SEQUENCE LENGTH: 25 base pairs

STRANDEDNESS:  single

TOPOLOGY:  linear

MOLECULE SEQUENCE TYPE:  Other nucleic acid

ANTI-SENSE:  Yes

GCTCTCCCGG GAGGGGGGT CCTGG                                                    25

SEQ ID NO:  270

SEQUENCE TYPE:  nucleic acid

SEQUENCE LENGTH:  26 base pairs

STRANDEDNESS:  single

TOPOLOGY:  linear

MOLECULE SEQUENCE TYPE:  Other nucleic acid

ANTI-SENSE:  Yes

GGCTCTCCCG GGAGGGGGG TCCTGG                                                   26

SEQ ID NO:  271

SEQUENCE TYPE:  nucleic acid

SEQUENCE LENGTH:  27 base pairs

STRANDEDNESS:  single

TOPOLOGY:  linear

MOLECULE SEQUENCE TYPE:  Other nucleic acid

ANTI-SENSE:  Yes

TGGCTCTCCC GGGAGGGGGG GTCCTGG                                    27

SEQ ID NO:  272
SEQUENCE TYPE:  nucleic acid
SEQUENCE LENGTH:  28 base pairs
STRANDEDNESS:  single
TOPOLOGY:  linear
MOLECULE SEQUENCE TYPE:  Other nucleic acid
ANTI-SENSE:  Yes
ATGGCTCTCC CGGGAGGGGG GGTCCTGG                                   28

SEQ ID NO:  273
SEQUENCE TYPE:  nucleic acid
SEQUENCE LENGTH:  29 base pairs
STRANDEDNESS:  single
TOPOLOGY:  linear
MOLECULE SEQUENCE TYPE:  Other nucleic acid
ANTI-SENSE:  Yes

TATGGCTCTC CCGGGAGGGG GGGTCCTGG                                  29

SEQ ID NO: 274

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

CTATGGCTCT CCCGGGAGGG GGGGTCCTGG 30

SEQ ID NO: 275

SEQUENCE TYPE: nucleic acid

SEQUENCE LENGTH: 15 base pairs

STRANDEDNESS: single

TOPOLOGY: linear

MOLECULE SEQUENCE TYPE: Other nucleic acid

ANTI-SENSE: Yes

GGAGGGGGGG TCCTG 15

SEQ ID NO: 276

SEQUENCE TYPE: nucleic acid

SEQUENCE LENGTH: 16 base pairs

STRANDEDNESS:  single
TOPOLOGY:  linear
MOLECULE SEQUENCE TYPE:  Other nucleic acid
ANTI-SENSE:  Yes

GGGAGGGGGG GTCCTG                                                    16

SEQ ID NO:  277
SEQUENCE TYPE:  nucleic acid
SEQUENCE LENGTH:  17 base pairs
STRANDEDNESS:  single
TOPOLOGY:  linear
MOLECULE SEQUENCE TYPE:  Other nucleic acid
ANTI-SENSE:  Yes

CGGGAGGGGG GGTCCTG                                                   17

SEQ ID NO:  278
SEQUENCE TYPE:  nucleic acid
SEQUENCE LENGTH:  18 base pairs
STRANDEDNESS:  single
TOPOLOGY:  linear
MOLECULE SEQUENCE TYPE:  Other nucleic acid

ANTI-SENSE: Yes

CCGGGAGGGG GGGTCCTG 18

SEQ ID NO: 279
SEQUENCE TYPE: nucleic acid
SEQUENCE LENGTH: 19 base pairs
STRANDEDNESS: single
TOPOLOGY: linear
MOLECULE SEQUENCE TYPE: Other nucleic acid
ANTI-SENSE: Yes

CCCGGGAGGG GGGGTCCTG 19

SEQ ID NO: 280
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

TCCCGGGAGG GGGGGTCCTG 20

SEQ ID NO: 281
SEQUENCE TYPE: nucleic acid
SEQUENCE LENGTH: 21 base pairs
STRANDEDNESS: single
TOPOLOGY: linear
MOLECULE SEQUENCE TYPE: Other nucleic acid
ANTI-SENSE: Yes

CTCCCGGGAG GGGGGTCCT G 21

SEQ ID NO: 282
SEQUENCE TYPE: nucleic acid
SEQUENCE LENGTH: 22 base pairs
STRANDEDNESS: single
TOPOLOGY: linear
MOLECULE SEQUENCE TYPE: Other nucleic acid
ANTI-SENSE: Yes

TCTCCCGGGA GGGGGGTCC TG 22

SEQ ID NO: 283
SEQUENCE TYPE: nucleic acid

SEQUENCE LENGTH:  23 base pairs

STRANDEDNESS:  single

TOPOLOGY:  linear

MOLECULE SEQUENCE TYPE:  Other nucleic acid

ANTI-SENSE:  Yes

CTCTCCCGGG AGGGGGGTC CTG                                    23

SEQ ID NO:  284

SEQUENCE TYPE:  nucleic acid

SEQUENCE LENGTH:  24 base pairs

STRANDEDNESS:  single

TOPOLOGY:  linear

MOLECULE SEQUENCE TYPE:  Other nucleic acid

ANTI-SENSE:  Yes

GCTCTCCCGG GAGGGGGGT CCTG                                   24

SEQ ID NO:  285

SEQUENCE TYPE:  nucleic acid

SEQUENCE LENGTH:  25 base pairs

STRANDEDNESS:  single

TOPOLOGY:  linear

MOLECULE SEQUENCE TYPE:  Other nucleic acid
ANTI-SENSE:  Yes

GGCTCTCCCG GGAGGGGGGG TCCTG                                    25

SEQ ID NO:  286
SEQUENCE TYPE:  nucleic acid
SEQUENCE LENGTH:  26 base pairs
STRANDEDNESS:  single
TOPOLOGY:  linear
MOLECULE SEQUENCE TYPE:  Other nucleic acid
ANTI-SENSE:  Yes

TGGCTCTCCC GGGAGGGGGG GTCCTG                                   26

SEQ ID NO:  287
SEQUENCE TYPE:  nucleic acid
SEQUENCE LENGTH:  27 base pairs
STRANDEDNESS:  single
TOPOLOGY:  linear
MOLECULE SEQUENCE TYPE:  Other nucleic acid
ANTI-SENSE:  Yes

ATGGCTCTCC CGGGAGGGGG GGTCCTG 27

SEQ ID NO: 288
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid
ANTI-SENSE: Yes

TATGGCTCTC CCGGGAGGGG GGGTCCTG 28

SEQ ID NO: 289
SEQUENCE TYPE: nucleic acid
SEQUENCE LENGTH: 29 base pairs
STRANDEDNESS: single
TOPOLOGY: linear
MOLECULE SEQUENCE TYPE: Other nucleic acid
ANTI-SENSE: Yes

CTATGGCTCT CCCGGGAGGG GGGGTCCTG 29

SEQ ID NO: 290
SEQUENCE TYPE: nucleic acid
SEQUENCE LENGTH: 30 base pairs
STRANDEDNESS: single
TOPOLOGY: linear
MOLECULE SEQUENCE TYPE: Other nucleic acid
ANTI-SENSE: Yes

ACTATGGCTC TCCCGGGAGG GGGGGTCCTG 30

SEQ ID NO: 291
SEQUENCE TYPE: nucleic acid
SEQUENCE LENGTH: 15 base pairs
STRANDEDNESS: single
TOPOLOGY: linear
MOLECULE SEQUENCE TYPE: Other nucleic acid
ANTI-SENSE: Yes

GGGAGGGGGG GTCCT 15

SEQ ID NO: 292
SEQUENCE TYPE: nucleic acid
SEQUENCE LENGTH: 16 base pairs

STRANDEDNESS: single

TOPOLOGY: linear

MOLECULE SEQUENCE TYPE: Other nucleic acid

ANTI-SENSE: Yes

CGGGAGGGGG GGTCCT 16

SEQ ID NO: 293

SEQUENCE TYPE: nucleic acid

SEQUENCE LENGTH: 17 base pairs

STRANDEDNESS: single

TOPOLOGY: linear

MOLECULE SEQUENCE TYPE: Other nucleic acid

ANTI-SENSE: Yes

CCGGGAGGGG GGGTCCT 17

SEQ ID NO: 294

SEQUENCE TYPE: nucleic acid

SEQUENCE LENGTH: 18 base pairs

STRANDEDNESS: single

TOPOLOGY: linear

MOLECULE SEQUENCE TYPE: Other nucleic acid

ANTI-SENSE: Yes

CCCGGGAGGG GGGGTCCT 18

SEQ ID NO: 295
SEQUENCE TYPE: nucleic acid
SEQUENCE LENGTH: 19 base pairs
STRANDEDNESS: single
TOPOLOGY: linear
MOLECULE SEQUENCE TYPE: Other nucleic acid
ANTI-SENSE: Yes

TCCCGGGAGG GGGGGTCCT 19

SEQ ID NO: 296
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes
CTCCCGGGAG GGGGGGTCCT 20

SEQ ID NO: 297

SEQUENCE TYPE: nucleic acid

SEQUENCE LENGTH: 21 base pairs

STRANDEDNESS: single

TOPOLOGY: linear

MOLECULE SEQUENCE TYPE: Other nucleic acid

ANTI-SENSE: Yes

TCTCCCGGGA GGGGGGGTCC T 21

SEQ ID NO: 298

SEQUENCE TYPE: nucleic acid

SEQUENCE LENGTH: 22 base pairs

STRANDEDNESS: single

TOPOLOGY: linear

MOLECULE SEQUENCE TYPE: Other nucleic acid

ANTI-SENSE: Yes

CTCTCCCGGG AGGGGGGGTC CT 22

SEQ ID NO: 299

SEQUENCE TYPE: nucleic acid

SEQUENCE LENGTH: 23 base pairs

STRANDEDNESS:  single

TOPOLOGY:  linear

MOLECULE SEQUENCE TYPE:  Other nucleic acid

ANTI-SENSE:  Yes

GCTCTCCCGG GAGGGGGGT CCT                                    23

SEQ ID NO:  300

SEQUENCE TYPE:  nucleic acid

SEQUENCE LENGTH:  24 base pairs

STRANDEDNESS:  single

TOPOLOGY:  linear

MOLECULE SEQUENCE TYPE:  Other nucleic acid

ANTI-SENSE:  Yes

GGCTCTCCCG GGAGGGGGG TCCT                                   24

SEQ ID NO:  301

SEQUENCE TYPE:  nucleic acid

SEQUENCE LENGTH:  25 base pairs

STRANDEDNESS:  single

TOPOLOGY:  linear

MOLECULE SEQUENCE TYPE:  Other nucleic acid

ANTI-SENSE: Yes

TGGCTCTCCC GGGAGGGGGG GTCCT 25

SEQ ID NO: 302
SEQUENCE TYPE: nucleic acid
SEQUENCE LENGTH: 26 base pairs
STRANDEDNESS: single
TOPOLOGY: linear
MOLECULE SEQUENCE TYPE: Other nucleic acid
ANTI-SENSE: Yes

ATGGCTCTCC CGGGAGGGGG GGTCCT 26

SEQ ID NO: 303
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

TATGGCTCTC CCGGGAGGGG GGGTCCT 27

SEQ ID NO: 304

SEQUENCE TYPE: nucleic acid

SEQUENCE LENGTH: 28 base pairs

STRANDEDNESS: single

TOPOLOGY: linear

MOLECULE SEQUENCE TYPE: Other nucleic acid

ANTI-SENSE: Yes

CTATGGCTCT CCCGGGAGGG GGGGTCCT 28

SEQ ID NO: 305

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

ACTATGGCTC TCCCGGGAGG GGGGGTCCT 29

SEQ ID NO: 306

SEQUENCE TYPE: nucleic acid

SEQUENCE LENGTH:  30 base pairs
STRANDEDNESS:  single
TOPOLOGY:  linear
MOLECULE SEQUENCE TYPE:  Other nucleic acid
ANTI-SENSE:  Yes

CACTATGGCT CTCCCGGGAG GGGGGGTCCT                                    30

SEQ ID NO:  307
SEQUENCE TYPE:  nucleic acid
SEQUENCE LENGTH:  15 base pairs
STRANDEDNESS:  single
TOPOLOGY:  linear
MOLECULE SEQUENCE TYPE:  Other nucleic acid
ANTI-SENSE:  Yes

CGGGAGGGGG GGTCC                                                    15

SEQ ID NO:  308
SEQUENCE TYPE:  nucleic acid
SEQUENCE LENGTH:  16 base pairs
STRANDEDNESS:  single
TOPOLOGY:  linear

MOLECULE SEQUENCE TYPE:  Other nucleic acid
ANTI-SENSE:  Yes

CCGGGAGGGG GGGTCC                                                    16

SEQ ID NO:  309
SEQUENCE TYPE:  nucleic acid
SEQUENCE LENGTH:  17 base pairs
STRANDEDNESS:  single
TOPOLOGY:  linear
MOLECULE SEQUENCE TYPE:  Other nucleic acid
ANTI-SENSE:  Yes

CCCGGGAGGG GGGGTCC                                                   17

SEQ ID NO:  310
SEQUENCE TYPE:  nucleic acid
SEQUENCE LENGTH:  18 base pairs
STRANDEDNESS:  single
TOPOLOGY:  linear
MOLECULE SEQUENCE TYPE:  Other nucleic acid
ANTI-SENSE:  Yes

TCCCGGGAGG GGGGGTCC 18

SEQ ID NO: 311
SEQUENCE TYPE: nucleic acid
SEQUENCE LENGTH: 19 base pairs
STRANDEDNESS: single
TOPOLOGY: linear
MOLECULE SEQUENCE TYPE: Other nucleic acid
ANTI-SENSE: Yes

CTCCCGGGAG GGGGGGTCC 19

SEQ ID NO: 312
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes
TCTCCCGGGA GGGGGGGTCC 20

SEQ ID NO: 313

SEQUENCE TYPE: nucleic acid
SEQUENCE LENGTH: 21 base pairs
STRANDEDNESS: single
TOPOLOGY: linear
MOLECULE SEQUENCE TYPE: Other nucleic acid
ANTI-SENSE: Yes

CTCTCCCGGG AGGGGGGTC C 21

SEQ ID NO: 314
SEQUENCE TYPE: nucleic acid
SEQUENCE LENGTH: 22 base pairs
STRANDEDNESS: single
TOPOLOGY: linear
MOLECULE SEQUENCE TYPE: Other nucleic acid
ANTI-SENSE: Yes

GCTCTCCCGG GAGGGGGGT CC 22

SEQ ID NO: 315
SEQUENCE TYPE: nucleic acid
SEQUENCE LENGTH: 23 base pairs
STRANDEDNESS: single

TOPOLOGY: linear
MOLECULE SEQUENCE TYPE: Other nucleic acid
ANTI-SENSE: Yes

GGCTCTCCCG GGAGGGGGGG TCC 23

SEQ ID NO: 316
SEQUENCE TYPE: nucleic acid
SEQUENCE LENGTH: 24 base pairs
STRANDEDNESS: single
TOPOLOGY: linear
MOLECULE SEQUENCE TYPE: Other nucleic acid
ANTI-SENSE: Yes

TGGCTCTCCC GGGAGGGGGG GTCC 24

SEQ ID NO: 317
SEQUENCE TYPE: nucleic acid
SEQUENCE LENGTH: 25 base pairs
STRANDEDNESS: single
TOPOLOGY: linear
MOLECULE SEQUENCE TYPE: Other nucleic acid
ANTI-SENSE: Yes

ATGGCTCTCC CGGGAGGGGG GGTCC 25

SEQ ID NO: 318
SEQUENCE TYPE: nucleic acid
SEQUENCE LENGTH: 26 base pairs
STRANDEDNESS: single
TOPOLOGY: linear
MOLECULE SEQUENCE TYPE: Other nucleic acid
ANTI-SENSE: Yes

TATGGCTCTC CCGGGAGGGG GGGTCC 26

SEQ ID NO: 319
SEQUENCE TYPE: nucleic acid
SEQUENCE LENGTH: 27 base pairs
STRANDEDNESS: single
TOPOLOGY: linear
MOLECULE SEQUENCE TYPE: Other nucleic acid
ANTI-SENSE: Yes

CTATGGCTCT CCCGGGAGGG GGGGTCC 27

SEQ ID NO: 320
SEQUENCE TYPE: nucleic acid
SEQUENCE LENGTH: 28 base pairs
STRANDEDNESS: single
TOPOLOGY: linear
MOLECULE SEQUENCE TYPE: Other nucleic acid
ANTI-SENSE: Yes
ACTATGGCTC TCCCGGGAGG GGGGGTCC 28

SEQ ID NO: 321
SEQUENCE TYPE: nucleic acid
SEQUENCE LENGTH: 29 base pairs
STRANDEDNESS: single
TOPOLOGY: linear
MOLECULE SEQUENCE TYPE: Other nucleic acid
ANTI-SENSE: Yes

CACTATGGCT CTCCCGGGAG GGGGGGTCC 29

SEQ ID NO: 322
SEQUENCE TYPE: nucleic acid
SEQUENCE LENGTH: 30 base pairs
STRANDEDNESS: single

TOPOLOGY: linear
MOLECULE SEQUENCE TYPE: Other nucleic acid
ANTI-SENSE: Yes

CCACTATGGC TCTCCCGGGA GGGGGGGTCC 30

SEQ ID NO: 323
SEQUENCE TYPE: nucleic acid
SEQUENCE LENGTH: 15 base pairs
STRANDEDNESS: single
TOPOLOGY: linear
MOLECULE SEQUENCE TYPE: Other nucleic acid
ANTI-SENSE: Yes

CCGGGAGGGG GGGTC 15

SEQ ID NO: 324
SEQUENCE TYPE: nucleic acid
SEQUENCE LENGTH: 16 base pairs
STRANDEDNESS: single
TOPOLOGY: linear
MOLECULE SEQUENCE TYPE: Other nucleic acid
ANTI-SENSE: Yes

CCCGGGAGGG GGGGTC 16

SEQ ID NO: 325

SEQUENCE TYPE: nucleic acid

SEQUENCE LENGTH: 17 base pairs

STRANDEDNESS: single

TOPOLOGY: linear

MOLECULE SEQUENCE TYPE: Other nucleic acid

ANTI-SENSE: Yes

TCCCGGGAGG GGGGGTC 17

SEQ ID NO: 326

SEQUENCE TYPE: nucleic acid

SEQUENCE LENGTH: 18 base pairs

STRANDEDNESS: single

TOPOLOGY: linear

MOLECULE SEQUENCE TYPE: Other nucleic acid

ANTI-SENSE: Yes

CTCCCGGGAG GGGGGGTC 18

SEQ ID NO: 327
SEQUENCE TYPE: nucleic acid
SEQUENCE LENGTH: 19 base pairs
STRANDEDNESS: single
TOPOLOGY: linear
MOLECULE SEQUENCE TYPE: Other nucleic acid
ANTI-SENSE: Yes

TCTCCCGGGA GGGGGGTC 19

SEQ ID NO: 328
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes
CTCTCCCGGG AGGGGGGTC 20

SEQ ID NO: 329
SEQUENCE TYPE: nucleic acid
SEQUENCE LENGTH: 21 base pairs
STRANDEDNESS: single

TOPOLOGY: linear

MOLECULE SEQUENCE TYPE: Other nucleic acid

ANTI-SENSE: Yes

GCTCTCCCGG GAGGGGGGGT C 21

SEQ ID NO: 330

SEQUENCE TYPE: nucleic acid

SEQUENCE LENGTH: 22 base pairs

STRANDEDNESS: single

TOPOLOGY: linear

MOLECULE SEQUENCE TYPE: Other nucleic acid

ANTI-SENSE: Yes

GGCTCTCCCG GGAGGGGGGG TC 22

SEQ ID NO: 331

SEQUENCE TYPE: nucleic acid

SEQUENCE LENGTH: 23 base pairs

STRANDEDNESS: single

TOPOLOGY: linear

MOLECULE SEQUENCE TYPE: Other nucleic acid

ANTI-SENSE: Yes

TGGCTCTCCC GGGAGGGGGG GTC 23

SEQ ID NO: 332
SEQUENCE TYPE: nucleic acid
SEQUENCE LENGTH: 24 base pairs
STRANDEDNESS: single
TOPOLOGY: linear
MOLECULE SEQUENCE TYPE: Other nucleic acid
ANTI-SENSE: Yes

ATGGCTCTCC CGGGAGGGGG GGTC 24

SEQ ID NO: 333
SEQUENCE TYPE: nucleic acid
SEQUENCE LENGTH: 25 base pairs
STRANDEDNESS: single
TOPOLOGY: linear
MOLECULE SEQUENCE TYPE: Other nucleic acid
ANTI-SENSE: Yes

TATGGCTCTC CCGGGAGGGG GGGTC 25

SEQ ID NO: 334

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

CTATGGCTCT CCCGGGAGGG GGGGTC 26

SEQ ID NO: 335

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

ACTATGGCTC TCCCGGGAGG GGGGGTC 27

SEQ ID NO: 336

SEQUENCE TYPE: nucleic acid

SEQUENCE LENGTH: 28 base pairs

STRANDEDNESS: single

TOPOLOGY: linear

MOLECULE SEQUENCE TYPE: Other nucleic acid

ANTI-SENSE: Yes

CACTATGGCT CTCCCGGGAG GGGGGGTC 28

SEQ ID NO: 337

SEQUENCE TYPE: nucleic acid

SEQUENCE LENGTH: 29 base pairs

STRANDEDNESS: single

TOPOLOGY: linear

MOLECULE SEQUENCE TYPE: Other nucleic acid

ANTI-SENSE: Yes

CCACTATGGC TCTCCCGGGA GGGGGGGTC 29

SEQ ID NO: 338

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

ACCACTATGG CTCTCCCGGG AGGGGGGGTC 30

SEQ ID NO: 339
SEQUENCE TYPE: nucleic acid
SEQUENCE LENGTH: 15 base pairs
STRANDEDNESS: single
TOPOLOGY: linear
MOLECULE SEQUENCE TYPE: Other nucleic acid
ANTI-SENSE: Yes

CCCGGGAGGG GGGGT 15

SEQ ID NO: 340
SEQUENCE TYPE: nucleic acid
SEQUENCE LENGTH: 16 base pairs
STRANDEDNESS: single
TOPOLOGY: linear
MOLECULE SEQUENCE TYPE: Other nucleic acid
ANTI-SENSE: Yes

TCCCGGGAGG GGGGGT 16

SEQ ID NO: 341

SEQUENCE TYPE: nucleic acid

SEQUENCE LENGTH: 17 base pairs

STRANDEDNESS: single

TOPOLOGY: linear

MOLECULE SEQUENCE TYPE: Other nucleic acid

ANTI-SENSE: Yes

CTCCCGGGAG GGGGGGT 17

SEQ ID NO: 342

SEQUENCE TYPE: nucleic acid

SEQUENCE LENGTH: 18 base pairs

STRANDEDNESS: single

TOPOLOGY: linear

MOLECULE SEQUENCE TYPE: Other nucleic acid

ANTI-SENSE: Yes

TCTCCCGGGA GGGGGGGT 18

SEQ ID NO: 343

SEQUENCE TYPE: nucleic acid

SEQUENCE LENGTH:  19 base pairs
STRANDEDNESS:  single
TOPOLOGY:  linear
MOLECULE SEQUENCE TYPE:  Other nucleic acid
ANTI-SENSE:  Yes

CTCTCCCGGG AGGGGGGGT                                                    19

SEQ ID NO:  344
SEQUENCE TYPE:  nucleic acid
SEQUENCE LENGTH:  20 base pairs
STRANDEDNESS:  single
TOPOLOGY:  linear
MOLECULE SEQUENCE TYPE:  Other nucleic acid
ANTI-SENSE:  Yes

GCTCTCCCGG GAGGGGGGGT                                                   20

SEQ ID NO:  345
SEQUENCE TYPE:  nucleic acid
SEQUENCE LENGTH:  21 base pairs
STRANDEDNESS:  single
TOPOLOGY:  linear

MOLECULE SEQUENCE TYPE:  Other nucleic acid
ANTI-SENSE:  Yes

GGCTCTCCCG GGAGGGGGGG T                                    21

SEQ ID NO:  346
SEQUENCE TYPE:  nucleic acid
SEQUENCE LENGTH:  22 base pairs
STRANDEDNESS:  single
TOPOLOGY:  linear
MOLECULE SEQUENCE TYPE:  Other nucleic acid
ANTI-SENSE:  Yes

TGGCTCTCCC GGGAGGGGGG GT                                   22

SEQ ID NO:  347
SEQUENCE TYPE:  nucleic acid
SEQUENCE LENGTH:  23 base pairs
STRANDEDNESS:  single
TOPOLOGY:  linear
MOLECULE SEQUENCE TYPE:  Other nucleic acid
ANTI-SENSE:  Yes

ATGGCTCTCC CGGGAGGGGG GGT 23

SEQ ID NO: 348

SEQUENCE TYPE: nucleic acid

SEQUENCE LENGTH: 24 base pairs

STRANDEDNESS: single

TOPOLOGY: linear

MOLECULE SEQUENCE TYPE: Other nucleic acid

ANTI-SENSE: Yes

TATGGCTCTC CCGGGAGGGG GGGT 24

SEQ ID NO: 349

SEQUENCE TYPE: nucleic acid

SEQUENCE LENGTH: 25 base pairs

STRANDEDNESS: single

TOPOLOGY: linear

MOLECULE SEQUENCE TYPE: Other nucleic acid

ANTI-SENSE: Yes

CTATGGCTCT CCCGGGAGGG GGGGT 25

SEQ ID NO: 350

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

ACTATGGCTC TCCCGGGAGG GGGGGT 26

SEQ ID NO: 351

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

CACTATGGCT CTCCCGGGAG GGGGGGT 27

SEQ ID NO: 352

SEQUENCE TYPE: nucleic acid

SEQUENCE LENGTH: 28 base pairs

STRANDEDNESS:  single

TOPOLOGY:  linear

MOLECULE SEQUENCE TYPE:  Other nucleic acid

ANTI-SENSE:  Yes

CCACTATGGC TCTCCCGGGA GGGGGGGT                                    28

SEQ ID NO:  353

SEQUENCE TYPE:  nucleic acid

SEQUENCE LENGTH:  29 base pairs

STRANDEDNESS:  single

TOPOLOGY:  linear

MOLECULE SEQUENCE TYPE:  Other nucleic acid

ANTI-SENSE:  Yes

ACCACTATGG CTCTCCCGGG AGGGGGGGT                                   29

SEQ ID NO:  354

SEQUENCE TYPE:  nucleic acid

SEQUENCE LENGTH:  30 base pairs

STRANDEDNESS:  single

TOPOLOGY:  linear

MOLECULE SEQUENCE TYPE:  Other nucleic acid

ANTI-SENSE: Yes

GACCACTATG GCTCTCCCGG GAGGGGGGGT 30

SEQ ID NO: 355

SEQUENCE TYPE: nucleic acid

SEQUENCE LENGTH: 15 base pairs

STRANDEDNESS: single

TOPOLOGY: linear

MOLECULE SEQUENCE TYPE: Other nucleic acid

ANTI-SENSE: Yes

TCCCGGGAGG GGGGG 15

SEQ ID NO: 356

SEQUENCE TYPE: nucleic acid

SEQUENCE LENGTH: 16 base pairs

STRANDEDNESS: single

TOPOLOGY: linear

MOLECULE SEQUENCE TYPE: Other nucleic acid

ANTI-SENSE: Yes

CTCCCGGGAG GGGGGG 16

SEQ ID NO: 357

SEQUENCE TYPE: nucleic acid

SEQUENCE LENGTH: 17 base pairs

STRANDEDNESS: single

TOPOLOGY: linear

MOLECULE SEQUENCE TYPE: Other nucleic acid

ANTI-SENSE: Yes

TCTCCCGGGA GGGGGGG 17

SEQ ID NO: 358

SEQUENCE TYPE: nucleic acid

SEQUENCE LENGTH: 18 base pairs

STRANDEDNESS: single

TOPOLOGY: linear

MOLECULE SEQUENCE TYPE: Other nucleic acid

ANTI-SENSE: Yes

CTCTCCCGGG AGGGGGGG 18

SEQ ID NO: 359

SEQUENCE TYPE: nucleic acid

SEQUENCE LENGTH:  19 base pairs

STRANDEDNESS:  single

TOPOLOGY:  linear

MOLECULE SEQUENCE TYPE:  Other nucleic acid

ANTI-SENSE:  Yes

GCTCTCCCGG GAGGGGGGG                                                    19

SEQ ID NO:  360

SEQUENCE TYPE:  nucleic acid

SEQUENCE LENGTH:  20 base pairs

STRANDEDNESS:  single

TOPOLOGY:  linear

MOLECULE SEQUENCE TYPE:  Other nucleic acid

ANTI-SENSE:  Yes

GGCTCTCCCG GGAGGGGGGG                                                   20

SEQ ID NO:  361

SEQUENCE TYPE:  nucleic acid

SEQUENCE LENGTH:  21 base pairs

STRANDEDNESS:  single

TOPOLOGY:  linear

MOLECULE SEQUENCE TYPE:  Other nucleic acid
ANTI-SENSE:  Yes

TGGCTCTCCC GGGAGGGGGG G                                    21

SEQ ID NO:  362
SEQUENCE TYPE:  nucleic acid
SEQUENCE LENGTH:  22 base pairs
STRANDEDNESS:  single
TOPOLOGY:  linear
MOLECULE SEQUENCE TYPE:  Other nucleic acid
ANTI-SENSE:  Yes

ATGGCTCTCC CGGGAGGGGG GG                                   22

SEQ ID NO:  363
SEQUENCE TYPE:  nucleic acid
SEQUENCE LENGTH:  23 base pairs
STRANDEDNESS:  single
TOPOLOGY:  linear
MOLECULE SEQUENCE TYPE:  Other nucleic acid
ANTI-SENSE:  Yes

TATGGCTCTC CCGGGAGGGG GGG 23

SEQ ID NO: 364
SEQUENCE TYPE: nucleic acid
SEQUENCE LENGTH: 24 base pairs
STRANDEDNESS: single
TOPOLOGY: linear
MOLECULE SEQUENCE TYPE: Other nucleic acid
ANTI-SENSE: Yes

CTATGGCTCT CCCGGGAGGG GGGG 24

SEQ ID NO: 365
SEQUENCE TYPE: nucleic acid
SEQUENCE LENGTH: 25 base pairs
STRANDEDNESS: single
TOPOLOGY: linear
MOLECULE SEQUENCE TYPE: Other nucleic acid
ANTI-SENSE: Yes

ACTATGGCTC TCCCGGGAGG GGGGG 25

SEQ ID NO: 366

SEQUENCE TYPE: nucleic acid

SEQUENCE LENGTH: 26 base pairs

STRANDEDNESS: single

TOPOLOGY: linear

MOLECULE SEQUENCE TYPE: Other nucleic acid

ANTI-SENSE: Yes

CACTATGGCT CTCCCGGGAG GGGGGG 26

SEQ ID NO: 367

SEQUENCE TYPE: nucleic acid

SEQUENCE LENGTH: 27 base pairs

STRANDEDNESS: single

TOPOLOGY: linear

MOLECULE SEQUENCE TYPE: Other nucleic acid

ANTI-SENSE: Yes

CCACTATGGC TCTCCCGGGA GGGGGGG 27

SEQ ID NO: 368

SEQUENCE LENGTH: 28 base pairs

SEQUENCE TYPE: nucleic acid

STRANDEDNESS:  single

TOPOLOGY:  linear

MOLECULE SEQUENCE TYPE:  Other nucleic acid

ANTI-SENSE:  Yes

ACCACTATGG CTCTCCCGGG AGGGGGGG                                    28

SEQ ID NO:  369

SEQUENCE TYPE:  nucleic acid

SEQUENCE LENGTH:  29 base pairs

STRANDEDNESS:  single

TOPOLOGY:  linear

MOLECULE SEQUENCE TYPE:  Other nucleic acid

ANTI-SENSE:  Yes

GACCACTATG GCTCTCCCGG GAGGGGGGG                                   29

SEQ ID NO:  370

SEQUENCE TYPE:  nucleic acid

SEQUENCE LENGTH:  20 base pairs

STRANDEDNESS:  single

TOPOLOGY:  linear

MOLECULE SEQUENCE TYPE:  Other nucleic acid

ANTI-SENSE: Yes

GGGGGTCCTG GAGGCTGCAC 20

SEQ ID NO: 371

SEQUENCE TYPE: nucleic acid

SEQUENCE LENGTH: 15 base pairs

STRANDEDNESS: single

TOPOLOGY: linear

MOLECULE SEQUENCE TYPE: Other nucleic acid

ANTI-SENSE: Yes

CCCGGACGCC ATGCG 15

SEQ ID NO: 372

SEQUENCE TYPE: nucleic acid

SEQUENCE LENGTH: 17 base pairs

STRANDEDNESS: single

TOPOLOGY: linear

MOLECULE SEQUENCE TYPE: Other nucleic acid

ANTI-SENSE: Yes

CCCGGACGCC ATGCGCT 17

SEQ ID NO: 373

SEQUENCE TYPE: nucleic acid

SEQUENCE LENGTH: 20 base pairs

STRANDEDNESS: single

TOPOLOGY: linear

MOLECULE SEQUENCE TYPE: Other nucleic acid

ANTI-SENSE: Yes

CCCGGACGCC ATGCGCTAGA 20

SEQ ID NO: 374

SEQUENCE TYPE: nucleic acid

SEQUENCE LENGTH: 25 base pairs

STRANDEDNESS: single

TOPOLOGY: linear

MOLECULE SEQUENCE TYPE: Other nucleic acid

ANTI-SENSE: Yes

CCCGGACGCC ATGCGCTAGA GCCCT 25

SEQ ID NO: 375

SEQUENCE TYPE: nucleic acid

SEQUENCE LENGTH:  30 base pairs
STRANDEDNESS:  single
TOPOLOGY:  linear
MOLECULE SEQUENCE TYPE:  Other nucleic acid
ANTI-SENSE:  Yes

CCCGGACGCC ATGCGCTAGA GCCCTGGCAG                                    30

SEQ ID NO:  376
SEQUENCE TYPE:  nucleic acid
SEQUENCE LENGTH:  16 base pairs
STRANDEDNESS:  single
TOPOLOGY:  linear
MOLECULE SEQUENCE TYPE:  Other nucleic acid
ANTI-SENSE:  Yes

GAACCCGGAC GCCATG                                                   16

SEQ ID NO:  377
SEQUENCE TYPE:  nucleic acid
SEQUENCE LENGTH:  20 base pairs
STRANDEDNESS:  single
TOPOLOGY:  linear

MOLECULE SEQUENCE TYPE:  Other nucleic acid
ANTI-SENSE:  Yes

GAACCCGGAC GCCATGCGCT                                                    20

SEQ ID NO:  378
SEQUENCE TYPE:  nucleic acid
SEQUENCE LENGTH:  25 base pairs
STRANDEDNESS:  single
TOPOLOGY:  linear
MOLECULE SEQUENCE TYPE:  Other nucleic acid
ANTI-SENSE:  Yes

GAACCCGGAC GCCATGCGCT AGAGC                                              25

SEQ ID NO:  379
SEQUENCE TYPE:  nucleic acid
SEQUENCE LENGTH:  30 base pairs
STRANDEDNESS:  single
TOPOLOGY:  linear
MOLECULE SEQUENCE TYPE:  Other nucleic acid
ANTI-SENSE:  Yes

GAACCCGGAC GCCATGCGCT AGAGCCCTGG 30

SEQ ID NO: 380

SEQUENCE TYPE: nucleic acid

SEQUENCE LENGTH: 15 base pairs

STRANDEDNESS: single

TOPOLOGY: linear

MOLECULE SEQUENCE TYPE: Other nucleic acid

ANTI-SENSE: Yes

CAGAACCCGG ACGCC 15

SEQ ID NO: 381

SEQUENCE TYPE: nucleic acid

SEQUENCE LENGTH: 18 base pairs

STRANDEDNESS: single

TOPOLOGY: linear

MOLECULE SEQUENCE TYPE: Other nucleic acid

ANTI-SENSE: Yes

CAGAACCCGG ACGCCATG 18

SEQ ID NO: 382
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

CAGAACCCGG ACGCCATGCG 20

SEQ ID NO: 383
SEQUENCE TYPE: nucleic acid
SEQUENCE LENGTH: 25 base pairs
STRANDEDNESS: single
TOPOLOGY: linear
MOLECULE SEQUENCE TYPE: Other nucleic acid
ANTI-SENSE: Yes

CAGAACCCGG ACGCCATGCG CTAGA 25

SEQ ID NO: 384
SEQUENCE TYPE: nucleic acid
SEQUENCE LENGTH: 30 base pairs

STRANDEDNESS:  single

TOPOLOGY:  linear

MOLECULE SEQUENCE TYPE:  Other nucleic acid

ANTI-SENSE:  Yes

CAGAACCCGG ACGCCATGCG CTAGAGCCCT                                    30

SEQ ID NO:  385

SEQUENCE TYPE:  nucleic acid

SEQUENCE LENGTH:  20 base pairs

STRANDEDNESS:  single

TOPOLOGY:  linear

MOLECULE SEQUENCE TYPE:  Other nucleic acid

ANTI-SENSE:  Yes

CGTCCTCCAG AACCCGGACG                                               20

SEQ ID NO:  386

SEQUENCE TYPE:  nucleic acid

SEQUENCE LENGTH:  25 base pairs

STRANDEDNESS:  single

TOPOLOGY:  linear

MOLECULE SEQUENCE TYPE:  Other nucleic acid

ANTI-SENSE:  Yes

CGTCCTCCAG AACCCGGACG CCATG                                    25

SEQ ID NO:  387
SEQUENCE TYPE:  nucleic acid
SEQUENCE LENGTH:  30 base pairs
STRANDEDNESS:  single
TOPOLOGY:  linear
MOLECULE SEQUENCE TYPE:  Other nucleic acid
ANTI-SENSE:  Yes

CGTCCTCCAG AACCCGGACG CCATGCGCTA                               30

SEQ ID NO:  388
SEQUENCE TYPE:  nucleic acid
SEQUENCE LENGTH:  25 base pairs
STRANDEDNESS:  single
TOPOLOGY:  linear
MOLECULE SEQUENCE TYPE:  Other nucleic acid
ANTI-SENSE:  Yes

CACGCCGTCC TCCAGAACCC GGACG                                    25

SEQ ID NO: 389

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

CACGCCGTCC TCCAGAACCC GGACGCCATG 30

SEQ ID NO: 390

SEQUENCE TYPE: nucleic acid

SEQUENCE LENGTH: 30 base pairs

STRANDEDNESS: single

TOPOLOGY: linear

MOLECULE SEQUENCE TYPE: Other nucleic acid

ANTI-SENSE: Yes

TAGTTCACGC CGTCCTCCAG AACCCGGACG 30

SEQ ID NO: 391

SEQUENCE TYPE: nucleic acid

SEQUENCE LENGTH:  20 base pairs
STRANDEDNESS:  single
TOPOLOGY:  linear
MOLECULE SEQUENCE TYPE:  Other nucleic acid
ANTI-SENSE:  Yes

GCGGGGGCAC GCCCAAATCT                                                    20

SEQ ID NO:  392
SEQUENCE TYPE:  nucleic acid
SEQUENCE LENGTH:  19 base pairs
STRANDEDNESS:  single
TOPOLOGY:  linear
MOLECULE SEQUENCE TYPE:  Other nucleic acid
ANTI-SENSE:  Yes
AAGGGTGGGG GGGAAACGG                                                     19

SEQ ID NO:  393
SEQUENCE TYPE:  nucleic acid
SEQUENCE LENGTH:  20 base pairs
STRANDEDNESS:  single
TOPOLOGY:  linear
MOLECULE SEQUENCE TYPE:  Other nucleic acid

ANTI-SENSE: Yes

TGTGTTCTCC ATGTTCGGTG 20

SEQ ID NO: 394
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

GTCAATGTCC ATGCCCCAAA 20

SEQ ID NO: 395
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

GCGAGACTGC TAGCCGAGTG 20

SEQ ID NO: 396
SEQUENCE TYPE: nucleic acid
SEQUENCE LENGTH: 20 base pairs
STRANDEDNESS: single
TOPOLOGY: linear
MOLECULE SEQUENCE TYPE: Other nucleic acid
ANTI-SENSE: Yes

CCTCCAGAGC ATCTGGCACG 20

SEQ ID NO: 397
SEQUENCE TYPE: nucleic acid
SEQUENCE LENGTH: 16 base pairs
STRANDEDNESS: single
TOPOLOGY: linear
MOLECULE SEQUENCE TYPE: Other nucleic acid
ANTI-SENSE: Yes

GCGAGACTGC TAGCCG 16

SEQ ID NO: 398
SEQUENCE TYPE: nucleic acid

SEQUENCE LENGTH:  20 base pairs

STRANDEDNESS:  single

TOPOLOGY:  linear

MOLECULE SEQUENCE TYPE:  Other nucleic acid

ANTI-SENSE:  Yes

CATCACAACC CAGCGCTTTC                                                    20

SEQ ID NO:  399

SEQUENCE TYPE:  nucleic acid

SEQUENCE LENGTH:  17 base pairs

TOPOLOGY:  linear

MOLECULE SEQUENCE TYPE:  Other nucleic acid, synthetic DNA

GTAAAACGAC GGCCAGT                                                       17

SEQ ID NO:  400

SEQUENCE TYPE:  nucleic acid

SEQUENCE LENGTH:  17 base pairs

TOPOLOGY:  linear

MOLECULE SEQUENCE TYPE:  Other nucleic acid, synthetic DNA

CAGGAAACAG CTATGAC                                                       17

SEQ ID NO: 401
SEQUENCE TYPE: nucleic acid
SEQUENCE LENGTH: 16 base pairs
TOPOLOGY: linear
MOLECULE SEQUENCE TYPE: Other nucleic acid, synthetic DNA

CAGATCTGCA AGCTTG 16

SEQ ID NO: 402
SEQUENCE TYPE: nucleic acid
SEQUENCE LENGTH: 17 base pairs
TOPOLOGY: linear
MOLECULE SEQUENCE TYPE: Other nucleic acid, synthetic DNA

CAGGAAACAG CTATGAC 17

SEQ ID NO: 403
SEQUENCE TYPE: nucleic acid
SEQUENCE LENGTH: 22 base pairs
TOPOLOGY: linear
MOLECULE SEQUENCE TYPE: Other nucleic acid, synthetic DNA

GAATAGTTTT TCAATTTTTA CG 22

SEQ ID NO: 404
SEQUENCE TYPE: nucleic acid
SEQUENCE LENGTH: 22 base pairs
TOPOLOGY: linear
MOLECULE SEQUENCE TYPE: Other nucleic acid, synthetic DNA

CGTAAAAATT GAAAAACTAT TC 22

SEQ ID NO: 405
SEQUENCE TYPE: nucleic acid
SEQUENCE LENGTH: 17 base pairs
TOPOLOGY: linear
MOLECULE SEQUENCE TYPE: Other nucleic acid, synthetic DNA

GTAAAACGAC GGCCAGT 17

SEQ ID NO: 406
SEQUENCE TYPE: nucleic acid
SEQUENCE LENGTH: 40 base pairs
TOPOLOGY: linear

MOLECULE SEQUENCE TYPE: Other nucleic acid, synthetic DNA

GATCCAAAAA TTGAAAAACT AGTCTAATTT ATTGCACGGA 40
    GTTTTT AACTTTTTGA TCAGATTAAA TAACGTGCCT CTAG

SEQ ID NO: 407
SEQUENCE TYPE: nucleic acid
SEQUENCE LENGTH: 25 base pairs
TOPOLOGY: linear
MOLECULE SEQUENCE TYPE: Other nucleic acid, synthetic DNA

CGAAGCTTGCC AGCCCCCTGA TGGG 25

SEQ ID NO: 408
SEQUENCE TYPE: nucleic acid
SEQUENCE LENGTH: 28 base pairs
TOPOLOGY: linear
MOLECULE SEQUENCE TYPE: Other nucleic acid, synthetic DNA

CCGGATCCCG GAAGCTGGGA TGGTCAAC 28

SEQ ID NO: 409

SEQUENCE TYPE: nucleic acid

SEQUENCE LENGTH: 2360 base pairs

STRANDEDNESS: double

TOPOLOGY: linear

ORIGINAL SOURCE

ORGANISM: Vaccinia virus

IMMEDIATE SOURCE

CLONE: pHASE

TCGACGATTG TTCATGATGG CAAGATTTAT ATATCTGGAG GTTACAACAA TAGTAGTGTA 60

GTTAATGTAA TATCGAATCT AGTCCTTAGC TATAATCCGA TATATGATGA ATGGACCAAA 120

TTATCATCAT TAAACATTCC TAGAATTAAT CCCGCTCTAT GGTCAGCGCA TAATAAATTA 180

TATGTAGGAG GAGGAATATC TGATGATGTT CGAACTAATA CATCTGAAAC ATACGATAAA 240

GAAAAAGATT GTTGGACATT GGATAATGGT CACGTGTTAC CACGCAATTA TATAATGTAT 300

AAATGCGAAC CGATTAAACA TAAATATCCA TTGGAAAAAA CACAGTACAC GAATGATTTT 360

CTAAAGTATT TGGAAAGTTT TATAGGTAGT TGATAGAACA AAATACATAA TTTTGTAAAA 420

ATAAATCACT TTTTATACTA ATATGACACG ATTACCAATA CTTTTGTTAC TAATATCATT 480

AGTATACGCT ACACCTTTTC CTCAGACATC TAAAAAAATA GGTGATGATG CAACTCTATC 540

ATGTAATCGA AATAATACAA ATGACTACGT TGTTATGAGT GCTTGGTATA AGGAGCCCAA 600

TTCCATTATT CTTTTAGCTG CTAAAAGCGA CGTCTTGTAT TTTGATAATT ATACCAAGGA 660

TAAAATATCT TACGACTCTC CATACGATGA TCTAGTTACA ACTATCACAA TTAAATCATT 720

GACTGCTAGA GATGCCGGTA CTTATGTATG TGCATTCTTT ATGACATCAA CTACAAATGA 780

CACTGATAAA GTAGATTATG AAGAATACTC CACAGAGTTG ATTGTAAATA CAGATAGTGA 840

ATCGACTATA GACATAATAC TATCTGGATC TACACATTCA CCAGAAACTA GCTAGTTCTG 900

AGAAACCAGA GGATATAGAT AATTTTAATT GCTCGTCGGT ATTCGAAATC GGGTCGACAT 960

CTATATACTA TATAGTAATA CCAATACTCA AGACTACGAA ACTGATACAA TCTCTTATCA 1020
TGTGGGTAAT GTTCTCGATG TCGATAGCCA TATGCCCGGT AGTTGCGATA TACATAAACT 1080
GATCACTAAT TCCAAACCCA CCCACTTTTT ATAGTAAGTT TTTCACCCAT AAATAATAAA 1140
TACAATAATT AATTTCTCGT AAAAATTGAA AAACTATTCT AATTTATTGC ACGGTAAGGA 1200
AGTAGAATCA TAAAGAACAG TGACTCTAGA GGATCCAAAA ATTGAAAAAC TAGTCTAATT 1260
TATTGCACGG AGATCCAAAA ATTGAAAAAC TAGTCTAATT TATTGCACGG AGATCCAAAA 1320
ATTGAAAAAC TAGTCTAATT TATTGCACGG AGATCCAAAA ATTGAAAAAC TAGTCTAATT 1380
TATTGCACGG AGATCCAAAA ATTGAAAAAC TAGTCTAATT TATTGCACGG AGATCCAAAA 1440
ATTGAAAAAC TAGTCTAATT TATTGCACGG AGATCTGCAA GCTTGGGGTA CCGAGCTCGA 1500
ATTCGACTCC GGAACCAATT ACTGATAATG TAGAAGATCA TACAGACACC GTCACATACA 1560
CTAGCTAGTG ATAGCATTAA TACAGTAAGT GCATCATCTG GAGAATCCAC AACAGACGAG 1620
ACTCCGGAAC CAATTACTGA TAAAGAAGAA GATCATACAG TCACAGACAC TGTCTCATAC 1680
ACTACAGTAA GTACATCATC TGGAATTGTC ACTACTAAAT CAACCACCGA TGATGCGGAT 1740
CTTTATGATA CGTACAATGA TAATGATACA GTACCACCAA CTACTGTAGG CGGTAGTACA 1800
ACCTCTATTA GCAATTATAA AACCAAGGAC TTTGTAGAAA TATTTGGTAT TACCGCATTA 1860
ATTATATTGT CGGCCGTGGC AATATTCTGT ATTACATATT ATATATATAA TAAACGTTCA 1920
CGTAAATACA AAACAGAGAA CAAAGTCTAG ATTTTTGACT TACATAAATG TCTGGGATAG 1980
TAAAATCTAT CATATTGAGC GGACCATCTG GTTCAGGAAA GACAGCCATA GCCAAAAGAC 2040
TATGGGAATA TATTTGGATT TGTGGTGTCC CATACCACTA GATTTCCTCG TCCTATGGAA 2100
CGAGAAGGTG TCGATTACCA TTACGTTAAC AGAGAGGCCA TCTGGAAGGG AATAGCCGCC 2160
GGAAACTTTC TAGAACATAC TGAGTTTTTA GGAAATATTT ACGGAACTTC TAAAACTGCT 2220
GTGAATACAG CGGCTATTAA TAATCGTATT TGTGTGATGG ATCTAAACAT CGATGGCGTT 2280
AGAAGTCTTA AAAATACGTA CCTAATGCCT TACTCGGTGT ATATAAGACC TACCTCTCTT 2340
AAAATGGTTG AGACCAAGCT 2360

SEQ ID NO: 410

SEQUENCE TYPE: nucleic acid

SEQUENCE LENGTH: 4987 base pairs

STRANDEDNESS: double

TOPOLOGY: linear

ORIGINAL SOURCE

ORGANISM: Vaccinia virus, Hepatitis C virus, Firefly luciferase gene

IMMEDIATE SOURCE

CLONE: pHA5CL

TCGACGATTG TTCATGATGG CAAGATTTAT ATATCTGGAG GTTACAACAA TAGTAGTGTA 60

GTTAATGTAA TATCGAATCT AGTCCTTAGC TATAATCCGA TATATGATGA ATGGACCAAA 120

TTATCATCAT TAAACATTCC TAGAATTAAT CCCGCTCTAT GGTCAGCGCA TAATAAATTA 180

TATGTAGGAG GAGGAATATC TGATGATGTT CGAACTAATA CATCTGAAAC ATACGATAAA 240

GAAAAAGATT GTTGGACATT GGATAATGGT CACGTGTTAC CACGCAATTA TATAATGTAT 300

AAATGCGAAC CGATTAAACA TAAATATCCA TTGGAAAAAA CACAGTACAC GAATGATTTT 360

CTAAAGTATT TGGAAAGTTT TATAGGTAGT TGATAGAACA AAATACATAA TTTTGTAAAA 420

ATAAATCACT TTTTATACTA ATATGACACG ATTACCAATA CTTTTGTTAC TAATATCATT 480

AGTATACGCT ACACCTTTTC CTCAGACATC TAAAAAAATA GGTGATGATG CAACTCTATC 540

ATGTAATCGA AATAATACAA ATGACTACGT TGTTATGAGT GCTTGGTATA AGGAGCCCAA 600

TTCCATTATT CTTTTAGCTG CTAAAAGCGA CGTCTTGTAT TTTGATAATT ATACCAAGGA 660

TAAAATATCT TACGACTCTC CATACGATGA TCTAGTTACA ACTATCACAA TTAAATCATT 720

GACTGCTAGA GATGCCGGTA CTTATGTATG TGCATTCTTT ATGACATCAA CTACAAATGA 780

CACTGATAAA GTAGATTATG AAGAATACTC CACAGAGTTG ATTGTAAATA CAGATAGTGA 840

ATCGACTATA GACATAATAC TATCTGGATC TACACATTCA CCAGAAACTA GCTAGTTCTG 900

AGAAACCAGA GGATATAGAT AATTTTAATT GCTCGTCGGT ATTCGAAATC GGGTCGACAT 960

CTATATACTA TATAGTAATA CCAATACTCA AGACTACGAA ACTGATACAA TCTCTTATCA 1020

TGTGGGTAAT GTTCTCGATG TCGATAGCCA TATGCCCGGT AGTTGCGATA TACATAAACT 1080

GATCACTAAT TCCAAACCCA CCCACTTTTT ATAGTAAGTT TTTCACCCAT AAATAATAAA 1140

TACAATAATT AATTTCTCGT AAAAATTGAA AAACTATTCT AATTTATTGC ACGGTAAGGA 1200

AGTAGAATCA TAAAGAACAG TGACTCTAGA GGATCCAAAA ATTGAAAAAC TAGTCTAATT 1260

TATTGCACGG AGATCCAAAA ATTGAAAAAC TAGTCTAATT TATTGCACGG AGATCCAAAA 1320

ATTGAAAAAC TAGTCTAATT TATTGCACGG AGATCCAAAA ATTGAAAAAC TAGTCTAATT 1380

TATTGCACGG AGATCCAAAA ATTGAAAAAC TAGTCTAATT TATTGCACGG AGATCCAAAA 1440

ATTGAAAAAC TAGTCTAATT TATTGCACGG AGATCTGCAA GCTTGCCAGC CCCCTGATGG 1500

GGGCGACACT CCACCATAGA TCACTCCCCT GTGAGGAACT ACTGTCTTCA CGCAGAAAGC 1560

GTCTAGCCAT GGCGTTAGTA TGAGTGTCGT GCAGCCTCCA GGACCCCCCC TCCCGGGAGA 1620

GCCATAGTGG TCTGCGGAAC CGGTGAGTAC ACCGGAATTG CCAGGACGAC CGGGTCCTTT 1680

CTTGGATCAA CCCGCTCAAT GCCTGGAGAT TTGGGCGTGC CCCCGCGAGA CTGCTAGCCG 1740

AGTAGTGTTG GGTCGCGAAA GGCCTTGTGG TACTGCCTGA TAGGGTGCTT GCGAGTGCCC 1800

CGGGAGGTCT CGTAGACCGT GCATC ATG AGC ACA AAT CCA AAA CCC CAA AGA 1852

Met Ser Thr Asn Pro Lys Pro Gln Arg

1               5

AAA ATC AAA CGT AAC ACC AAC CGC CGC CCA CAG GAC GTT AAG TTC CCG 1900

Lys Ile Lys Arg Asn Thr Asn Arg Arg Pro Gln Asp Val Lys Phe Pro

10                  15                  20                  25

GGC GGT GGT CAG ATC GTT GGT GGA GTT TAC CTG TTG CCG CGC AGG GGC 1948

Gly Gly Gly Gln Ile Val Gly Gly Val Tyr Leu Leu Pro Arg Arg Gly

                30                  35                  40

CCC AGG TTG GGT GTG CGC GCG ACT AGG AAG ACT TCC GAG CGG CCG CAA 1996
Pro Arg Leu Gly Val Arg Ala Thr Arg Lys Thr Ser Glu Arg Pro Gln
            45                  50                  55
CCT CGT GGA AGG CGA CAA CCT ATC CCC AAG GCT CGC CAA CCC GAG GGT 2044
Pro Arg Gly Arg Arg Gln Pro Ile Pro Lys Ala Arg Gln Pro Glu Gly
        60                  65                  70
AGG GCC TGG GCT CAG CCC GGG TAC CCT TGG CCC CTC TAT GGC AAT GAG 2092
Arg Ala Trp Ala Gln Pro Gly Tyr Pro Trp Pro Leu Tyr Gly Asn Glu
    75                  80                  85
GGC TTG GGG TGG GCA GGA TGG CTC CTG TCA CCC CGC GGC TCC CGG CCT 2140
Gly Leu Gly Trp Ala Gly Trp Leu Leu Ser Pro Arg Gly Ser Arg Pro
90                  95                  100                 105
AGT TGG GGC CCC ACG GAC CCC CGG CGT AGG TCG CGT AAT TTG GGT AAG 2188
Ser Trp Gly Pro Thr Asp Pro Arg Arg Arg Ser Arg Asn Leu Gly Lys
                110                 115                 120
GTC ATC GAT ACC CTC ACA TGC GGC TTC GCC GAC CTC ATG GGG TAC ATT 2236
Val Ile Asp Thr Leu Thr Cys Gly Phe Ala Asp Leu Met Gly Tyr Ile
            125                 130                 135
CCG CTC GTC GGC GCC CCC CTA GGG GGC GCT GCC AGG GCT CTA GCG CAT 2284
Pro Leu Val Gly Ala Pro Leu Gly Gly Ala Ala Arg Ala Leu Ala His
        140                 145                 150
GGC GTC CGG GTT CTG GAG GAC GGC GTG AAC TAT GCA ACA GGG AAT CTG 2332
Gly Val Arg Val Leu Glu Asp Gly Val Asn Tyr Ala Thr Gly Asn Leu
    155                 160                 165

CCT GGT TGC TCC TTT TCT ATC TTC CTT TTG GCT TTG CTG TCC TGT TTG 2380
Pro Gly Cys Ser Phe Ser Ile Phe Leu Leu Ala Leu Leu Ser Cys Leu
170                 175                 180                 185
ACC ATC CCA GCT TCC GGG ATC CAA ATG GAA GAC GCC AAA AAC ATA AAG 2428
Thr Ile Pro Ala Ser Gly Ile Gln Met Glu Asp Ala Lys Asn Ile Lys
                190                 195                 200
AAA GGC CCG GCG CCA TTC TAT CCT CTA GAG GAT GGA ACC GCT GGA GAG 2476
Lys Gly Pro Ala Pro Phe Tyr Pro Leu Glu Asp Gly Thr Ala Gly Glu
            205                 210                 215
CAA CTG CAT AAG GCT ATG AAG AGA TAC GCC CTG GTT CCT GGA ACA ATT 2524
Gln Leu His Lys Ala Met Lys Arg Tyr Ala Leu Val Pro Gly Thr Ile
        220                 225                 230
GCT TTT ACA GAT GCA CAT ATC GAG GTG AAC ATC ACG TAC GCG GAA TAC 2572
Ala Phe Thr Asp Ala His Ile Glu Val Asn Ile Thr Tyr Ala Glu Tyr
    235                 240                 245
TTC GAA ATG TCC GTT CGG TTG GCA GAA GCT ATG AAA CGA TAT GGG CTG 2620
Phe Glu Met Ser Val Arg Leu Ala Glu Ala Met Lys Arg Tyr Gly Leu
250                 255                 260                 265
AAT ACA AAT CAC AGA ATC GTC GTA TGC AGT GAA AAC TCT CTT CAA TTC 2668
Asn Thr Asn His Arg Ile Val Val Cys Ser Glu Asn Ser Leu Gln Phe
                270                 275                 280
TTT ATG CCG GTG TTG GGC GCG TTA TTT ATC GGA GTT GCA GTT GCG CCC 2716
Phe Met Pro Val Leu Gly Ala Leu Phe Ile Gly Val Ala Val Ala Pro
            285                 290                 295

GCG AAC GAC ATT TAT AAT GAA CGT GAA TTG CTC AAC AGT ATG AAC ATT 2764
Ala Asn Asp Ile Tyr Asn Glu Arg Glu Leu Leu Asn Ser Met Asn Ile
        300                 305                 310
TCG CAG CCT ACC GTA GTG TTT GTT TCC AAA AAG GGG TTG CAA AAA ATT 2812
Ser Gln Pro Thr Val Val Phe Val Ser Lys Lys Gly Leu Gln Lys Ile
    315                 320                 325
TTG AAC GTG CAA AAA AAA TTA CCA ATA ATC CAG AAA ATT ATT ATC ATG 2860
Leu Asn Val Gln Lys Lys Leu Pro Ile Ile Gln Lys Ile Ile Ile Met
330                 335                 340                 345
GAT TCT AAA ACG GAT TAC CAG GGA TTT CAG TCG ATG TAC ACG TTC GTC 2908
Asp Ser Lys Thr Asp Tyr Gln Gly Phe Gln Ser Met Tyr Thr Phe Val
                350                 355                 360
ACA TCT CAT CTA CCT CCC GGT TTT AAT GAA TAC GAT TTT GTA CCA GAG 2956
Thr Ser His Leu Pro Pro Gly Phe Asn Glu Tyr Asp Phe Val Pro Glu
            365                 370                 375
TCC TTT GAT CGT GAC AAA ACA ATT GCA CTG ATA ATG AAT TCC TCT GGA 3004
Ser Phe Asp Arg Asp Lys Thr Ile Ala Leu Ile Met Asn Ser Ser Gly
        380                 385                 390
TCT ACT GGG TTA CCT AAG GGT GTG GCC CTT CCG CAT AGA ACT GCC TGC 3052
Ser Thr Gly Leu Pro Lys Gly Val Ala Leu Pro His Arg Thr Ala Cys
    395                 400                 405
GTC AGA TTC TCG CAT GCC AGA GAT CCT ATT TTT GGC AAT CAA ATC ATT 3100
Val Arg Phe Ser His Ala Arg Asp Pro Ile Phe Gly Asn Gln Ile Ile
410                 415                 420                 425

CCG GAT ACT GCG ATT TTA AGT GTT GTT CCA TTC CAT CAC GGT TTT GGA 3148

Pro Asp Thr Ala Ile Leu Ser Val Val Pro Phe His His Gly Phe Gly

                430                 435                 440

ATG TTT ACT ACA CTC GGA TAT TTG ATA TGT GGA TTT CGA GTC GTC TTA 3196

Met Phe Thr Thr Leu Gly Tyr Leu Ile Cys Gly Phe Arg Val Val Leu

            445                 450                 455

ATG TAT AGA TTT GAA GAA GAG CTG TTT TTA CGA TCC CTT CAG GAT TAC 3244

Met Tyr Arg Phe Glu Glu Glu Leu Phe Leu Arg Ser Leu Gln Asp Tyr

        460                 465                 470

AAA ATT CAA AGT GCG TTG CTA GTA CCA ACC CTA TTT TCA TTC TTC GCC 3292

Lys Ile Gln Ser Ala Leu Leu Val Pro Thr Leu Phe Ser Phe Phe Ala

    475                 480                 485

AAA AGC ACT CTG ATT GAC AAA TAC GAT TTA TCT AAT TTA CAC GAA ATT 3340

Lys Ser Thr Leu Ile Asp Lys Tyr Asp Leu Ser Asn Leu His Glu Ile

490                 495                 500                 505

GCT TCT GGG GGC GCA CCT CTT TCG AAA GAA GTC GGG GAA GCG GTT GCA 3388

Ala Ser Gly Gly Ala Pro Leu Ser Lys Glu Val Gly Glu Ala Val Ala

                510                 515                 520

AAA CGC TTC CAT CTT CCA GGG ATA CGA CAA GGA TAT GGG CTC ACT GAG 3436

Lys Arg Phe His Leu Pro Gly Ile Arg Gln Gly Tyr Gly Leu Thr Glu

            525                 530                 535

ACT ACA TCA GCT ATT CTG ATT ACA CCC GAG GGG GAT GAT AAA CCG GGC 3484

Thr Thr Ser Ala Ile Leu Ile Thr Pro Glu Gly Asp Asp Lys Pro Gly

        540                 545                 550

GCG GTC GGT AAA GTT GTT CCA TTT TTT GAA GCG AAG GTT GTG GAT CTG 3532

Ala Val Gly Lys Val Val Pro Phe Phe Glu Ala Lys Val Val Asp Leu

    555                 560                 565

GAT ACC GGG AAA ACG CTG GGC GTT AAT CAG AGA GGC GAA TTA TGT GTC 3580

Asp Thr Gly Lys Thr Leu Gly Val Asn Gln Arg Gly Glu Leu Cys Val

570                 575                 580                 585

AGA GGA CCT ATG ATT ATG TCC GGT TAT GTA AAC AAT CCG GAA GCG ACC 3628

Arg Gly Pro Met Ile Met Ser Gly Tyr Val Asn Asn Pro Glu Ala Thr

                590                 595                 600

AAC GCC TTG ATT GAC AAG GAT GGA TGG CTA CAT TCT GGA GAC ATA GCT 3676

Asn Ala Leu Ile Asp Lys Asp Gly Trp Leu His Ser Gly Asp Ile Ala

            605                 610                 615

TAC TGG GAC GAA GAC GAA CAC TTC TTC ATA GTT GAC CTC TTG AAG TCT 3724

Tyr Trp Asp Glu Asp Glu His Phe Phe Ile Val Asp Leu Leu Lys Ser

        620                 625                 630

TTA ATT AAA TAC AAA GGA TAT CAG GTG GCC CCC GCT GAA TTG GAA TCG 3772

Leu Ile Lys Tyr Lys Gly Tyr Gln Val Ala Pro Ala Glu Leu Glu Ser

    635                 640                 645

ATA TTG TTA CAA CAC CCC AAC ATC TTC GAC GCG GGC GTG GCA GGT CTT 3820

Ile Leu Leu Gln His Pro Asn Ile Phe Asp Ala Gly Val Ala Gly Leu

650                 655                 660                 665

CCC GAC GAT GAC GCC GGT GAA CTT CCC GCC GCC GTT GTT GTT TTG GAG 3868

Pro Asp Asp Asp Ala Gly Glu Leu Pro Ala Ala Val Val Val Leu Glu

                670                 675                 680

CAC GGA AAG ACG ATG ACG GAA AAA GAG ATC GTG GAT TAC GTG GCC AGT 3916
His Gly Lys Thr Met Thr Glu Lys Glu Ile Val Asp Tyr Val Ala Ser
            685                 690                 695
CAA GTA ACA ACC GCG AAA AAG TTG CGC GGA GGA GTT GTG TTT GTG GAC 3964
Gln Val Thr Thr Ala Lys Lys Leu Arg Gly Gly Val Val Phe Val Asp
        700                 705                 710
GAA GTA CCG AAA GGT CTT ACC GGA AAA CTC GAC GCA AGA AAA ATC AGA 4012
Glu Val Pro Lys Gly Leu Thr Gly Lys Leu Asp Ala Arg Lys Ile Arg
    715                 720                 725
GAG ATC CTC ATA AAG GCC AAG AAG GGC GGA AAG TCC AAA TTG TAA AAT 4060
Glu Ile Leu Ile Lys Ala Lys Lys Gly Gly Lys Ser Lys Leu Stop
730                 735                 740
GTAACTGTAT TCAGCGATGA CGAAATTCTT AGCTATTGTA ATCCTCCGAG GCCTCGAGGT 4120
CGACGAATTC CGACTCCGGA ACCAATTACT GATAATGTAG AAGATCATAC AGACACCGTC 4180
ACATACACTA GCTAGTGATA GCATTAATAC AGTAAGTGCA TCATCTGGAG AATCCACAAC 4240
AGACGAGACT CCGGAACCAA TTACTGATAA AGAAGAAGAT CATACAGTCA CAGACACTGT 4300
CTCATACACT ACAGTAAGTA CATCATCTGG AATTGTCACT ACTAAATCAA CCACCGATGA 4360
TGCGGATCTT TATGATACGT ACAATGATAA TGATACAGTA CCACCAACTA CTGTAGGCGG 4420
TAGTACAACC TCTATTAGCA ATTATAAAAC CAAGGACTTT GTAGAAATAT TTGGTATTAC 4480
CGCATTAATT ATATTGTCGG CCGTGGCAAT ATTCTGTATT ACATATTATA TATATAATAA 4540
ACGTTCACGT AAATACAAAA CAGAGAACAA AGTCTAGATT TTTGACTTAC ATAAATGTCT 4600
GGGATAGTAA AATCTATCAT ATTGAGCGGA CCATCTGGTT CAGGAAAGAC AGCCATAGCC 4660
AAAAGACTAT GGGAATATAT TTGGATTTGT GGTGTCCCAT ACCACTAGAT TTCCTCGTCC 4720
TATGGAACGA GAAGGTGTCG ATTACCATTA CGTTAACAGA GAGGCCATCT GGAAGGGAAT 4780
AGCCGCCGGA AACTTTCTAG AACATACTGA GTTTTTAGGA AATATTTACG GAACTTCTAA 4840

AACTGCTGTG AATACAGCGG CTATTAATAA TCGTATTTGT GTGATGGATC TAAACATCGA 4900
TGGCGTTAGA AGTCTTAAAA ATACGTACCT AATGCCTTAC TCGGTGTATA TAAGACCTAC 4960
CTCTCTTAAA ATGGTTGAGA CCAAGCT 4987

## Claims

1. An antisense compound having a sequence complementary to a base sequence which consists of 10-34 bases and is extracted from:
(i) 93 bases from thymine at position 107 to adenine at position 199,
(ii) 152 bases from adenine at position 250 to cytosine at position 401, or
(iii) 52 bases from cytosine at position 808 to adenine at position 859,
of the base sequence shown in SEQ ID NO: 1.

2. An antisense compound according to claim 1 characterized in that said base sequence is extracted from:
(iv) 54 bases from guanine at position 127 to guanine at position 180,
(v) 34 bases from adenine at position 284 to thymine at position 317, or
(vi) 34 bases from cytosine at position 343 to cytosine at position 376.

3. An antisense compound according to claim 1 characterized in that said base sequence contains 8 bases from cytosine at position 830 to guanine at position 837.

4. An antisense compound according to claim 1 characterized in that said base sequence is selected from:
(1) a base sequence which is included within 54 bases from guanine at position 127 to guanine at position 180, and which contains 16 bases from cytosine at position 131 to adenine at position 146, 7 bases from cytosine at position 147 to cytosine at position 153, 6 bases from cytosine at position 151 to cytosine at position 156, or 6 bases from cytosine at position 175 to guanine at position 180,
(2) a base sequence which is included within 34 bases from adenine at position 284 to thymine at position 317, and which contains 5 bases from guanine at position 285 to thymine at position 289, or 6 bases from thymine at position 309 to thymine at position 314, and
(3) a base sequence which is included within 34 bases from cytosine at position 343 to cytosine at position 376, and which contains 5 bases from guanine at position 355 to adenine at position 359, or 5 bases from adenine at position 369 to guanine at position 373.

5. An antisense compound according to claim 4 characterized in that said base sequence is selected from:
(4) a base sequence consisting of 16-24 bases which is included within 24 bases from guanine at position 127 to cytosine at position 150, and which contains at least 16 bases from cytosine at position 131 to adenine at position 146,
(5) a base sequence consisting of 15-30 bases which is included within 49 bases from guanine at position 127 to cytosine at position 175, and which contains at least 7 bases from cytosine at position 147 to cytosine at position 153,
(6) a base sequence consisting of 15-30 bases which is included within 31 bases from cytosine at position 150 to guanine at position 180, and which contains at least 6 bases from cytosine at position 151 to cytosine at position 156,
(7) a base sequence consisting of 15-30 bases which is included within 31 bases from cytosine at position 150 to guanine at position 180, and which contains at least 6 bases from cytosine at position 175 to guanine at position 180,
(8) a base sequence consisting of 15-33 bases which is included within 34 bases from adenine at position 284 to thymine at position 317, and which contains at least 5 bases from guanine at position 285 to thymine at position 289,
(9) a base sequence consisting of 15-33 bases which is included within 34 bases from adenine at position 284 to thymine at position 317, and which contains at least 6 bases from thymine at position 309 to thymine at position 314,
(10) a base sequence consisting of 15-30 bases which is included within 34 bases from cytosine at position 343 to cytosine at position 376, and which contains at least 5 bases from guanine at position 355 to adenine at position 359,
(11) a base sequence consisting of 15-30 bases which is included within 34 bases from cytosine at position 343 to cytosine at position 376, and which contains at least 5 bases from adenine at position 369 to guanine at position 373.
(12) a base sequence consisting of 15-26 bases which is included within 26 bases from thymine at position 351 to cytosine at position 376, and which contains at least 5 bases from guanine at position 355 to adenine at position 359, and
(13) a base sequence consisting of 15-26 bases which is included within 26 bases from thymine at position 351 to cytosine at position 376, and which contains at least 5 bases from adenine at position 369 to guanine at position 373.

6. An antisense compound according to claim 5 characterized in that said base sequence consists of 15-20 bases and is extracted from the 20 bases from cytosine at position 139 to guanine at position 158.

7. An antisense compound according to claim 5 characterized in that said base sequence is represented by the 30 bases from cytosine at position 151 to guanine at position 180.

8. An antisense compound according to claim 5 characterized in that said base sequence is represented by the 20 bases from cytosine at position 131 to cytosine at position 150.

9. An antisense compound according to claim 5 characterized in that said base sequence is represented by the 19 bases from cytosine at position 141 to guanine at position 159.

10. An antisense compound according to claim 5 characterized in that said base sequence is represented by the 20 bases from guanine at position 355 to cytosine at position 374.

11. An antisense compound according to claim 5 characterized in that said base sequence is represented by the 20 bases from thymine at position 353 to adenine at position 372.

12. An anti-hepatitis virus C formulation which comprises as an active ingredient an antisense compound according to claim 1.
